# EUROPEAN PATENT SPECIFICATION

(11) **EP 3 852 747 B1**
(45) Date of publication and mention of the grant of the patent: **31.05.2023**
(21) Application number: 19779300.3
(22) Date of filing: 16.09.2019
(51) Int. Cl.: A61K 31/42, A61K 31/4439, A61K 31/505, A61P 11/00, A61P 19/10, A61P 35/00, C07D 261/08, C07D 401/04, C07D 401/14, C07D 403/04, C07D 417/04, C07D 417/14

(54) **CYCLOHEPTYL ACIDS AS LPA ANTAGONISTS**
CYCLOHEPTYLSÄUREN ALS LPA-ANTAGONISTEN
ACIDES CYCLOHEPTYLIQUES UTILISÉS COMME ANTAGONISTES DE LPA

(30) Priority: 18.09.2018 US 201862732595 P
(43) Date of publication of application: 28.07.2021
(73) Proprietor: Bristol-Myers Squibb Company, Princeton, NJ 08543 (US)
(72) Inventor: CHENG, Peter Tai Wah, Princeton, New Jersey 08543 (US); SHI, Jun, Pennington, New Jersey 08534 (US); TAO, Shiwei, Hillsborough, New Jersey 08844 (US)
(74) Representative: Dehns
(86) International application number: PCT/US2019/051275
(87) International publication number: WO 2020/060916

(56) References cited:
- WO-A1-2011/071570
- WO-A1-2017/223016

## Description

### FIELD OF THE INVENTION

The present invention relates to novel substituted cycloheptyl acid compounds, compositions containing them, and the compounds for use in methods of treating disorders associated with one or more of the lysophosphatidic acid (LPA) receptors.

### BACKGROUND OF THE INVENTION

Lysophospholipids are membrane-derived bioactive lipid mediators, of which one of the most medically important is lysophosphatidic acid (LPA). LPA is not a single molecular entity but a collection of endogenous structural variants with fatty acids of varied lengths and degrees of saturation (Fujiwara et al., J Biol. Chem., 2005, 280, 35038-35050). The structural backbone of the LPAs is derived from glycerol-based phospholipids such as phosphatidylcholine (PC) or phosphatidic acid (PA).

The LPAs are bioactive lipids (signaling lipids) that regulate various cellular signaling pathways by binding to the same class of 7-transmembrane domain G protein-coupled (GPCR) receptors (Chun, J., Hla, T., Spiegel, S., Moolenaar, W., Editors, Lysophospholipid Receptors: Signaling and Biochemistry, 2013, Wiley; ISBN: 978-0-470-56905-4 & Zhao, Y. et al, Biochim. Biophys. Acta (BBA) Mol. Cell Biol. Of Lipids, 2013, 1831, 86-92). The currently known LPA receptors are designated as LPA₁, LPA₂, LPA₃, LPA₄, LPA₅ and LPA₆ (Choi, J. W., Annu. Rev. Pharmacol. Toxicol., 2010, 50, 157-186; Kihara, Y., et al, Br. J. Pharmacol., 2014, 171, 3575-3594).

The LPAs have long been known as precursors of phospholipid biosynthesis in both eukaryotic and prokaryotic cells, but the LPAs have emerged only recently as signaling molecules that are rapidly produced and released by activated cells, notably platelets, to influence target cells by acting on specific cell-surface receptors (see, *e.g.*, Moolenaar et al., BioEssays, 2004, 26, 870-881, and van Leewen et al., Biochem. Soc. Trans., 2003, 31, 1209-1212). Besides being synthesized and processed to more complex phospholipids in the endoplasmic reticulum, LPAs can be generated through the hydrolysis of pre-existing phospholipids following cell activation; for example, the sn-2 position is commonly missing a fatty acid residue due to deacylation, leaving only the sn-1 hydroxyl esterified to a fatty acid. Moreover, a key enzyme in the production of LPA, autotaxin (lysoPLD/NPP2), may be the product of an oncogene, as many tumor types up-regulate autotaxin (Brindley, D., J. Cell Biochem. 2004, 92, 900-12). The concentrations of LPAs in human plasma & serum as well as human bronchoalveolar lavage fluid (BALF) have been reported, including determinations made using sensitive and specific LC/MS & LC/MS/MS procedures (Baker et al. Anal. Biochem., 2001, 292, 287-295; Onorato et al., J. Lipid Res., 2014, 55, 1784-1796).

LPA influences a wide range of biological responses, ranging from induction of cell proliferation, stimulation of cell migration and neurite retraction, gap junction closure, and even slime mold chemotaxis (Goetzl, et al., Scientific World J., 2002, 2, 324-338; Chun, J., Hla, T., Spiegel, S., Moolenaar, W., Editors, Lysophospholipid Receptors: Signaling and Biochemistry, 2013, Wiley; ISBN: 978-0-470-56905-4). The body of knowledge about the biology of LPA continues to grow as more and more cellular systems are tested for LPA responsiveness. For instance, it is now known that, in addition to stimulating cell growth and proliferation, LPAs promote cellular tension and cell-surface fibronectin binding, which are important events in wound repair and regeneration (Moolenaar et al., BioEssays, 2004, 26, 870-881). Recently, anti-apoptotic activity has also been ascribed to LPA, and it has recently been reported that PPAPγ is a receptor/target for LPA (Simon et al., J. Biol. Chem., 2005, 280, 14656-14662).

Fibrosis is the result of an uncontrolled tissue healing process leading to excessive accumulation and insufficient resorption of extracellular matrix (ECM) which ultimately results in end-organ failure (Rockey, D. C., et al., New Engl. J. Med., 2015, 372, 1138-1149). Recently it was reported that the LPA1 receptor was over-expressed in idiopathic pulmonary fibrosis (IPF) patients. LPA₁ receptor knockout mice were also protected from bleomycin-induced lung fibrosis (Tager et al., Nature Med., 2008, 14, 45-54). LPA pathway inhibitors (e.g. an LPA₁ antagonist) were recently shown to be chemopreventive anti-fibrotic agents in the treatment of hepatocellular carcinoma in a rat model (Nakagawa et al., Cancer Cell, 2016, 30, 879-890).

Thus, antagonizing the LPA₁ receptor may be useful for the treatment of fibrosis such as pulmonary fibrosis, hepatic fibrosis, renal fibrosis, arterial fibrosis and systemic sclerosis, and thus the diseases that result from fibrosis (pulmonary fibrosis-Idiopathic Pulmonary Fibrosis [IPF], hepatic fibrosis-Non-alcoholic Steatohepatitis [NASH], renal fibrosis-diabetic nephropathy, systemic sclerosis-scleroderma, etc.). WO 2017/223016 discloses carbamoyloxymethyl triazole cyclohexyl acids as LPA antagonists. WO 2011/071570 discloses oxadiazole compounds and pharmaceutical compositions of such compounds.

### SUMMARY OF THE INVENTION

The present invention provides novel cycloheptyl acid compounds including stereoisomers, tautomers, pharmaceutically acceptable salts or solvates thereof, which are useful as antagonists against one or more of the lysophosphatidic acid (LPA) receptors, especially the LPA1 receptor.

The present invention also provides pharmaceutical compositions comprising a pharmaceutically acceptable carrier and at least one of the compounds of the present invention or stereoisomers, tautomers, pharmaceutically acceptable salts or solvates thereof.

The compounds of the invention may be used in the treatment of conditions in which LPA plays a role.

The compounds of the present invention may be used in therapy.

The compounds of the present invention may be used for the manufacture of a medicament for the treatment of a condition in which inhibition of the physiological activity of LPA is useful, such as diseases in which an LPA receptor participates, is involved in the etiology or pathology of the disease, or is otherwise associated with at least one symptom of the disease.

In another aspect, the present invention is directed to compounds of the present invention for use in a method of treating fibrosis of organs (liver, kidney, lung, heart and the like as well as skin), liver diseases (acute hepatitis, chronic hepatitis, liver fibrosis, liver cirrhosis, portal hypertension, regenerative failure, non-alcoholic steatohepatitis (NASH), liver hypofunction, hepatic blood flow disorder, and the like), cell proliferative disease [cancer (solid tumor, solid tumor metastasis, vascular fibroma, myeloma, multiple myeloma, Kaposi's sarcoma, leukemia, chronic lymphocytic leukemia (CLL) and the like) and invasive metastasis of cancer cell, and the like], inflammatory disease (psoriasis, nephropathy, pneumonia and the like), gastrointestinal tract disease (irritable bowel syndrome (IBS), inflammatory bowel disease (IBD), abnormal pancreatic secretion, and the like), renal disease, urinary tract-associated disease (benign prostatic hyperplasia or symptoms associated with neuropathic bladder disease, spinal cord tumor, hernia of intervertebral disk, spinal canal stenosis, symptoms derived from diabetes, lower urinary tract disease (obstruction of lower urinary tract, and the like), inflammatory disease of lower urinary tract, dysuria, frequent urination, and the like), pancreas disease, abnormal angiogenesis-associated disease (arterial obstruction and the like), scleroderma, brain-associated disease (cerebral infarction, cerebral hemorrhage, and the like), neuropathic pain, peripheral neuropathy, and the like, ocular disease (age-related macular degeneration (AMD), diabetic retinopathy, proliferative vitreoretinopathy (PVR), cicatricial pemphigoid, glaucoma filtration surgery scarring, and the like).

Compounds of the present invention may be used in a method of treating diseases, disorders, or conditions in which activation of at least one LPA receptor by LPA contributes to the symptomology or progression of the disease, disorder or condition. These diseases, disorders, or conditions may arise from one or more of a genetic, iatrogenic, immunological, infectious, metabolic, oncological, toxic, surgical, and/or traumatic etiology.

Compounds of the the present invention may be used in a method of treating renal fibrosis, pulmonary fibrosis, hepatic fibrosis, arterial fibrosis or systemic sclerosis comprising administering to a patient in need of such treatment a compound of the present invention as described above.

In one aspect, the present invention provides compounds, pharmaceutical compositions, and medicaments described herein that comprise antagonists of LPA receptors, especially antagonists of LPA1.

The compounds of the invention can be used alone, in combination with other compounds of the present invention, or in combination with one or more, preferably one to two other agent(s).

These and other features of the invention will be set forth in expanded form as the disclosure continues.

### DETAILED DESCRIPTION OF THE INVENTION

### I. COMPOUNDS OF THE INVENTION

In a 1st aspect, the present invention provides, *inter alia,* a compound of Formula (I): 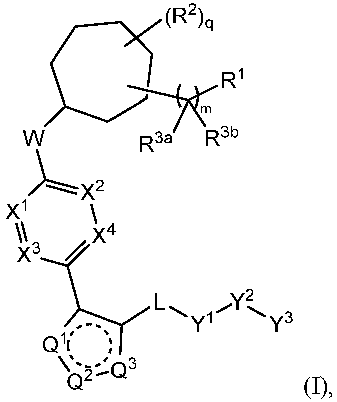 or a stereoisomer, tautomer, or pharmaceutically acceptable salt or solvate thereof, wherein:
X¹, X², X³, and X⁴ are each independently CR⁶ or N; provided that no more than two of X¹, X², X³, or X⁴ are N;
the 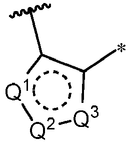 moiety is independently 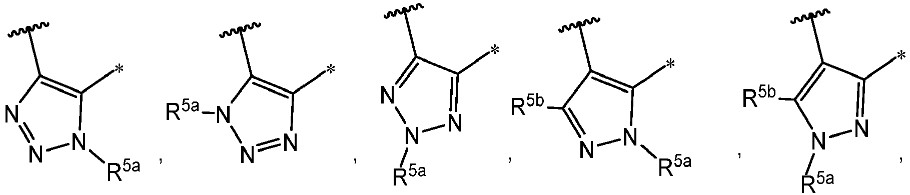 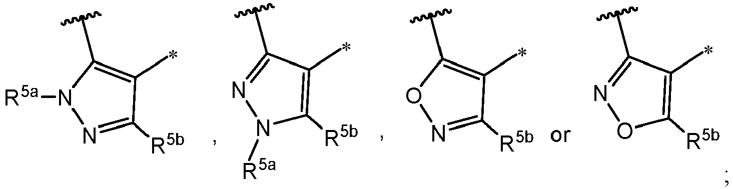
* denotes the attachment point to L;
L is independently a covalent bond or CH₂;
WisO;
the 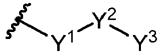 moiety is independently -OR^{4b}, -NR⁷R^{4b}, 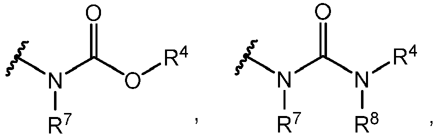 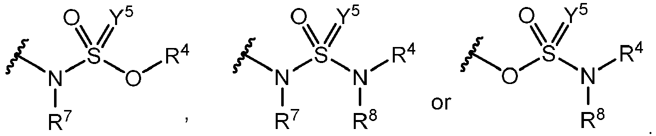
Y⁵ independently is O or NH;
R¹ is CO₂H;
R² is each independently halo, cyano, hydroxyl, amino, C₁₋₄ alkoxy, C₁₋₄ haloalkyl, C₁₋₄ haloalkoxy, C₁₋₄ alkylamino, -(CH₂)₀₋₁-(C₃₋₆ cycloalkyl), -(CH₂)₀₋₁-phenyl, or C₁₋₆ alkyl;
R^{3a} is independently hydrogen, halo, hydroxyl, or C₁₋₄ alkyl;
R^{3b} is independently hydrogen, halo, cyano, hydroxyl, amino, C₁₋₄ alkyl, C₁₋₄ haloalkyl, C₁₋₄ alkoxy, C₁₋₄ haloalkoxy or C₁₋₆ alkyl substituted with 0 to 2 R^{a};
or alternatively, R^{3a} and R^{3b} together, with the carbon atom they are attached to, form a C₃₋₄ carbocyclyl;
R⁴ is -L₁-R^{4a};
L₁ is independently a covalent bond or C₁₋₄ alkylene substituted with 0 to 4 R⁹;
R^{4a} is independently C₁₋₁₀ alkyl, C₁₋₁₀ haloalkyl, C₁₋₁₀ alkenyl, C₃₋₈ cycloalkyl, C₆₋₁₀ aryl, 3 to 8-membered heterocyclyl, 5 to 6-membered heteroaryl; wherein each of the alkyl, alkenyl, alkylene, cycloalkyl, aryl, heterocyclyl, and heteroaryl, by itself or as part of other moiety, is independently substituted with 0 to 3 R¹⁰;
R^{4b} is independently C₁₋₁₀ alkyl, C₁₋₁₀ haloalkyl, C₁₋₁₀ alkenyl, C₃₋₈ cycloalkyl, C₆₋₁₀ aryl, 3 to 8-membered heterocyclyl, 5 to 6-membered heteroaryl; wherein each of the alkyl, alkenyl, alkylene, cycloalkyl, aryl, heterocyclyl, and heteroaryl, by itself or as part of other moiety, is independently substituted with 0 to 3 R^{10a};
R^{5a} is independently hydrogen, C₁₋₄ haloalkyl, -(CH₂)₀₋₁-(C₃₋₆ cycloalkyl), -(CH₂)₀₋₁-phenyl, or C₁₋₆ alkyl substituted with 0 to 3 R^{a};
R^{5b} is independently hydrogen, halo, cyano, hydroxyl, amino, C₁₋₄ alkoxy, C₁₋₄ haloalkyl, C₁₋₄ haloalkoxy, C₁₋₄ alkylamino, -(CH₂)₀₋₁-(C₃₋₆ cycloalkyl), -(CH₂)₀₋₁-phenyl, or C₁₋₆ alkyl substituted with 0 to 3 R^{b};
R⁶ is each independently hydrogen, halo, cyano, hydroxyl, amino, C₁₋₄ alkylamino, C₁₋₄ haloalkyl, C₁₋₄ alkoxy, C₁₋₄ haloalkoxy or C₁₋₆ alkyl substituted with 0 to 1 R^{b};
R⁷ and R⁸ are each independently hydrogen, C₁₋₆ alkyl, C₁₋₄ haloalkyl, C₃₋₆ cycloalkyl or C₁₋₆ alkyl;
R⁹ is each independently halo, oxo, cyano, hydroxyl, amino, C₁₋₄ haloalkyl, C₁₋₄ alkoxy, C₁₋₄ haloalkoxy, C₃₋₆ cycloalkyl, or C₁₋₆ alkyl substituted with 0 to 3 R^{a};
R¹⁰ and R^{10a} are each independently halo, hydroxyl, amino, cyano, C₂₋₆ alkenyl, C₂₋₆ alkynyl, C₁₋₄ alkylamino, C₁₋₄ haloalkyl, C₁₋₄ alkoxy, C₁₋₄ haloalkoxy, phenyl, or 5 to 6 membered heteroaryl, C₁₋₆ alkyl substituted with 0 to 3 R^{b};
R^{a} is independently halo, cyano, hydroxyl, C₁₋₄ alkoxy, C₁₋₄ haloalkyl, or C₁₋₄ haloalkoxy;
R^{b} is independently halo, cyano, hydroxyl, amino, C₁₋₄ alkoxy, C₁₋₄ haloalkyl, or C₁₋₄ haloalkoxy;
m is an integer of 0, 1, or 2; and
q is an integer of 0, 1, or 2.

In one embodiment of Formula (I), within the scope of the 1st aspect, wherein X¹, X², X³, and X⁴ are CR⁶; where R⁶ is hydrogen halo or C₁₋₄ alkyl, e.g., methyl.

In another aspect, within the scope of any of the 1^{st} aspect, wherein X¹, X², X³, and X⁴ are independently CH or CR^{6a}; or one of X¹, X², X³, and X⁴ is N, and the remaining ones are CH or CR^{6a}; or two of X¹, X², X³, and X⁴ are N, and the remaining ones are CH or CR^{6a}; and R^{6a} is independently halo, hydroxyl, C₁₋₆ alkyl, C₁₋₄ haloalkyl, or C₁₋₄ alkoxy.

In a 2nd aspect, within the scope of the 1st aspect, wherein R^{3a} and R^{3b} are independently hydrogen or C₁₋₄ alkyl;
or alternatively, R^{3a} and R^{3b} together, with the carbon atom they are attached to, form C₃₋₄ cycloalkyl.

In a 3rd aspect, within the scope of any of the 1st or 2nd aspects, wherein:
X¹, X², X³, and X⁴ are independently CR⁶; or X¹, X² and X³ are CR⁶ and X⁴ is N; or X² and X³ are CR⁶ and X¹ and X⁴ are N; or X¹ and X² are CR⁶ and X³ and X⁴ are N; and R⁶ is independently hydrogen, halo, hydroxyl, C₁₋₆ alkyl, C₁₋₄ haloalkyl, or C₁₋₄ alkoxy.

In an 4th aspect, within the scope of any of the 1st to 3rd aspects, wherein:
X¹, X², X³, and X⁴ are independently CR⁶; or X¹, X² and X³ are CR⁶ and X⁴ is N; or X² and X³ are CR⁶ and X¹ and X⁴ are N;
the 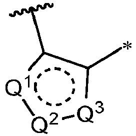 moiety is independently 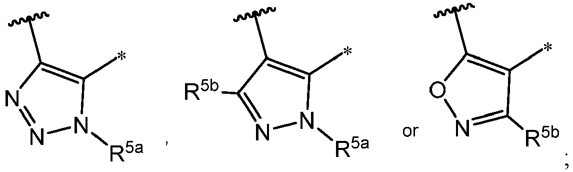
* denotes the attachment point to L;
the 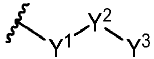 moiety is independently -OR^{4b}, or 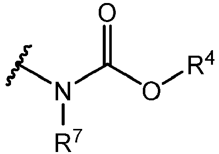
R² is each independently halo, cyano, hydroxyl, C₁₋₄ alkoxy, C₁₋₄ haloalkyl or C₁₋₄ haloalkoxy;
R⁴ is independently C₃₋₆ alkyl, -(CH₂)₀₋₁-C₃₋₆ cycloalkyl, -(CH(C₁₋₂ alkyl))-C₃₋₆ cycloalkyl, -(CH₂)₀₋₁-phenyl or -(CH(C₁₋₂ alkyl))-phenyl, wherein each of said cycloalkyl and phenyl is independently substituted with 0 to 3 R¹⁰;
R^{4b} is independently 5 to 6-membered heteroaryl substituted with 0 to 2 R^{10a};
R^{5a} is independently C₁₋₆ alkyl, or -(CH₂)₀₋₁-(C₃₋₆ cycloalkyl);
R^{5b} is independently hydrogen, halo, cyano, hydroxyl, C₁₋₄ alkyl, C₁₋₄ alkoxy, C₁₋₄ haloalkyl, C₁₋₄ haloalkoxy, or -(CH₂)₀₋₁-(C₃₋₆ cycloalkyl);
R⁶ is each independently hydrogen, halo, hydroxyl, C₁₋₆ alkyl, C₁₋₄ haloalkyl, or C₁₋₄ alkoxy;
R⁷ and R⁸ are each independently hydrogen or C₁₋₂ alkyl;
R¹⁰ is each independently halo, cyano, hydroxyl, C₁₋₆ alkyl, C₁₋₆ haloalkyl, C₁₋₆ alkoxy, or C₁₋₆ haloalkoxy;
R^{10a} is each independently halo, cyano, hydroxyl, C₁₋₆ alkyl, C₁₋₆ haloalkyl, C₁₋₆ alkoxy, C₁₋₆ haloalkoxy, -(CH₂)₀₋₃-C₃₋₆ cycloalkyl, phenyl or 5 to 6 membered heteroaryl;
m is 0 or 1; and
q is 0 or 1.

In a 5th aspect, within the scope of the 1st to 4th aspect, wherein:
the 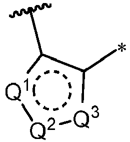 moiety is independently 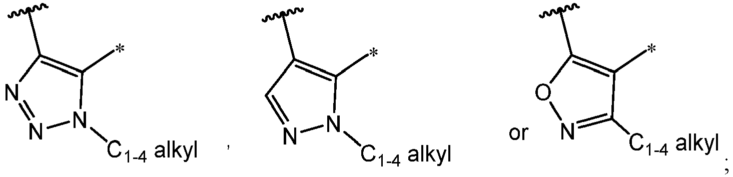
* denotes the attachment point to L;
the 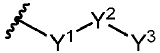 moiety is independently -OR^{4b}, or 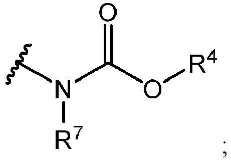
R^{3a} and R^{3b} are each independently hydrogen or C₁₋₄ alkyl;
or alternatively, R^{3a} and R^{3b} together, with the carbon atom they are attached to, form C₃₋₄ cycloalkyl;
R⁴ is independently C₃₋₆ alkyl, -(CH₂)₀₋₁-C₃₋₆ cycloalkyl, -(CH₂)-phenyl, or -(CH(CH₃))-phenyl; wherein wherein each of said alkyl, cycloalkyl and phenyl is independently substituted with 0 to 2 R¹⁰;
R^{4b} is independently 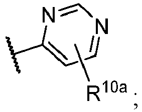
R⁶ is independently hydrogen, halo or C₁₋₄ alkyl;
R⁷ and R⁸ are each independently hydrogen or C₁₋₂ alkyl;
R¹⁰ is each independently halo or C₁₋₄ alkyl; and
R^{10a} is independently C₁₋₄ alkyl, C₁₋₄ alkoxy, -(CH₂)₁₋₃-C₃₋₆ cycloalkyl, phenyl or pyridyl.

In another aspect, within the scope of any one of the 1st to 5th aspects, wherein the compound is of Formula (Ia): 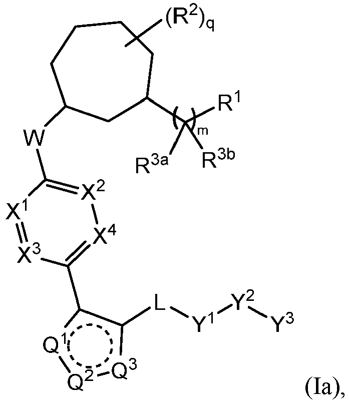 or a stereoisomer, tautomer, or pharmaceutically acceptable salt or solvate thereof.

In a 6th aspect, the present invention provides a compound of Formula (II): 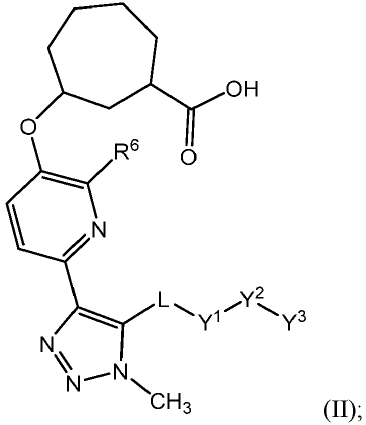 or a stereoisomer, tautomer, or pharmaceutically acceptable salt or solvate thereof, wherein:
the 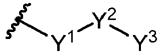 moiety is independently -OR^{4b}, or 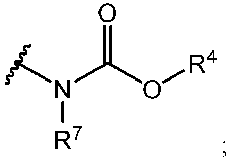
R⁴ is independently C₃₋₆ alkyl, -(CH₂)₀₋₁-C₃₋₆ cycloalkyl, -(CH₂)-phenyl, or -(CH(CH₃))-phenyl;
R^{4b} is independently 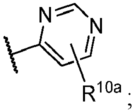
R⁶ is independently hydrogen, halo or C₁₋₄ alkyl;
R⁷ and R⁸ are each independently hydrogen or methyl;
R^{10a} is independently C₁₋₄ alkyl or C₁₋₄ alkoxy.

In one embodiment of the present invention, the compound is selected from any one of the Examples as described in the specification, or a stereoisomer, a tautomer, or a pharmaceutically acceptable salt or solvate thereof.

In one embodiment, the compounds of the present invention have hLPA1 EC₅₀ values ≤ 5000 nM, using the LPA1 functional antagonist assay; in another embodiment, the compounds of the present invention have hLPA1 EC₅₀ values ≤ 1000 nM; in another embodiment, the compounds of the present invention have hLPA1 EC₅₀ values ≤ 500 nM; in another embodiment, the compounds of the present invention have hLPA1 EC₅₀ values ≤ 200 nM; in another embodiment, the compounds of the present invention have hLPA1 EC₅₀ values ≤ 100 nM; in another embodiment, the compounds of the present invention have hLPA1 EC₅₀ values ≤ 50 nM.

### II. OTHER EMBODIMENTS OF THE INVENTION

In some embodiments, the compound of the present invention, or a pharmaceutically acceptable salt thereof, is an antagonist of at least one LPA receptor. In some embodiments, the compound of the present invention, or a pharmaceutically acceptable salt thereof, is an antagonist of LPA₁. In some embodiments, the compound of the present invention, or a pharmaceutically acceptable salt thereof, is an antagonist of LPA₂. In some embodiments, the compound of the present invention, or a pharmaceutically acceptable salt thereof, is an antagonist of LPA₃.

In some embodiments, presented herein are compounds selected from tautomers, pharmaceutically acceptable salts or solvates of a compound of the present invention.

In another embodiment, the present invention provides a composition comprising at least one of the compounds of the present invention or a stereoisomer, a tautomer, a pharmaceutically acceptable salt, or a solvate thereof.

In another embodiment, the present invention provides a pharmaceutical composition, comprising a pharmaceutically acceptable carrier and a therapeutically effective amount of at least one of the compounds of the present invention or a stereoisomer, a tautomer, a pharmaceutically acceptable salt, or a solvate thereof.

In another embodiment, the present invention provides a pharmaceutical composition further comprising additional therapeutic agent(s).

In another embodiment, the present invention provides compounds for use in a method for the treatment of a condition associated with LPA receptor mediated fibrosis, comprising administering to a patient in need of such treatment a therapeutically effective amount of at least one of the compounds of the present invention or a stereoisomer, a tautomer, a pharmaceutically acceptable salt, or a solvate thereof. As used herein, the term "patient" encompasses all mammalian species.

In another embodiment, the present invention provides compounds for use in a method of treating a disease, disorder, or condition associated with dysregulation of lysophosphatidic acid receptor 1 (LPA1) in a patient in need thereof, comprising administering a therapeutically effective amount of a compound of the present invention, or a stereoisomer, a tautomer, or a pharmaceutically acceptable salt or solvate thereof, to the patient. The disease, disorder, or condition may be related to pathological fibrosis, transplant rejection, cancer, osteoporosis, or inflammatory disorders. The pathological fibrosis may be pulmonary, liver, renal, cardiac, dernal, ocular, or pancreatic fibrosis. The disease, disorder, or condition may be idiopathic pulmonary fibrosis (IPF), non-alcoholic steatohepatitis (NASH), non-alcoholic fatty liver disease (NAFLD), chronic kidney disease, diabetic kidney disease, or systemic sclerosis. The cancer may be of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid.

In another embodiment, the present invention provides compounds for use in a method of treating fibrosis in a mammal comprising administering a therapeutically effective amount of a compound of the present invention, or a stereoisomer, a tautomer, or a pharmaceutically acceptable salt or solvate thereof, to the mammal in need thereof. The fibrosis may be idiopathic pulmonary fibrosis (IPF), nonalcoholic steatohepatitis (NASH), chronic kidney disease, diabetic kidney disease, or systemic sclerosis.

In another embodiment, the present invention provides compounds for use in a method of treating lung fibrosis (idiopathic pulmonary fibrosis), asthma, chronic obstructive pulmonary disease (COPD), renal fibrosis, acute kidney injury, chronic kidney disease, liver fibrosis (non-alcoholic steatohepatitis), skin fibrosis, fibrosis of the gut, breast cancer, pancreatic cancer, ovarian cancer, prostate cancer, glioblastoma, bone cancer, colon cancer, bowel cancer, head and neck cancer, melanoma, multiple myeloma, chronic lymphocytic leukemia, cancer pain, tumor metastasis, transplant organ rejection, scleroderma, ocular fibrosis, age related macular degeneration (AMD), diabetic retinopathy, collagen vascular disease, atherosclerosis, Raynaud's phenomenon, or neuropathic pain in a mammal ccomprising administering a therapeutically effective amount of a compound of the present invention, or a stereoisomer, a tautomer, or a pharmaceutically acceptable salt or solvate thereof, to the mammal in need thereof.

As used herein, "treating" or "treatment" cover the treatment of a disease-state in a mammal, particularly in a human, and include: (a) inhibiting the disease-state, i.e., arresting it development; and/or (b) relieving the disease-state, i.e., causing regression of the disease state. As used herein, "treating" or "treatment" also include the protective treatment of a disease state to reduce and/or minimize the risk and/or reduction in the risk of recurrence of a disease state by administering to a patient a therapeutically effective amount of at least one of the compounds of the present invention or a or a stereoisomer, a tautomer, a pharmaceutically acceptable salt, or a solvate thereof. Patients may be selected for such protective therapy based on factors that are known to increase risk of suffering a clinical disease state compared to the general population. For protective treatment, conditions of the clinical disease state may or may not be presented yet. The protective treatment can be divided into (a) primary prophylaxis and (b) secondary prophylaxis. Primary prophylaxis is defined as treatment to reduce or minimize the risk of a disease state in a patient that has not yet presented with a clinical disease state, whereas secondary prophylaxis is defined as minimizing or reducing the risk of a recurrence or second occurrence of the same or similar clinical disease state.

### III. CHEMISTRY

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Many geometric isomers of C=C double bonds, C=N double bonds, ring systems, and the like can also be present in the compounds, and all such stable isomers are contemplated in the present invention. *Cis-* and *trans*- (or *E*- and Z-) geometric isomers of the compounds of the present invention are described and may be isolated as a mixture of isomers or as separated isomeric forms. The present compounds can be isolated in optically active or racemic forms. Optically active forms may be prepared by resolution of racemic forms or by synthesis from optically active starting materials. All processes used to prepare compounds of the present invention and intermediates made therein are considered to be part of the present invention. When enantiomeric or diastereomeric products are prepared, they may be separated by conventional methods, for example, by chromatography or fractional crystallization. Depending on the process conditions the end products of the present invention are obtained either in free (neutral) or salt form. Both the free form and the salts of these end products are within the scope of the invention. If so desired, one form of a compound may be converted into another form. A free base or acid may be converted into a salt; a salt may be converted into the free compound or another salt; a mixture of isomeric compounds of the present invention may be separated into the individual isomers. Compounds of the present invention, free form and salts thereof, may exist in multiple tautomeric forms, in which hydrogen atoms are transposed to other parts of the molecules and the chemical bonds between the atoms of the molecules are consequently rearranged. It should be understood that all tautomeric forms, insofar as they may exist, are included within the invention.

The term "stereoisomer" refers to isomers of identical constitution that differ in the arrangement of their atoms in space. Enantiomers and diastereomers are examples of stereoisomers. The term "enantiomer" refers to one of a pair of molecular species that are mirror images of each other and are not superimposable. The term "diastereomer" refers to stereoisomers that are not mirror images. The term "racemate" or "racemic mixture" refers to a composition composed of equimolar quantities of two enantiomeric species, wherein the composition is devoid of optical activity.

The symbols "R" and "S" represent the configuration of substituents around a chiral carbon atom(s). The isomeric descriptors "R" and "S" are used as described herein for indicating atom configuration(s) relative to a core molecule and are intended to be used as defined in the literature (IUPAC Recommendations 1996, Pure and Applied Chemistry, 68:2193-2222 (1996)).

The term "chiral" refers to the structural characteristic of a molecule that makes it impossible to superimpose it on its mirror image. The term "homochiral" refers to a state of enantiomeric purity. The term "optical activity" refers to the degree to which a homochiral molecule or nonracemic mixture of chiral molecules rotates a plane of polarized light.

As used herein, the term "alkyl" or "alkylene" is intended to include both branched and straight-chain saturated aliphatic hydrocarbon groups having the specified number of carbon atoms. While "alkyl" denotes a monovalent saturated aliphatic radical (such as ethyl), "alkylene" denotes a bivalent saturated aliphatic radical (such as ethylene). For example, "C₁ to C₁₀ alkyl" or "C₁₋₁₀ alkyl" is intended to include C₁, C₂, C₃, C₄, C₅, C₆, C₇, C₈, C₉, and C₁₀ alkyl groups. "C₁ to C₁₀ alkylene" or "C₁₋₁₀ alkylene", is intended to include C₁, C₂, C₃, C₄, C₅, C₆, C₇, C₈, C₉, and C₁₀ alkylene groups. Additionally, for example, "C₁ to C₆ alkyl" or "C₁₋₆ alkyl" denotes alkyl having 1 to 6 carbon atoms; and "C₁ to C₆ alkylene" or " C₁₋₆ alkylene" denotes alkylene having 1 to 6 carbon atoms; and "C₁ to C₄ alkyl" or "C₁₋₄ alkyl" denotes alkyl having 1 to 4 carbon atoms; and "C₁ to C₄ alkylene" or "C₁₋₄ alkylene" denotes alkylene having 1 to 4 carbon atoms. Alkyl group can be unsubstituted or substituted with at least one hydrogen being replaced by another chemical group. Example alkyl groups include, but are not limited to, methyl (Me), ethyl (Et), propyl (*e.g*., n-propyl and isopropyl), butyl (*e.g*., n-butyl, isobutyl, *t*-butyl), and pentyl (*e.g*., n-pentyl, isopentyl, neopentyl). When "C₀ alkyl" or "C₀ alkylene" is used, it is intended to denote a direct bond. Furthermore, the term "alkyl", by itself or as part of another group, such as alkylamino, haloalkyl, hydroxyalkyl, aminoalkyl, alkoxy, alkoxyalkyl, haloalkoxyalkyl, and haloalkoxy, can be an alkyl having 1 to 4 carbon atoms, or 1 to 6 carbon atoms, or 1 to 10 carbon atoms.

"Heteroalkyl" refers to an alkyl group where one or more carbon atoms have been replaced with a heteroatom, such as, O, N, or S. For example, if the carbon atom of the alkyl group which is attached to the parent molecule is replaced with a heteroatom (*e.g*., O, N, or S) the resulting heteroalkyl groups are, respectively, an alkoxy group (*e.g*., -OCH₃, etc.), an alkylamino (*e.g*., -NHCH₃, -N(CH₃)₂, etc.), or a thioalkyl group (*e.g*., -SCH₃). If a non-terminal carbon atom of the alkyl group which is not attached to the parent molecule is replaced with a heteroatom (*e.g*., O, N, or S) and the resulting heteroalkyl groups are, respectively, an alkyl ether (*e.g*., -CH₂CH₂-O-CH₃, etc.), an alkylaminoalkyl (*e.g*., -CH₂NHCH₃, -CH₂N(CH₃)₂, etc.), or a thioalkyl ether (*e.g*.,-CH₂-S-CH₃). If a terminal carbon atom of the alkyl group is replaced with a heteroatom (*e.g*., O, N, or S), the resulting heteroalkyl groups are, respectively, a hydroxyalkyl group (*e.g*., -CH₂CH₂-OH), an aminoalkyl group (*e.g*., -CH₂NH₂), or an alkyl thiol group (*e.g*., -CH₂CH₂-SH). A heteroalkyl group can have, for example, 1 to 20 carbon atoms, 1 to 10 carbon atoms, or 1 to 6 carbon atoms. A C₁-C₆ heteroalkyl group means a heteroalkyl group having 1 to 6 carbon atoms.

"Alkenyl" or "alkenylene" is intended to include hydrocarbon chains of either straight or branched configuration having the specified number of carbon atoms and one or more, preferably one to two, carbon-carbon double bonds that may occur in any stable point along the chain. For example, "C₂ to C₆ alkenyl" or "C₂₋₆ alkenyl" (or alkenylene), is intended to include C₂, C₃, C₄, C₅, and C₆ alkenyl groups. Examples of alkenyl include, but are not limited to, ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, and 4-methyl-3-pentenyl.

"Alkynyl" or "alkynylene" is intended to include hydrocarbon chains of either straight or branched configuration having one or more, preferably one to three, carbon-carbon triple bonds that may occur in any stable point along the chain. For example, "C₂ to C₆ alkynyl" or "C₂₋₆ alkynyl" (or alkynylene), is intended to include C₂, C₃, C₄, C₅, and C₆ alkynyl groups; such as ethynyl, propynyl, butynyl, pentynyl, and hexynyl.

As used herein, "arylalkyl" (a.k.a. aralkyl), "heteroarylalkyl" "carbocyclylalkyl" or "heterocyclylalkyl" refers to an acyclic alkyl radical in which one of the hydrogen atoms bonded to a carbon atom, typically a terminal or sp³ carbon atom, is replaced with an aryl, heteroaryl, carbocyclyl, or heterocyclyl radical, respectively. Typical arylalkyl groups include, but are not limited to, benzyl, 2-phenylethan-1-yl, naphthylmethyl, 2-naphthylethan-1-yl, naphthobenzyl, 2-naphthophenylethan-1-yl and the like. The arylalkyl, heteroarylalkyl, carbocyclylalkyl, or heterocyclylalkyl group can comprise 4 to 20 carbon atoms and 0 to 5 heteroatoms, *e.g*., the alkyl moiety may contain 1 to 6 carbon atoms.

The term "benzyl", as used herein, refers to a methyl group on which one of the hydrogen atoms is replaced by a phenyl group.

"Benzyl" can also be represented by formula "Bn".

The term "alkoxy" or "alkyloxy" refers to an -O-alkyl group. "C₁ to C₆ alkoxy" or "C₁₋₆ alkoxy" (or alkyloxy), is intended to include C₁, C₂, C₃, C₄, C₅, and C₆ alkoxy groups. Example alkoxy groups include, but are not limited to, methoxy, ethoxy, propoxy (*e.g*., n-propoxy and isopropoxy), and *t*-butoxy. Similarly, "alkylthio" or "thioalkoxy" represents an alkyl group as defined above with the indicated number of carbon atoms attached through a sulphur bridge; for example, methyl-S- and ethyl-S-.

The term "alkanoyl" or "alkylcarbonyl" as used herein alone or as part of another group refers to alkyl linked to a carbonyl group. For example, alkylcarbonyl may be represented by alkyl-C(O)-. "C₁ to C₆ alkylcarbonyl" (or alkylcarbonyl), is intended to include C₁, C₂, C₃, C₄, C₅, and C₆ alkyl-C(O)- groups.

The term "alkylsulfonyl" or "sulfonamide" as used herein alone or as part of another group refers to alkyl or amino linked to a sulfonyl group. For example, alkylsulfonyl may be represented by -S(O)₂R', while sulfonamide may be represented by -S(O)₂NR^{c}R^{d}. R' is C₁ to C₆ alkyl; and R^{c} and R^{d} are the same as defined below for "amino".

The term "carbamate" as used herein alone or as part of another group refers to oxygen linked to an amido group. For example, carbamate may be represented by N(R^{c}R^{d})-C(O)-O-, and R^{c} and R^{d} are the same as defined below for "amino".

The term "amido" as used herein alone or as part of another group refers to amino linked to a carbonyl group. For example, amido may be represented by N(R^{c}R^{d})-C(O)-, and R^{c} and R^{d} are the same as defined below for "amino".

The term "amino" is defined as -NR^{c1}R^{c2}, wherein R^{c1} and R^{c2} are independently H or C₁₋₆ alkyl; or alternatively, R^{c1} and R^{c2}, taken together with the atoms to which they are attached, form a 3- to 8-membered heterocyclic ring which is optionally substituted with one or more group selected from halo, cyano, hydroxyl, amino, oxo, C₁₋₆ alkyl, alkoxy, and aminoalkyl. When R^{c1} or R^{c2} (or both of them) is C₁₋₆ alkyl, the amino group can also be referred to as alkylamino. Examples of alkylamino group include, without limitation, methylamino, ethylamino, propylamino, isopropylamino and the like. In one embodiment, amino is -NH₂.

The term "aminoalkyl" refers to an alkyl group on which one of the hydrogen atoms is replaced by an amino group. For example, aminoalkyl may be represented by N(R^{c1}R^{c2})-alkylene-. "C₁ to C₆" or "C₁₋₆ aminoalkyl" (or aminoalkyl), is intended to include C₁, C₂, C₃, C₄, C₅, and C₆ aminoalkyl groups.

The term "halogen" or "halo" as used herein alone or as part of another group refers to chlorine, bromine, fluorine, and iodine, with chlorine or fluorine being preferred.

"Haloalkyl" is intended to include both branched and straight-chain saturated aliphatic hydrocarbon groups having the specified number of carbon atoms, substituted with one or more halogens. "C₁ to C₆ haloalkyl" or "C₁₋₆ haloalkyl" (or haloalkyl), is intended to include C₁, C₂, C₃, C₄, C₅, and C₆ haloalkyl groups. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" that is intended to include both branched and straight-chain saturated aliphatic hydrocarbon groups having the specified number of carbon atoms, substituted with 1 or more fluorine atoms. The term "polyhaloalkyl" as used herein refers to an "alkyl" group as defined above which includes from 2 to 9, preferably from 2 to 5, halo substituents, such as F or Cl, preferably F, such as polyfluoroalkyl, for example, CF₃CH₂, CF₃ or CF₃CF₂CH₂.

"Haloalkoxy" or "haloalkyloxy" represents a haloalkyl group as defined above with the indicated number of carbon atoms attached through an oxygen bridge. For example, " C₁ to C₆ haloalkoxy" or "C₁₋₆ haloalkoxy", is intended to include C₁, C₂, C₃, C₄, C₅, and C₆ haloalkoxy groups. Examples of haloalkoxy include, but are not limited to, trifluoromethoxy, 2,2,2-trifluoroethoxy, and pentafluorothoxy. Similarly, "haloalkylthio" or "thiohaloalkoxy" represents a haloalkyl group as defined above with the indicated number of carbon atoms attached through a sulphur bridge; for example trifluoromethyl-S-, and pentafluoroethyl-S-. The term "polyhaloalkyloxy" as used herein refers to an "alkoxy" or "alkyloxy" group as defined above which includes from 2 to 9, preferably from 2 to 5, halo substituents, such as F or Cl, preferably F, such as polyfluoroalkoxy, for example, CF₃CH₂O, CF₃O or CF₃CF₂CH₂O.

"Hydroxyalkyl" is intended to include both branched and straight-chain saturated aliphatic hydrocarbon groups having the specified number of carbon atoms, substituted with 1 or more hydroxyl (OH). "C₁ to C₆ hydroxyalkyl" (or hydroxyalkyl), is intended to include C₁, C₂, C₃, C₄, C₅, and C₆ hydroxyalkyl groups.

The term "cycloalkyl" refers to cyclized alkyl groups, including mono-, bi- or poly-cyclic ring systems. "C₃ to C₈ cycloalkyl" or "C₃₋₈ cycloalkyl" is intended to include C₃, C₄, C₅, C₆, C₇, and C₈ cycloalkyl groups, including monocyclic, bicyclic, and polycyclic rings. Example cycloalkyl groups include, but are not limited to, cyclopropyl, cyclobutyl, cyclopentyl, cyclohexyl, and norbornyl. Branched cycloalkyl groups such as 1-methylcyclopropyl and 2-methylcyclopropyl and spiro and bridged cycloalkyl groups are included in the definition of "cycloalkyl".

The term "cycloheteroalkyl" refers to cyclized heteroalkyl groups, including mono-, bi- or poly-cyclic ring systems. "C₃ to C₇ cycloheteroalkyl" or "C₃₋₇ cycloheteroalkyl" is intended to include C₃, C₄, C₅, C₆, and C₇ cycloheteroalkyl groups. Example cycloheteroalkyl groups include, but are not limited to, oxetanyl, tetrahydrofuranyl, tetrahydropyranyl, azetidinyl, pyrrolidinyl, piperidinyl, morpholinyl, and piperazinyl. Branched cycloheteroalkyl groups, such as piperidinylmethyl, piperazinylmethyl, morpholinylmethyl, pyridinylmethyl, pyridizylmethyl, pyrimidylmethyl, and pyrazinylmethyl, are included in the definition of "cycloheteroalkyl".

As used herein, "carbocycle", "carbocyclyl" or "carbocyclic residue" is intended to mean any stable 3-, 4-, 5-, 6-, 7-, or 8-membered monocyclic or bicyclic or 7-, 8-, 9-, 10-, 11-, 12-, or 13-membered bicyclic or tricyclic hydrocarbon ring, any of which may be saturated, partially unsaturated, unsaturated or aromatic. Examples of such carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane (decalin), [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocycle (*e.g*., [2.2.2]bicyclooctane). Preferred carbocycles, unless otherwise specified, are cyclopropyl, cyclobutyl, cyclopentyl, cyclohexyl, phenyl, and indanyl. When the term "carbocyclyl" is used, it is intended to include "aryl". A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge.

Furthermore, the term "carbocyclyl", including "cycloalkyl" and "cycloalkenyl", as employed herein alone or as part of another group includes saturated or partially unsaturated (containing 1 or 2 double bonds) cyclic hydrocarbon groups containing 1 to 3 rings, including monocyclicalkyl, bicyclicalkyl and tricyclicalkyl, containing a total of 3 to 20 carbons forming the rings, preferably 3 to 10 carbons or 3 to 6 carbons, forming the ring and which may be fused to 1 or 2 aromatic rings as described for aryl, which include cyclopropyl, cyclobutyl, cyclopentyl, cyclohexyl, cycloheptyl, cyclooctyl, cyclodecyl and cyclododecyl, cyclohexenyl, 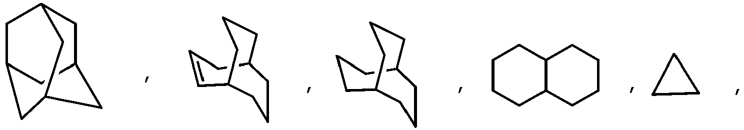 any of which groups may be optionally substituted with 1 to 4 substituents such as halogen, alkyl, alkoxy, hydroxy, aryl, aryloxy, arylalkyl, cycloalkyl, alkylamido, alkanoylamino, oxo, acyl, arylcarbonylamino, nitro, cyano, thiol and/or alkylthio and/or any of the alkyl substituents .

As used herein, the term "bicyclic carbocyclyl" or "bicyclic carbocyclic group" is intended to mean a stable 9- or 10-membered carbocyclic ring system that contains two fused rings and consists of carbon atoms. Of the two fused rings, one ring is a benzo ring fused to a second ring; and the second ring is a 5- or 6-membered carbon ring which is saturated, partially unsaturated, or unsaturated. The bicyclic carbocyclic group may be attached to its pendant group at any carbon atom which results in a stable structure. The bicyclic carbocyclic group described herein may be substituted on any carbon if the resulting compound is stable. Examples of a bicyclic carbocyclic group are, but not limited to, naphthyl, 1,2-dihydronaphthyl, 1,2,3,4-tetrahydronaphthyl, and indanyl.

As used herein, the term "aryl", as employed herein alone or as part of another group, refers to monocyclic or polycyclic (including bicyclic and tricyclic) aromatic hydrocarbons, including, for example, phenyl, naphthyl, anthracenyl, and phenanthranyl. Aryl moieties are well known and described, for example, in Lewis, R.J., ed., Hawley's Condensed Chemical Dictionary, 13th Edition, John Wiley & Sons, Inc., New York (1997). In one embodiment, the term "aryl" denotes monocyclic and bicyclic aromatic groups containing 6 to 10 carbons in the ring portion (such as phenyl or naphthyl including 1-naphthyl and 2-naphthyl). For example, "C₆ or C₁₀ aryl" or "C₆₋₁₀ aryl" refers to phenyl and naphthyl. Unless otherwise specified, "aryl", "C₆ or C₁₀ aryl", "C₆₋₁₀ aryl", or "aromatic residue" may be unsubstituted or substituted with 1 to 5 groups, preferably 1 to 3 groups, selected from OH, OCH₃, Cl, F, Br, I, CN, NO₂, NH₂, N(CH₂)H, N(CH₃)₂, CF₃, OCF₃, C(=O)CH₃, SCH₃, S(=O)CH₃, S(=O)₂CH₃, CH₃, CH₂CH₃, CO₂H, and CO₂CH₃.

The term "benzyl", as used herein, refers to a methyl group on which one of the hydrogen atoms is replaced by a phenyl group, wherein said phenyl group may optionally be substituted with 1 to 5 groups, preferably 1 to 3 groups, OH, OCH₃, Cl, F, Br, I, CN, NO₂, NH₂, N(CH₃)H, N(CH₃)₂, CF₃, OCF₃, C(=O)CH₃, SCH₃, S(=O)CH₃, S(=O)₂CH₃, CH₃, CH₂CH₃, CO₂H, and CO₂CH₃.

As used herein, the term "heterocycle", "heterocyclyl", or "heterocyclic group" is intended to mean a stable 3-, 4-, 5-, 6-, or 7-membered monocyclic or 5-, 6-, 7-, 8-, 9-, 10-, 11-, 12-, 13-, or 14-membered polycyclic (including bicyclic and tricyclic) heterocyclic ring that is saturated, or partially unsaturated, and that contains carbon atoms and 1, 2, 3 or 4 heteroatoms independently selected from N, O and S; and including any polycyclic group in which any of the above-defined heterocyclic rings is fused to a carbocyclic or an aryl (e.g., benzene) ring. That is, the term "heterocycle", "heterocyclyl", or "heterocyclic group" includes non-aromatic ring systems, such as heterocycloalkyl and heterocycloalkenyl. The nitrogen and sulfur heteroatoms may optionally be oxidized (*i.e*., N→O and S(O)ₚ, wherein p is 0, 1 or 2). The nitrogen atom may be substituted or unsubstituted (*i.e*., N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocycle may optionally be quaternized. It is preferred that when the total number of S and O atoms in the heterocycle exceeds 1, then these heteroatoms are not adjacent to one another. It is preferred that the total number of S and O atoms in the heterocycle is not more than 1. Examples of hetercyclyl include, without limitation, azetidinyl, piperazinyl, piperidinyl, piperidonyl, piperonyl, pyranyl, morpholinyl, tetrahydrofuranyl, tetrahydroisoquinolinyl, tetrahydroquinolinyl, morpholinyl, dihydrofuro[2,3-*b*]tetrahydrofuran.

As used herein, the term "bicyclic heterocycle" or "bicyclic heterocyclic group" is intended to mean a stable 9- or 10-membered heterocyclic ring system which contains two fused rings and consists of carbon atoms and 1, 2, 3, or 4 heteroatoms independently selected from N, O and S. Of the two fused rings, one ring is a 5- or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocycle, a 6-membered heterocycle or a carbocycle (provided the first ring is not benzo when the second ring is a carbocycle).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocycle exceeds 1, then these heteroatoms are not adjacent to one another. It is preferred that the total number of S and O atoms in the heterocycle is not more than 1. Examples of a bicyclic heterocyclic group are, but not limited to, 1,2,3,4-tetrahydroquinolinyl, 1,2,3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl, and 1,2,3,4-tetrahydro-quinazolinyl.

Bridged rings are also included in the definition of heterocycle. A bridged ring occurs when one or more atoms (*i.e*., C, O, N, or S) link two non-adjacent carbon or nitrogen atoms. Examples of bridged rings include, but are not limited to, one carbon atom, two carbon atoms, one nitrogen atom, two nitrogen atoms, and a carbon-nitrogen group. It is noted that a bridge always converts a monocyclic ring into a tricyclic ring. When a ring is bridged, the substituents recited for the ring may also be present on the bridge.

As used herein, the term "heteroaryl" is intended to mean stable monocyclic and polycyclic (including bicyclic and tricyclic) aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted (*i.e*., N or NR wherein R is H or another substituent, if defined). The nitrogen and sulfur heteroatoms may optionally be oxidized (*i.e*., N→O and S(O)ₚ, wherein p is 0, 1 or 2).

Examples of heteroaryl also include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, furanyl, furazanyl, imidazolidinyl, imidazolinyl, imidazolyl, 1*H*-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathianyl, phenoxazinyl, phthalazinyl, pteridinyl, purinyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4*H*-quinolizinyl, quinoxalinyl, quinuclidinyl, tetrazolyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

Examples of 5- to 10-membered heteroaryl include, but are not limited to, pyridinyl, furanyl, thienyl, pyrazolyl, imidazolyl, imidazolidinyl, indolyl, tetrazolyl, isoxazolyl, oxazolyl, oxadiazolyl, oxazolidinyl, thiadiazinyl, thiadiazolyl, thiazolyl, triazinyl, triazolyl, benzimidazolyl, 1*H*-indazolyl, benzofuranyl, benzothiofuranyl, benztetrazolyl, benzotriazolyl, benzisoxazolyl, benzoxazolyl, oxindolyl, benzoxazolinyl, benzthiazolyl, benzisothiazolyl, isatinoyl, isoquinolinyl, octahydroisoquinolinyl, isoxazolopyridinyl, quinazolinyl, quinolinyl, isothiazolopyridinyl, thiazolopyridinyl, oxazolopyridinyl, imidazolopyridinyl, and pyrazolopyridinyl. Examples of 5- to 6-membered heteroaryl include, but are not limited to, pyridinyl, furanyl, thienyl, pyrrolyl, pyrazolyl, pyrazinyl, imidazolyl, imidazolidinyl, indolyl, tetrazolyl, isoxazolyl, oxazolyl, oxadiazolyl, oxazolidinyl, thiadiazinyl, thiadiazolyl, thiazolyl, triazinyl, and triazolyl. In some embodiments, the heteroaryl are selected from benzthiazolyl, imidazolpyridinyl, pyrrolopyridinyl, quinolinyl, and indolyl.

Unless otherwise indicated, "carbocyclyl" or "heterocyclyl" includes one to three additional rings fused to the carbocyclic ring or the heterocyclic ring (such as aryl, cycloalkyl, heteroaryl or cycloheteroalkyl rings), for example, 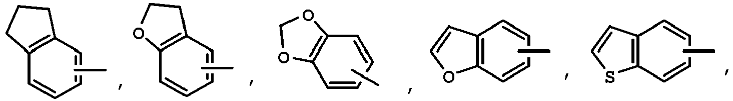 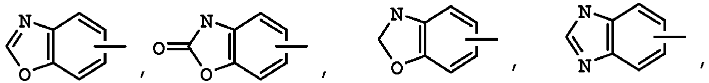 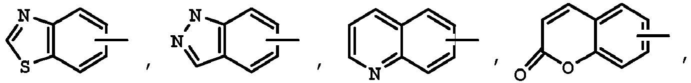 and may be optionally substituted through available carbon or nitrogen atoms (as applicable) with 1, 2, or 3 groups selected from hydrogen, halo, haloalkyl, alkyl, haloalkyl, alkoxy, haloalkoxy, alkenyl, trifluoromethyl, trifluoromethoxy, alkynyl, cycloalkyl-alkyl, cycloheteroalkyl, cycloheteroalkylalkyl, aryl, heteroaryl, arylalkyl, aryloxy, aryloxyalkyl, arylalkoxy, alkoxycarbonyl, arylcarbonyl, arylalkenyl, aminocarbonylaryl, arylthio, arylsulfinyl, arylazo, heteroarylalkyl, heteroarylalkenyl, heteroarylheteroaryl, heteroaryloxy, hydroxy, nitro, cyano, thiol, alkylthio, arylthio, heteroarylthio, arylthioalkyl, alkoxyarylthio, alkylcarbonyl, arylcarbonyl, alkylaminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino and arylsulfonaminocarbonyl and/or any of the alkyl substituents set out herein.

When any of the terms alkyl, alkenyl, alkynyl, cycloalkyl, carbocyclyl, heterocyclyl, aryl, and heteroaryl are used as part of another group, the number of carbon atoms and ring members are the same as those defined in the terms by themselves. For example, alkoxy, haloalkoxy, alkylamino, haloalkyl, hydroxyalkyl, aminoalkyl, haloalkoxy, alkoxyalkoxy, haloalkylamino, alkoxyalkylamino, haloalkoxyalkylamino, alkylthio, and the like each independently contains the number of carbon atoms which are the same as defined for the term "alkyl", such as 1 to 4 carbon atoms, 1 to 6 carbon atoms, 1 to 10 carbon atoms, etc. Similarly, cycloalkoxy, heterocyclyloxy, cycloalkylamino, heterocyclylamino, aralkylamino, arylamino, aryloxy, aralkyloxy, heteroaryloxy, heteroarylalkyloxy, and the like each indepdently contains ring members which are the same as defined for the terms "cycloalkyl", "heterocyclyl", "aryl", and "heteroaryl", such as 3 to 6-membered, 4 to 7-membered, 6 to 10-membered, 5 to 10-membered, 5 or 6-membered, etc.

In accordance with a convention used in the art, a bond pointing to a bold line, such as 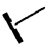 as used in structural formulas herein, depicts the bond that is the point of attachment of the moiety or substituent to the core or backbone structure.

In accordance with a convention used in the art, a wavy or squiggly bond in a structural formula, such as 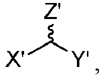 is used to depict a stereogenic center of the carbon atom to which X', Y', and Z' are attached and is intended to represent both enantiomers in a single figure. That is, a structural formula with such as wavy bond denotes each of the enantiomers individually, such as 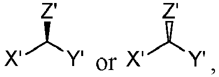 as well as a racemic mixture thereof. When a wavy or squiggly bond is attached to a double bond (such as C=C or C=N) moiety, it includes *cis-* or *trans-* (or *E*- and *Z*-) geometric isomers or a mixture thereof.

It is understood herein that if a carbocyclic or heterocyclic moiety may be bonded or otherwise attached to a designated substrate through differing ring atoms without denoting a specific point of attachment, then all possible points are intended, whether through a carbon atom or, for example, a trivalent nitrogen atom. For example, the term "pyridyl" means 2-, 3- or 4-pyridyl, the term "thienyl" means 2- or 3-thienyl, and so forth.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

One skilled in the art will recognize that substituents and other moieties of the compounds of the present invention should be selected in order to provide a compound which is sufficiently stable to provide a pharmaceutically useful compound which can be formulated into an acceptably stable pharmaceutical composition. Compounds of the present invention which have such stability are contemplated as falling within the scope of the present invention.

The term "counter ion" is used to represent a negatively charged species such as chloride, bromide, hydroxide, acetate, and sulfate. The term "metal ion" refers to alkali metal ions such as sodium, potassium or lithium and alkaline earth metal ions such as magnesium and calcium, as well as zinc and aluminum.

As referred to herein, the term "substituted" means that at least one hydrogen atom (attached to carbon atom or heteroatom) is replaced with a non-hydrogen group, provided that normal valencies are maintained and that the substitution results in a stable compound. When a substituent is oxo (*i.e*., =O), then 2 hydrogens on the atom are replaced. Oxo substituents are not present on aromatic moieties. When a ring system (*e.g*., carbocyclic or heterocyclic) is said to be substituted with a carbonyl group or a double bond, it is intended that the carbonyl group or double bond be part (*i.e*., within) of the ring. Ring double bonds, as used herein, are double bonds that are formed between two adjacent ring atoms (*e.g*., C=C, C=N, or N=N). The term "substituted" in reference to alkyl, cycloalkyl, heteroalkyl, cycloheteroalkyl, alkylene, aryl, arylalkyl, heteroaryl, heteroarylalkyl, carbocyclyl, and heterocyclyl, means alkyl, cycloalkyl, heteroalkyl, cycloheteroalkyl, alkylene, aryl, arylalkyl, heteroaryl, heteroarylalkyl, carbocyclyl, and heterocyclyl, respectively, in which one or more hydrogen atoms, which are attached to either carbon or heteroatom, are each independently replaced with one or more non-hydrogen substituent(s).

In cases wherein there are nitrogen atoms (*e.g*., amines) on compounds of the present invention, these may be converted to N-oxides by treatment with an oxidizing agent (*e.g*., mCPBA and/or hydrogen peroxides) to afford other compounds of this invention. Thus, shown and claimed nitrogen atoms are considered to cover both the shown nitrogen and its N-oxide (N→O) derivative.

When any variable occurs more than one time in any constituent or formula for a compound, its definition at each occurrence is independent of its definition at every other occurrence. Thus, for example, if a group is shown to be substituted with 0, 1, 2, or 3 R groups, then said group be unsubstituted when it is substituted with 0 R group, or be substituted with up to three R groups, and at each occurrence R is selected independently from the definition of R.

Also, combinations of substituents and/or variables are permissible only if such combinations result in stable compounds.

As used herein, the term "tautomer" refers to each of two or more isomers of a compound that exist together in equilibrium, and are readily interchanged by migration of an atom or group within the molecule. For example, one skilled in the art would readily understand that a 1,2,3-triazole exists in two tautomeric forms as defined above:

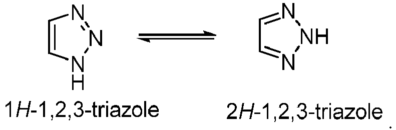

Thus, this disclosure is intended to cover all possible tautomers even when a structure depicts only one of them.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

The compounds of the present invention can be present as salts, which are also within the scope of this invention. Pharmaceutically acceptable salts are preferred. As used herein, "pharmaceutically acceptable salts" refer to derivatives of the disclosed compounds wherein the parent compound is modified by making acid or base salts thereof. The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 18th Edition, Mack Publishing Company, Easton, PA (1990).

If the compounds of the present invention have, for example, at least one basic center, they can form acid addition salts. These are formed, for example, with strong inorganic acids, such as mineral acids, for example sulfuric acid, phosphoric acid or a hydrohalic acid, with organic carboxylic acids, such as alkanecarboxylic acids of 1 to 4 carbon atoms, for example acetic acid, which are unsubstituted or substituted, for example, by halogen as chloroacetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, (for example aspartic or glutamic acid or lysine or arginine), or benzoic acid, or with organic sulfonic acids, such as (C₁-C₄) alkyl or arylsulfonic acids which are unsubstituted or substituted, for example by halogen, for example methyl- or p-toluene- sulfonic acid. Corresponding acid addition salts can also be formed having, if desired, an additionally present basic center. The compounds of the present invention having at least one acid group (for example COOH) can also form salts with bases. Suitable salts with bases are, for example, metal salts, such as alkali metal or alkaline earth metal salts, for example sodium, potassium or magnesium salts, or salts with ammonia or an organic amine, such as morpholine, thiomorpholine, piperidine, pyrrolidine, a mono, di or tri-lower alkylamine, for example ethyl, tert-butyl, diethyl, diisopropyl, triethyl, tributyl or dimethyl-propylamine, or a mono, di or trihydroxy lower alkylamine, for example mono, di or triethanolamine. Corresponding internal salts may furthermore be formed. Salts which are unsuitable for pharmaceutical uses but which can be employed, for example, for the isolation or purification of free compounds of the present invention, or their pharmaceutically acceptable salts, are also included.

Preferred salts of the compounds of the present invention which contain a basic group include monohydrochloride, hydrogensulfate, methanesulfonate, phosphate, nitrate or acetate.

Preferred salts of the compounds of the present invention which contain an acid group include sodium, potassium and magnesium salts and pharmaceutically acceptable organic amines.

The present invention is intended to include all isotopes of atoms occurring in the present compounds. Isotopes include those atoms having the same atomic number but different mass numbers. By way of general example and without limitation, isotopes of hydrogen include deuterium and tritium. Deuterium has one proton and one neutron in its nucleus and that has twice the mass of ordinary hydrogen. Deuterium can be represented by symbols such as "²H" or "D". The term "deuterated" herein, by itself or used to modify a compound or group, refers to replacement of one or more hydrogen atom(s), which is attached to carbon(s), with a deuterium atom. Isotopes of carbon include ¹³C and ¹⁴C.

Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds have a variety of potential uses, *e.g*., as standards and reagents in determining the ability of a potential pharmaceutical compound to bind to target proteins or receptors, or for imaging compounds of this invention bound to biological receptors *in vivo* or *in vitro.*

"Stable compound" and "stable structure" are meant to indicate a compound that is sufficiently robust to survive isolation to a useful degree of purity from a reaction mixture, and formulation into an efficacious therapeutic agent. It is preferred that compounds of the present invention do not contain a N-halo, S(O)₂H, or S(O)H group.

The term "solvate" means a physical association of a compound of this invention with one or more solvent molecules, whether organic or inorganic. This physical association includes hydrogen bonding. In certain instances the solvate will be capable of isolation, for example, when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates. Methods of solvation are generally known in the art.

### ABBREVIATIONS

Abbreviations as used herein, are defined as follows: "1 x" for once, "2 x" for twice, "3 x" for thrice, " °C" for degrees Celsius, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "mmol" for millimole or millimoles, "min" for minute or minutes, "h" for hour or hours, "rt" for room temperature, "RT" for retention time, "RBF" for round bottom flask, "atm" for atmosphere, "psi" for pounds per square inch, "conc." for concentrate, "RCM" for ring-closing metathesis, "sat" or "sat'd " for saturated, "SFC" for supercritical fluid chromatography "MW" for molecular weight, "mp" for melting point, "ee" for enantiomeric excess, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "¹H" for proton, "δ" for delta, "s" for singlet, "d" for doublet, "t" for triplet, "q" for quartet, "m" for multiplet, "br" for broad, "Hz" for hertz, and "α" "β", "γ", "R", "S", "E", and "Z" are stereochemical designations familiar to one skilled in the art.
- Me: methyl
- Et: ethyl
- Pr: propyl
- i-Pr: isopropyl
- Bu: butyl
- *i*-Bu: isobutyl
- *t*-Bu: *tert*-butyl
- Ph: phenyl
- Bn: benzyl
- Boc or BOC: *tert*-butyloxycarbonyl
- Boc₂O: di-*tert*-butyl dicarbonate
- AcOH or HOAc: acetic acid
- AlCl₃: aluminum trichloride
- AIBN: Azobis-isobutyronitrile
- BBr₃: boron tribromide
- BCl₃: boron trichloride
- BEMP: 2-*tert*-butylimino-2-diethylamino-1,3-dimethylperhydro-1,3,2-diazaphosphorine
- BOP reagent: benzotriazol-1-yloxytris(dimethylamino)phosphonium hexafluorophosphate
- Burgess reagent: 1-methoxy-N-triethylammoniosulfonyl-methanimidate
- CB₂: carbobenzyloxy
- DCM or CH₂Cl₂: dichloromethane
- CH₃CN or ACN: acetonitrile
- CDCl₃: deutero-chloroform
- CHCl₃: chloroform
- mCPBA or m-CPBA: *meta*-chloroperbenzoic acid
- Cs₂CO₃: cesium carbonate
- Cu(OAc)₂: copper (II) acetate
- Cy₂NMe: N-cyclohexyl-N-methylcyclohexanamine
- DBU: 1,8-diazabicyclo[5.4.0]undec-7-ene
- DCE: 1,2 dichloroethane
- DEA: Diethylamine
- DEAD: Diethyl azodicarboxylate
- Dess-Martin: 1,1,1-tris(acetyloxy)-1,1-dihydro-1,2-benziodoxol-3-(1H)-one
- DIAD: Diisopropyl azodicarboxylate
- DIBALH: Diisobutyl aluminum hydride
- DIC or DIPCDI: diisopropylcarbodiimide
- DIEA, DIPEA or Hunig's base: diisopropylethylamine
- DMAP: 4-dimethylaminopyridine
- DME: 1,2-dimethoxyethane
- DMF: dimethyl formamide
- DMSO: dimethyl sulfoxide
- cDNA: complementary DNA
- Dppp: (*R*)-(+)-1,2-bis(diphenylphosphino)propane
- DuPhos: (+)-1,2-bis((2 S, 5 S)-2, 5-diethylphospholano)b enzene
- EDC: *N*-(3-dimthylaminopropyl)-*N'*-ethylcarbodiimide
- EDCI: *N*-(3-dimthylaminopropyl)-*N'*-ethylcarbodiimide hydrochloride
- EDTA: ethylenediaminetetraacetic acid
- (*S*,*S*)-EtDuPhosRh(I): (+)-1,2-bis((2S,5S)-2,5-diethylphospholano)benzene(1,5-cyclooctadiene)rhodium(I) trifluoromethanesulfonate
- Et₃N or TEA: triethylamine
- EtOAc: ethyl acetate
- Et₂O: diethyl ether
- EtOH: ethanol
- GMF: glass microfiber filter
- Grubbs II: (1,3-bis(2,4,6-trimethylphenyl)-2-imidazolidinylidene)dichloro (phenylmethylene)(triycyclohexylphosphine)ruthenium
- HCl: hydrochloric acid
- HATU: O-(7-azabenzotriazol-1-yl)-N,N,N',N'-tetramethyluronium hexafluorophosphate
- HEPES: 4-(2-hydroxyethyl)piperaxine-1-ethanesulfonic acid
- Hex: hexane
- HOBt or HOBT: 1-hydroxybenzotriazole
- H₂O₂: hydrogen peroxide
- IBX: 2-iodoxybenzoic acid
- H₂SO₄: sulfuric acid
- Jones reagent: CrO₃ in aqueous H₂SO₄, 2 M solution
- K₂CO₃: potassium carbonate
- K₂HPO₄: potassium phosphate dibasic (potassium hydrogen phosphate)
- KOAc: potassium acetate
- K₃PO₄: potassium phosphate tribasic
- LAH: lithium aluminum hydride
- LG: leaving group
- LiOH: lithium hydroxide
- MeOH: methanol
- MgSO₄: magnesium sulfate
- MsOH or MSA: methylsulfonic acid/methanesulfonic acid
- NaCl: sodium chloride
- NaH: sodium hydride
- NaHCO₃: sodium bicarbonate
- Na₂CO₃: sodium carbonate
- NaOH: sodium hydroxide
- Na₂SO₃: sodium sulfite
- Na₂SO₄: sodium sulfate
- NBS: N-bromosuccinimide
- NCS: N-chlorosuccinimide
- NH₃: ammonia
- NH₄Cl: ammonium chloride
- NH₄OH: ammonium hydroxide
- NH₄⁺HCO₂⁻: ammonium formate
- NMM: N-methylmorpholine
- OTf: triflate or trifluoromethanesulfonate
- Pd₂(dba)₃: tris(dibenzylideneacetone)dipalladium(0)
- Pd(OAc)₂: palladium(II) acetate
- Pd/C: palladium on carbon
- Pd(dppf)Cl₂: [1,1'-bis(diphenylphosphino)-ferrocene]dichloropalladium(II)
- Ph₃PCl₂: triphenylphosphine dichloride
- PG: protecting group
- POCl₃: phosphorus oxychloride
- PPTS: pyridinium p-toluenesulfonate
- i-PrOH or IPA: isopropanol
- PS: Polystyrene
- RT or rt: room temperature
- SEM-Cl: 2-(trimethysilyl)ethoxymethyl chloride
- SiO₂: silica oxide

- SnCl₂: tin(II) chloride
- TBAF: tra-*n*-butylammonium fluoride
- TBAI: tetra-*n*-butylammonium iodide
- TFA: trifluoroacetic acid
- THF: tetrahydrofuran
- THP: tetrahydropyran
- TMSCHN₂: Trimethylsilyldiazomethane
- TMSCH₂N₃: Trimethylsilylmethyl azide
- T3P: propane phosphonic acid anhydride
- TRIS: tris (hydroxymethyl) aminomethane
- pTsOH: p-toluenesulfonic acid

### IV. BIOLOGY

Lysophospholipids are membrane-derived bioactive lipid mediators. Lysophospholipids include, but are not limited to, lysophosphatidic acid (1-acyl-2-hydroxy-*sn*-glycero-3-phosphate; LPA), sphingosine 1-phosphate (S1P), lysophosphatidylcholine (LPC), and sphingosylphosphorylcholine (SPC). Lysophospholipids affect fundamental cellular functions that include cellular proliferation, differentiation, survival, migration, adhesion, invasion, and morphogenesis. These functions influence many biological processes that include neurogenesis, angiogenesis, wound healing, immunity, and carcinogenesis.

LPA acts through sets of specific G protein-coupled receptors (GPCRs) in an autocrine and paracrine fashion. LPA binding to its cognate GPCRs (LPA₁, LPA₂, LPA₃, LPA₄, LPA₅, LPA₆) activates intracellular signaling pathways to produce a variety of biological responses.

Lysophospholipids, such as LPA, are quantitatively minor lipid species compared to their major phospholipid counterparts (*e.g*., phosphatidylcholine, phosphatidylethanolamine, and sphingomyelin). LPA has a role as a biological effector molecule, and has a diverse range of physiological actions such as, but not limited to, effects on blood pressure, platelet activation, and smooth muscle contraction, and a variety of cellular effects, which include cell growth, cell rounding, neurite retraction, and actin stress fiber formation and cell migration. The effects of LPA are predominantly receptor mediated.

Activation of the LPA receptors (LPA₁, LPA₂, LPA₃, LPA₄, LPA₅, LPA₆) with LPA mediates a range of downstream signaling cascades. These include, but are not limited to, mitogen-activated protein kinase (MAPK) activation, adenylyl cyclase (AC) inhibition/activation, phospholipase C (PLC) activation/Ca²⁺ mobilization, arachidonic acid release, Akt/PKB activation, and the activation of small GTPases, Rho, ROCK, Rac, and Ras. Other pathways that are affected by LPA receptor activation include, but are not limited to, cyclic adenosine monophosphate (cAMP), cell division cycle 42/GTP-binding protein (Cdc42) , proto-oncogene serine/threonine-protein kinase Raf (c-RAF), proto-oncogene tyrosine-protein kinase Src (c-src), extracellular signal-regulated kinase (ERK), focal adhesion kinase (FAK), guanine nucleotide exchange factor (GEF), glycogen synthase kinase 3b (GSK3b), c-jun amino-terminal kinase (JNK), MEK, myosin light chain II (MLC II), nuclear factor kB (NF-kB), N-methyl-D-aspartate (NMDA) receptor activation, phosphatidylinositol 3-kinase (PI3K), protein kinase A (PKA), protein kinase C (PKC), ras-related C3 botulinum toxin substrate 1 (RAC1). The actual pathway and realized end point are dependent on a range of variables that include receptor usage, cell type, expression level of a receptor or signaling protein, and LPA concentration. Nearly all mammalian cells, tissues and organs co-express several LPA-receptor subtypes, which indicates that LPA receptors signal in a cooperative manner. LPA₁, LPA₂, and LPA₃ share high amino acid sequence similarity.

LPA is produced from activated platelets, activated adipocytes, neuronal cells, and other cell types. Serum LPA is produced by multiple enzymatic pathways that involve monoacylglycerol kinase, phospholipase A₁, secretory phospholipase A₂, and lysophospholipase D (lysoPLD), including autotaxin. Several enzymes are involved in LPA degradation: lysophospholipase, lipid phosphate phosphatase, and LPA acyl transferase such as endophilin. LPA concentrations in human serum are estimated to be 1-5 µM. Serum LPA is bound to albumin, low-density lipoproteins, or other proteins, which possibly protect LPA from rapid degradation. LPA molecular species with different acyl chain lengths and saturation are naturally occurring, including 1-palmitoyl (16:0), 1-palmitoleoyl (16:1), 1-stearoyl (18:0), 1-oleoyl (18:1), 1-linoleoyl (18:2), and 1-arachidonyl (20:4) LPA. Quantitatively minor alkyl LPA has biological activities similar to acyl LPA, and different LPA species activate LPA receptor subtypes with varied efficacies.

### LPA RECEPTORS

LPA₁ (previously called VZG-1/EDG-2/mrec1.3) couples with three types of G proteins, G_{i/o}, G_{q}, and G_{12/13}. Through activation of these G proteins, LPA induces a range of cellular responses through LPA₁ including but not limited to: cell proliferation, serum-response element (SRE) activation, mitogen-activated protein kinase (MAPK) activation, adenylyl cyclase (AC) inhibition, phospholipase C (PLC) activation, Ca²⁺ mobilization, Akt activation, and Rho activation.

Wide expression of LPA₁ is observed in adult mice, with clear presence in testis, brain, heart, lung, small intestine, stomach, spleen, thymus, and skeletal muscle. Similarly, human tissues also express LPA₁; it is present in brain, heart, lung, placenta, colon, small intestine, prostate, testis, ovary, pancreas, spleen, kidney, skeletal muscle, and thymus.

LPA₂ (EDG-4) also couples with three types of G proteins, G_{i/o}, G_{q}, and G_{12/13}, to mediate LPA-induced cellular signaling. Expression of LPA₂ is observed in the testis, kidney, lung, thymus, spleen, and stomach of adult mice and in the human testis, pancreas, prostate, thymus, spleen, and peripheral blood leukocytes. Expression of LPA₂ is upregulated in various cancer cell lines, and several human LPA₂ transcriptional variants with mutations in the 3'-untranslated region have been observed. Targeted deletion of LPA₂ in mice has not shown any obvious phenotypic abnormalities, but has demonstrated a significant loss of normal LPA signaling (*e.g*., PLC activation, Ca²⁺ mobilization, and stress fiber formation) in primary cultures of mouse embryonic fibroblasts (MEFs). Creation of *lpa*1(-/-) *lpa*2 (-/-) double-null mice has revealed that many LPA-induced responses, which include cell proliferation, AC inhibition, PLC activation, Ca²⁺ mobilization, JNK and Akt activation, and stress fiber formation, are absent or severely reduced in double-null MEFs. All these responses, except for AC inhibition (AC inhibition is nearly abolished in LPA₁ (-/-) MEFs), are only partially affected in either LPA₁ (-/-) or LPA₂ (-/-) MEFs. LPA₂ contributes to normal LPA-mediated signaling responses in at least some cell types (Choi et al, Biochemica et Biophysica Acta 2008, 1781, p531-539).

LPA₃ (EDG-7) is distinct from LPA₁ and LPA₂ in its ability to couple with G_{i/o} and G_{q} but not G_{12/13} and is much less responsive to LPA species with saturated acyl chains. LPA₃ can mediate pleiotropic LPA-induced signaling that includes PLC activation, Ca²⁺ mobilization, AC inhibition/activation, and MAPK activation. Overexpression of LPA₃ in neuroblastoma cells leads to neurite elongation, whereas that of LPA₁ or LPA₂ results in neurite retraction and cell rounding when stimulated with LPA. Expression of LPA₃ is observed in adult mouse testis, kidney, lung, small intestine, heart, thymus, and brain. In humans, it is found in the heart, pancreas, prostate, testis, lung, ovary, and brain (frontal cortex, hippocampus, and amygdala).

LPA₄ (p2y₉/GPR23) is of divergent sequence compared to LPA₁, LPA₂, and LPA₃ with closer similarity to the platelet-activating factor (PAF) receptor. LPA₄ mediates LPA induced Ca²⁺ mobilization and cAMP accumulation, and functional coupling to the G protein Gs for AC activation, as well as coupling to other G proteins. The LPA₄ gene is expressed in the ovary, pancreas, thymus, kidney and skeletal muscle.

LPA₅ (GPR92) is a member of the purinocluster of GPCRs and is structurally most closely related to LPA₄. LPA₅ is expressed in human heart, placenta, spleen, brain, lung and gut. LPA₅ also shows very high expression in the CD8+ lymphocyte compartment of the gastrointestinal tract.

LPA₆ (p2y5) is a member of the purinocluster of GPCRs and is structurally most closely related to LPA₄. LPA₆ is an LPA receptor coupled to the G12/13-Rho signaling pathways and is expressed in the inner root sheaths of human hair follicles.

### Illustrative Biological Activity

### Wound Healing

Normal wound healing occurs by a highly coordinated sequence of events in which cellular, soluble factors and matrix components act in concert to repair the injury. The healing response can be described as taking place in four broad, overlapping phases-hemostasis, inflammation, proliferation, and remodeling. Many growth factors and cytokines are released into a wound site to initiate and perpetuate wound healing processes.

When wounded, damaged blood vessels activate platelets. The activated platelets play pivotal roles in subsequent repair processes by releasing bioactive mediators to induce cell proliferation, cell migration, blood coagulation, and angiogenesis. LPA is one such mediator that is released from activated platelets; this induces platelet aggregation along with mitogenic/migration effects on the surrounding cells, such as endothelial cells, smooth muscle cells, fibroblasts, and keratinocytes.

Topical application of LPA to cutaneous wounds in mice promotes repair processes (wound closure and increased neoepithelial thickness) by increasing cell proliferation/ migration without affecting secondary inflammation.

Activation of dermal fibroblasts by growth factors and cytokines leads to their subsequent migration from the edges of the wound into the provisional matrix formed by the fibrin clot whereupon the fibroblasts proliferate and start to restore the dermis by secreting and organizing the characteristic dermal extracellular matrix (ECM). The increasing number of fibroblasts within the wound and continuous precipitation of ECM enhances matrix rigidity by applying small tractional forces to the newly formed granulation tissue. The increase in mechanical stress, in conjunction with transforming growth factor β (TGFβ), induces α-smooth muscle actin (α-SMA) expression and the subsequent transformation of fibroblasts into myofibroblasts. Myofibroblasts facilitate granulation tissue remodeling via myofibroblast contraction and through the production of ECM components.

LPA regulates many important functions of fibroblasts in wound healing, including proliferation, migration, differentiation and contraction. Fibroblast proliferation is required in wound healing in order to fill an open wound. In contrast, fibrosis is characterized by intense proliferation and accumulation of myofibroblasts that actively synthesize ECM and proinflammatory cytokines. LPA can either increase or suppress the proliferation of cell types important in wound healing, such as epithelial and endothelial cells (EC),macrophages, keratinocytes, and fibroblasts. A role for LPA₁ in LPA-induced proliferation was provided by the observation that LPA-stimulated proliferation of fibroblasts isolated from LPA₁ receptor null mice was attenuated (Mills et al, Nat Rev. Cancer 2003; 3: 582-591). LPA induces cytoskeletal changes that are integral to fibroblast adhesion, migration, differentiation and contraction.

### Fibrosis

Tissue injury initiates a complex series of host wound-healing responses; if successful, these responses restore normal tissue structure and function. If not, these responses can lead to tissue fibrosis and loss of function.

For the majority of organs and tissues the development of fibrosis involves a multitude of events and factors. Molecules involved in the development of fibrosis include proteins or peptides (profibrotic cytokines, chemokines, metalloproteinases etc.) and phospholipids. Phospholipids involved in the development of fibrosis include platelet activating factor (PAF), phosphatidyl choline, sphingosine-1 phosphate (S1P) and lysophosphatidic acid (LPA).

A number of muscular dystrophies are characterized by a progressive weakness and wasting of musculature, and by extensive fibrosis. It has been shown that LPA treatment of cultured myoblasts induced significant expression of connective tissue growth factor (CTGF). CTGF subsequently induces collagen, fibronectin and integrin expression and induces dedifferentiation of these myoblasts. Treatment of a variety of cell types with LPA induces reproducible and high level induction of CTGF (J.P. Pradere, et al., LPA1 receptor activation promotes renal interstitial fibrosis, J. Am. Soc. Nephrol. 18 (2007) 3110-3118; N. Wiedmaier, et al., Int J Med Microbiol; 298(3-4):231-43, 2008). CTGF is a profibrotic cytokine, signaling down-stream and in parallel with TGFβ.

CTGF expression by gingival epithelial cells, which are involved in the development of gingival fibromatosis, was found to be exacerbated by LPA treatment (A. Kantarci, et al., J. Pathol. 210 (2006) 59-66).

LPA is associated with the progression of liver fibrosis. *In vitro,* LPA induces stellate cell and hepatocyte proliferation. These activated cells are the main cell type responsible for the accumulation of ECM in the liver. Furthermore, LPA plasma levels rise during CCl₄-induced liver fibrosis in rodents, or in hepatitis C virus-induced liver fibrosis in humans (N. Watanabe, et al., Plasma lysophosphatidic acid level and serum autotaxin activity are increased in liver injury in rats in relation to its severity, Life Sci. 81 (2007) 1009-1015; N.Watanabe, et al., J. Clin. Gastroenterol. 41 (2007) 616-623).

An increase of phospholipid concentrations in the bronchoalveolar lavage fluid in rabbits and rodents injected with bleomycin has been reported (K. Kuroda, et al., Phospholipid concentration in lung lavage fluid as biomarker for pulmonary fibrosis, Inhal. Toxicol. 18 (2006) 389-393; K. Yasuda, et al., Lung 172 (1994) 91-102).

LPA is associated with heart disease and mycocardial remodeling. Serum LPA levels are increased after myocardial infarction in patients and LPA stimulates rat cardiac fibroblast proliferation and collagen production (Chen et al. FEES Lett. 2006 Aug 21;580(19):4737-45).

### Pulmonary Fibrosis

In the lung, aberrant wound healing responses to injury contribute to the pathogenesis of fibrotic lung diseases. Fibrotic lung diseases, such as idiopathic pulmonary fibrosis (IPF), are associated with high morbidity and mortality.

LPA is an important mediator of fibroblast recruitment in pulmonary fibrosis. LPA and LPA₁ play key pathogenic roles in pulmonary fibrosis. Fibroblast chemoattractant activity plays an important role in the lungs in patients with pulmonary fibrosis. Profibrotic effects of LPA₁-receptor stimulation is explained by LPA₁-receptor-mediated vascular leakage and increased fibroblast recruitment, both profibrotic events. The LPA-LPA₁ pathway has a role in mediating fibroblast migration and vascular leakage in IPF. The end result is the aberrant healing process that characterizes this fibrotic condition.

The LPA₁ receptor is the LPA receptor most highly expressed on fibroblasts obtained from patients with IPF. Furthermore, BAL obtained from IPF patients induced chemotaxis of human foetal lung fibroblasts that was blocked by the dual LPA₁- LPA₃ receptor antagonist Ki16425. In an experimental bleomycin-induced lung injury mouse model, it was shown that LPA levels were high in bronchoalveolar lavage samples compared with unexposed controls. LPA₁ knockout mice are protected from fibrosis after bleomycin challenge with reduced fibroblast accumulation and vascular leakage. In human subjects with IPF, high LPA levels were observed in bronchoalveolar lavage samples compared with healthy controls. Increased fibroblast chemotactic activity in these samples was inhibited by the Ki 16425 indicating that fibroblast migration is mediated by the LPA-LPA receptor(s) pathway (Tager et al. Nature Medicine, 2008, 14, 45-54).

The LPA-LPA₁ pathway is crucial in fibroblast recruitment and vascular leakage in pulmonary fibrosis.

Activation of latent TGF-β by the αvβ6 integrin plays a critical role in the development of lung injury and fibrosis (Munger et al. Cell, vol. 96, 319-328, 1999). LPA induces αvβ6-mediated TGF-β activation on human lung epithelial cells (Xu et al. Am. J. Pathology, 2009, 174, 1264-1279). The LPA-induced αvβ6-mediated TGF-β activation is mediated by the LPA2 receptor. Expression of the LPA2 receptor is increased in epithelial cells and mesenchymal cells in areas of lung fibrosis from IPF patients compared to normal human lung tissue. The LPA-LPA2 pathway contributes to the activation of the TGF-β pathway in pulmonary fibrosis. In some embodiments, compounds that inhibit LPA2 show efficacy in the treatment of lung fibrosis. In some embodiments, compounds that inhibit both LPA1 and LPA2 show improved efficacy in the treatment of lung fibrosis compared to compounds which inhibit only LPA1 or LPA2.

### Renal Fibrosis

LPA and LPA₁ are involved in the etiology of kidney fibrosis. LPA has effects on both proliferation and contraction of glomerular mesangial cells and thus has been implicated in proliferative glomerulonephritis (C.N. Inoue, et al., Clin. Sci. (Colch.) 1999, 96, 431-436). In an animal model of renal fibrosis [unilateral ureteral obstruction (UUO)], it was found that renal LPA receptors are expressed under basal conditions with an expression order of LPA₂>LPA₃=LPA₁>>LPA₄. This model mimics in an accelerated manner the development of renal fibrosis including renal inflammation, fibroblast activation and accumulation of extracellular matrix in the tubulointerstitium. UUO significantly induced LPA₁-receptor expression. This was paralleled by renal LPA production (3.3 fold increase) in conditioned media from kidney explants. Contra-lateral kidneys exhibited no significant changes in LPA release and LPA-receptors expression. This shows that a prerequisite for an action of LPA in fibrosis is met: production of a ligand (LPA) and induction of one of its receptors (the LPA₁ receptor) (J.P. Pradere et al., Biochimica et Biophysica Acta, 2008, 1781, 582-587).

In mice where the LPA₁ receptor was knocked out (LPA₁ (-/-), the development of renal fibrosis was significantly attenuated. UUO mice treated with the LPA receptor antagonist Ki 16425 closely resembled the profile of LPA₁ (-/-) mice.

LPA can participate in intraperitonial accumulation of monocyte/macrophages and LPA can induce expression of the profibrotic cytokine CTGF in primary cultures of human fibroblasts (J.S. Koh,et al., J. Clin. Invest., 1998, 102, 716-727).

LPA treatment of a mouse epithelial renal cell line, MCT, induced a rapid increase in the expression of the profibrotic cytokine CTGF. CTGF plays a crucial role in UUO-induced tubulointerstitial fibrosis (TIF), and is involved in the profibrotic activity of TGFβ. This induction was almost completely suppressed by co-treatment with the LPA-receptor antagonist Ki 16425. The profibrotic activity of LPA in kidney may result from a direct action of LPA on kidney cells involving induction of CTGF.

### Hepatic fibrosis

LPA is implicated in liver disease and fibrosis. Plasma LPA levels and serum autotaxin (enzyme responsible for LPA production) are elevated in hepatitis patients and animal models of liver injury in correlation with increased fibrosis. LPA also regulates liver cell function. LPA₁ and LPA₂ receptors are expressed by mouse hepatic stellate cells and LPA stimulates migration of hepatic myofibroblasts.

### Ocular Fibrosis

LPA is in involved in wound healing in the eye. LPA₁ and LPA₃ receptors are detectable in the normal rabbit corneal epithelial cells, keratocytes and endothelial cells and LPA₁ and LPA₃ expression are increased in corneal epithelial cells following injury.

LPA and its homologues are present in the aqueous humor and the lacrimal gland fluid of the rabbit eye and these levels are increased in a rabbit corneal injury model.

LPA induces actin stress fiber formation in rabbit corneal endothelial and epithelial cells and promotes contraction corneal fibroblasts. LPA also stimulates proliferation of human retinal pigmented epithelial cells

### Cardiac fibrosis

LPA is implicated in myocardial infarction and cardiac fibrosis. Serum LPA levels are increased in patients following mycocardial infarction (MI) and LPA stimulates proliferation and collagen production (fibrosis) by rat cardiac fibroblasts. Both LPA1 and LPA3 receptors are highly expressed in human heart tissue.

### Treatment of Fibrosis

A compound of the present invention, or a pharmaceutically acceptable salt thereof, may be used in a method to treat or prevent fibrosis in a mammal. A compound of Formulas (I), or a pharmaceutically acceptable salt thereof, may be used in a method to treat fibrosis of an organ or tissue in a mammal. A compound of the invention may be used in a method for preventing a fibrosis condition in a mammal, the method comprising administering to the mammal at risk of developing one or more fibrosis conditions a therapeutically effective amount of a compound of Formulas (I), or a pharmaceutically acceptable salt thereof. The mammal may have been exposed to one or more environmental conditions that are known to increase the risk of fibrosis of an organ or tissue. The mammal may have been exposed to one or more environmental conditions that are known to increase the risk of lung, liver or kidney fibrosis. The mammal may have a genetic predisposition of developing fibrosis of an organ or tissue. A compound of the present invention, or a pharmaceutically acceptable salt thereof, may be administered to a mammal to prevent or minimize scarring following injury. Injury include surgery.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Exemplary diseases, disorders, or conditions that involve fibrosis include, but are not limited to: Lung diseases associated with fibrosis, *e.g*., idiopathic pulmonary fibrosis, pulmonary fibrosis secondary to systemic inflammatory disease such as rheumatoid arthritis, scleroderma, lupus, cryptogenic fibrosing alveolitis, radiation induced fibrosis, chronic obstructive pulmonary disease (COPD), scleroderma, chronic asthma, silicosis, asbestos induced pulmonary or pleural fibrosis, acute lung injury and acute respiratory distress (including bacterial pneumonia induced, trauma induced, viral pneumonia induced, ventilator induced, non-pulmonary sepsis induced, and aspiration induced); Chronic nephropathies associated with injury/fibrosis (kidney fibrosis), *e.g*., glomerulonephritis secondary to systemic inflammatory diseases such as lupus and scleroderma, diabetes, glomerular nephritis, focal segmental glomerular sclerosis, IgA nephropathy, hypertension, allograft and Alport; Gut fibrosis, *e.g*., scleroderma, and radiation induced gut fibrosis; Liver fibrosis, *e.g*., cirrhosis, alcohol induced liver fibrosis, nonalcoholic steatohepatitis (NASH), biliary duct injury, primary biliary cirrhosis, infection or viral induced liver fibrosis (*e.g*., chronic HCV infection), and autoimmune hepatitis; Head and neck fibrosis, *e.g*., radiation induced; Corneal scarring, *e.g*., LASIK (laser-assisted in situ keratomileusis), corneal transplant, and trabeculectomy; Hypertrophic scarring and keloids, *e.g*., burn induced or surgical; and other fibrotic diseases, *e.g*., sarcoidosis, scleroderma, spinal cord injury/fibrosis, myelofibrosis, vascular restenosis, atherosclerosis, arteriosclerosis, Wegener's granulomatosis, mixed connective tissue disease, and Peyronie's disease.

A mammal suffering from one of the following non-limiting exemplary diseases, disorders, or conditions may benefit from therapy with a compound of the present invention, or a pharmaceutically acceptable salt thereof: atherosclerosis, thrombosis, heart disease, vasculitis, formation of scar tissue, restenosis, phlebitis, COPD (chronic obstructive pulmonary disease), pulmonary hypertension, pulmonary fibrosis, pulmonary inflammation, bowel adhesions, bladder fibrosis and cystitis, fibrosis of the nasal passages, sinusitis, inflammation mediated by neutrophils, and fibrosis mediated by fibroblasts.

A compound of the present invention, or a pharmaceutically acceptable salt thereof, may be administered to a mammal with fibrosis of an organ or tissue or with a predisposition of developing fibrosis of an organ or tissue with one or more other agents that are used to treat fibrosis. The one or more agents may include corticosteroids. The one or more agents may include immunosuppressants. The one or more agents may include B-cell antagonists. The one or more agents may include uteroglobin.

A compound of the present invention, or a pharmaceutically acceptable salt thereof, may be used in a method to treat a dermatological disorders in a mammal. The term "dermatological disorder," as used herein refers to a skin disorder. Such dermatological disorders include, but are not limited to, proliferative or inflammatory disorders of the skin such as, atopic dermatitis, bullous disorders, collagenoses, psoriasis, scleroderma, psoriatic lesions, dermatitis, contact dermatitis, eczema, urticaria, rosacea, wound healing, scarring, hypertrophic scarring, keloids, Kawasaki Disease, rosacea, Sjogren-Larsso Syndrome, urticaria. A compound of the present invention, or a pharmaceutically acceptable salt thereof, may be used to treat systemic sclerosis.

### Pain

Since LPA is released following tissue injury, LPA₁ plays an important role in the initiation of neuropathic pain. LPA₁, unlike LPA₂ or LPA₃, is expressed in both dorsal root ganglion (DRG) and dorsal root neurons. Using the antisense oligodeoxynucleotide (AS-ODN) for LPA₁ and LPA₁-null mice, it was found that LPA-induced mechanical allodynia and hyperalgesia is mediated in an LPAi-dependent manner. LPA₁ and downstream Rho-ROCK activation play a role in the initiation of neuropathic pain signaling. Pretreatment with Clostridium botulinum C3 exoenzyme (BoTXC3, Rho inhibitor) or Y-27632 (ROCK inhibitor) completely abolished the allodynia and hyperalgesia in nerve-injured mice. LPA also induced demyelination of the dorsal root, which was prevented by BoTXC3. The dorsal root demyelination by injury was not observed in LPA₁-null mice or AS-ODN injected wild-type mice. LPA signaling appears to induce important neuropathic pain markers such as protein kinase Cγ (PKCy) and a voltage-gated calcium channel α2δ1 subunit (Caα2δ1) in an LPA₁ and Rho-dependent manner (M. Inoue, et al., Initiation of neuropathic pain requires lysophosphatidic acid receptor signaling, Nat. Med. 10 (2004) 712-718).

A compound of the present invention, or a pharmaceutically acceptable salt thereof, may be used in a method for treating pain in a mammal. The pain may be acute pain or chronic pain. The pain may be neuropathic pain.

A compound of the present invention, or a pharmaceutically acceptable salt thereof, may be ued in a method of treating fibromylagia. Fibromyalgia may stem from the formation of fibrous scar tissue in contractile (voluntary) muscles. Fibrosis binds the tissue and inhibits blood flow, resulting in pain.

### Cancer

Lysophospholipid receptor signaling plays a role in the etiology of cancer. Lysophosphatidic acid (LPA) and its G protein-coupled receptors (GPCRs) LPA₁, LPA₂, and/or LPA₃ play a role in the development of several types of cancers. The initiation, progression and metastasis of cancer involve several concurrent and sequential processes including cell proliferation and growth, survival and anti-apoptosis, migration of cells, penetration of foreign cells into defined cellular layers and/or organs, and promotion of angiogenesis. The control of each of these processes by LPA signaling in physiological and pathophysiological conditions underscores the potential therapeutic usefulness of modulating LPA signaling pathways for the treatment of cancer, especially at the level of the LPA receptors or ATX/lysoPLD. Autotaxin (ATX) is a prometastatic enzyme initially isolated from the conditioned medium of human melanoma cells that stimulates a myriad of biological activities, including angiogenesis and the promotion of cell growth, migration, survival, and differentiation through the production of LPA (Mol Cancer Ther 2008;7(10):3352-62).

LPA signals through its own GPCRs leading to activation of multiple downstream effector pathways. Such downstream effector pathways play a role in cancer. LPA and its GPCRs are linked to cancer through major oncogenic signaling pathways.

LPA contributes to tumorigenesis by increasing motility and invasiveness of cells. LPA has been implicated in the initiation or progression of ovarian cancer. LPA is present at significant concentrations (2-80 µM) in the ascitic fluid of ovarian cancer patients. Ovarian cancer cells constitutively produce increased amounts of LPA as compared to normal ovarian surface epithelial cells, the precursor of ovarian epithelial cancer. Elevated LPA levels are also detected in plasma from patients with early-stage ovarian cancers compared with controls. LPA receptors (LPA2 and LPA3) are also overexpressed in ovarian cancer cells as compared to normal ovarian surface epithelial cells. LPA stimulates Cox-2 expression through transcriptional activation and post-transcriptional enhancement of Cox-2 mRNA in ovarian cancer cells. Prostaglandins produced by Cox-2 have been implicated in a number of human cancers and pharmacological inhibition of Cox-2 activity reduces colon cancer development and decreases the size and number of adenomas in patients with familial adenomatous polyposis. LPA has also been implicated in the initiation or progression of prostate cancer, breast cancer, melanoma, head and neck cancer, bowel cancer (colorectal cancer), thyroid cancer and other cancers (Gardell et al, Trends in Molecular Medicine, vol. 12, no. 2, p 65-75, 2006; Ishii et al, Annu. Rev. Biochem, 73, 321-354, 2004; Mills et al., Nat. Rev. Cancer, 3, 582-591, 2003; Murph et al., Biochimica et Biophysica Acta, 1781, 547-557, 2008).

The cellular responses to LPA are mediated through the lysophosphatidic acid receptors. For example, LPA receptors mediate both migration of and invasion by pancreatic cancer cell lines: an antagonist of LPA₁ and LPA₃ (Ki16425) and LPA₁-specific siRNA effectively blocked in vitro migration in response to LPA and peritoneal fluid (ascites) from pancreatic cancer patients; in addition, Ki 16425 blocked the LPA-induced and ascites-induced invasion activity of a highly peritoneal metastatic pancreatic cancer cell line (Yamada et al, J. Biol. Chem., 279, 6595-6605, 2004).

Colorectal carcinoma cell lines show significant expression of LPA₁ mRNA and respond to LPA by cell migration and production of angiogenic factors. Overexpression of LPA receptors has a role in the pathogenesis of thyroid cancer. LPA₃ was originally cloned from prostate cancer cells, concordant with the ability of LPA to induce autocrine proliferation of prostate cancer cells.

LPA has stimulatory roles in cancer progression in many types of cancer. LPA is produced from and induces proliferation of prostate cancer cell lines. LPA induces human colon carcinoma DLD1 cell proliferation, migration, adhesion, and secretion of angiogenic factors through LPA₁ signaling. In other human colon carcinoma cells lines (HT29 and WiDR), LPA enhances cell proliferation and secretion of angiogenic factors. In other colon cancer cell lines, LPA2 and LPA3 receptor activation results in proliferation of the cells. The genetic or pharmacological manipulation of LPA metabolism, specific blockade of receptor signaling, and/or inhibition of downstream signal transduction pathways, represent approaches for cancer therapies.

It has been reported that LPA and other phospholipids stimulate expression of interleukin-8 (IL-8) in ovarian cancer cell lines. In some embodiments, high concentrations of IL-8 in ovarian cancer correlate with poor initial response to chemotherapy and with poor prognosis, respectively. In animal models, expression of IL-8 and other growth factors such as vascular endothelial growth factor (VEGF) is associated with increased tumorigenicity, ascites formation, angiogenesis, and invasiveness of ovarian cancer cells. IL-8 is an important modulator of cancer progression, drug resistance, and prognosis in ovarian cancer. A compound of the present invention may inhibit or reduce IL-8 expression in ovarian cancer cell lines.

A compound of the present invention, or a pharmaceutically acceptable salt thereof, may be used in a method of treating cancer. A compound of the present invention, or a pharmaceutically acceptable salt thereof, may be used in a method of treating malignant and benign proliferative disease. A compound of the present invention, or a pharmaceutically acceptable salt thereof, may be used to prevent or reduce proliferation of tumor cells, invasion and metastasis of carcinomas, pleural mesothelioma (Yamada, Cancer Sci., 2008, 99(8), 1603-1610) or peritoneal mesothelioma, cancer pain, bone metastases (Boucharaba et al, J. Clin. Invest., 2004, 114(12), 1714-1725; Boucharaba et al, Proc. Natl. acad. Sci., 2006, 103(25) 9643-9648). A method of treating cancer in a mammal, may comprise administering to the mammal a compound of the present invention, or a pharmaceutically acceptable salt thereof, and a second therapeutic agent, wherein the second therapeutic agent is an anticancer agent.

The term "cancer," as used herein refers to an abnormal growth of cells which tend to proliferate in an uncontrolled way and, in some cases, to metastasize (spread). The types of cancer include, but is not limited to, solid tumors (such as those of the bladder, bowel, brain, breast, endometrium, heart, kidney, lung, lymphatic tissue (lymphoma), ovary, pancreas or other endocrine organ (thyroid), prostate, skin (melanoma or basal cell cancer) or hematological tumors (such as the leukemias) at any stage of the disease with or without metastases.

Additional non-limiting examples of cancers include, acute lymphoblastic leukemia, acute myeloid leukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T-Cell lymphoma, embryonal tumors, endometrial cancer, ependymoblastoma, ependymoma, esophageal cancer, ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, Hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, Acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-cell lymphoma, Hodgkin lymphoma, non-Hodgkin lymphoma, lymphoma, Waldenström macroglobulinemia, medulloblastoma, medulloepithelioma, melanoma, mesothelioma, mouth cancer, chronic myelogenous leukemia, myeloid leukemia, multiple myeloma, nasopharyngeal cancer, neuroblastoma, non-Hodgkin lymphoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, sarcoma, kaposi, Sézary syndrome, skin cancer, small cell Lung cancer, small intestine cancer, soft tissue sarcoma, squamous cell carcinoma, stomach (gastric) cancer, supratentorial primitive neuroectodermal tumors, T-cell lymphoma, testicular cancer, throat cancer, thymoma and thymic carcinoma, thyroid cancer, urethral cancer, uterine cancer, uterine sarcoma, vaginal cancer, vulvar cancer, Waldenström macroglobulinemia, Wilms tumor.

The increased concentrations of LPA and vesicles in ascites from ovarian cancer patients and breast cancer effussions indicate that it could be an early diagnostic marker, a prognostic indicator or an indicator of response to therapy (Mills et al, Nat. Rev. Cancer., 3, 582-591, 2003; Sutphen et al., Cancer Epidemiol. Biomarkers Prev. 13, 1185-1191, 2004). LPA concentrations are consistently higher in ascites samples than in matched plasma samples.

### Respiratory and Allergic Disorders

LPA may be a contributor to the pathogenesis of respiratory diseases. The respiratory disease may be asthma. Proinflammatory effects of LPA include degranulation of mast cells, contraction of smooth-muscle cells and release of cytokines from dendritic cells. Airway smooth muscle cells, epithelial cells and lung fibroblasts all show responses to LPA. LPA induces the secretion of IL-8 from human bronchial epithelial cells. IL-8 is found in increased concentrations in BAL fluids from patients with asthma, chronic obstructive lung disease, pulmonary sarcoidosis and acute respiratory distress syndrome and Il-8 has been shown to exacerbate airway inflammation and airway remodeling of asthmatics. LPA1, LPA2 and LPA3 receptors have all been shown to contribute to the LPA-induced IL-8 production. Studies cloning multiple GPCRs that are activated by LPA allowed the demonstration of the presence of mRNA for the LPA₁, LPA₂ and LPA₃ in the lung (J.J.A. Contos, et al., Mol. Pharmacol. 58, 1188-1196, 2000).

The release of LPA from platelets activated at a site of injury and its ability to promote fibroblast proliferation and contraction are features of LPA as a mediator of wound repair. In the context of airway disease, asthma is an inflammatory disease where inappropriate airway "repair" processes lead to structural "remodeling" of the airway. In asthma, the cells of the airway are subject to ongoing injury due to a variety of insults, including allergens, pollutants, other inhaled environmental agents, bacteria and viruses, leading to the chronic inflammation that characterizes asthma.

In the asthmatic individual, the release of normal repair mediators, including LPA, may be exaggerated or the actions of the repair mediators are inappropriately prolonged leading to inappropriate airway remodeling. Major structural features of the remodeled airway observed in asthma include a thickened lamina reticularis (the basement membrane-like structure just beneath the airway epithelial cells), increased numbers and activation of myofibroblasts, thickening of the smooth muscle layer, increased numbers of mucus glands and mucus secretions, and alterations in the connective tissue and capillary bed throughout the airway wall. LPA may contribute to these structural changes in the airway. LPA may be involved in acute airway hyperresponsiveness in asthma. The lumen of the remodeled asthmatic airway is narrower due to the thickening of the airway wall, thus decreasing airflow. LPA may contribute to the long-term structural remodeling and the acute hyperresponsiveness of the asthmatic airway. LPA may contribute to the hyper-responsiveness that is a primary feature of acute exacerbations of asthma.

In addition to the cellular responses mediated by LPA, several of the LPA signaling pathway components leading to these responses are relevant to asthma. EGF receptor upregulation is induced by LPA and is also seen in asthmatic airways (M. Amishima, et al., Am. J. Respir. Crit. Care Med. 157, 1907- 1912, 1998). Chronic inflammation is a contributor to asthma, and several of the transcription factors that are activated by LPA are known to be involved in inflammation (Ediger et al., Eur Respir J 21:759-769, 2003).

The fibroblast proliferation and contraction and extracellular matrix secretion stimulated by LPA may contribute to the fibroproliferative features of other airway diseases, such as the peribronchiolar fibrosis present in chronic bronchitis, emphysema, and interstitial lung disease. Emphysema is also associated with a mild fibrosis of the alveolar wall, a feature which is believed to represent an attempt to repair alveolar damage. LPA may play a role in the fibrotic interstitial lung diseases and obliterative bronchiolitis, where both collagen and myofibroblasts are increased. LPA may be involved in several of the various syndromes that constitute chronic obstructive pulmonary disease.

Administration of LPA *in vivo* induces airway hyper-responsiveness, itch-scratch responses, infiltration and activation of eosinophils and neutrophils, vascular remodeling, and nociceptive flexor responses. LPA also induces histamine release from mouse and rat mast cells. In an acute allergic reaction, histamine induces various responses, such as contraction of smooth muscle, plasma exudation, and mucus production. Plasma exudation is important in the airway, because the leakage and subsequent airway-wall edema contribute to the development of airway hyperresponsiveness. Plasma exudation progresses to conjunctival swelling in ocular allergic disorder and nasal blockage in allergic rhinitis (Hashimoto et al., J Pharmacol Sci 100, 82 - 87, 2006). Plasma exudation induced by LPA may be mediated by histamine release from mast cells via one or more LPA receptors. The LPA receptor(s) may include LPA₁ and/or LPA₃. A compound of the present invention, or a pharmaceutically acceptable salt thereof, may be used in a method of treating various allergic disorders in a mammal. A compound of the present invention, or a pharmaceutically acceptable salt thereof, may be used in a method of treating respiratory diseases, disorders or conditions in a mammal. A compound of the present invention, or a pharmaceutically acceptable salt thereof, may be used in a method of treating asthma in a mammal. A compound of the present invention, or a pharmaceutically acceptable salt thereof, may be used in a method of treating chronic asthma in a mammal.

The term "respiratory disease," as used herein, refers to diseases affecting the organs that are involved in breathing, such as the nose, throat, larynx, eustachian tubes, trachea, bronchi, lungs, related muscles (e.g., diaphram and intercostals), and nerves. Respiratory diseases include, but are not limited to, asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child-onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

The term "asthma" as used herein refers to any disorder of the lungs characterized by variations in pulmonary gas flow associated with airway constriction of whatever cause (intrinsic, extrinsic, or both; allergic or non-allergic). The term asthma may be used with one or more adjectives to indicate cause.

In one aspect, presented herein is a compound of the present invention, or a pharmaceutically acceptable salt thereof, for use in a method of treating or preventing of chronic obstructive pulmonary disease in a mammal comprising administering to the mammal at least once an effective amount of at least one compound of the present invention, or a pharmaceutically acceptable salt thereof. In addition, chronic obstructive pulmonary disease includes, but is not limited to, chronic bronchitis or emphysema, pulmonary hypertension, interstitial lung fibrosis and/or airway inflammation, and cystic fibrosis.

### Nervous System

The nervous system is a major locus for LPA₁ expression; there it is spatially and temporally regulated throughout brain development. Oligodendrocytes, the myelinating cells in the central nervous system (CNS), express LPA₁ in mammals. In addition, Schwann cells, the myelinating cells of the peripheral nervous system, also express LPA₁, which is involved in regulating Schwann cell survival and morphology. These observations identify important functions for receptor-mediated LPA signaling in neurogenesis, cell survival, and myelination.

Exposure of peripheral nervous system cell lines to LPA produces a rapid retraction of their processes resulting in cell rounding, which was, in part, mediated by polymerization of the actin cytoskeleton. LPA may cause neuronal degeneration under pathological conditions when the blood-brain barrier is damaged and serum components leak into the brain (Moolenaar, Curr. Opin. Cell Biol. 7:203-10, 1995). Immortalized CNS neuroblast cell lines from the cerebral cortex also display retraction responses to LPA exposure through Rho activation and actomyosin interactions. LPA is associated with post-ischemic neural damage (J. Neurochem. 61, 340, 1993; J. Neurochem., 70:66, 1998).

A compound of the present invention, or a pharmaceutically acceptable salt thereof, may be used in a method of treating or preventing a nervous system disorder in a mammal. The term "nervous system disorder," as used herein, refers to conditions that alter the structure or function of the brain, spinal cord or peripheral nervous system, including but not limited to Alzheimer's Disease, cerebral edema, cerebral ischemia, stroke, multiple sclerosis, neuropathies, Parkinson's Disease, those found after blunt or surgical trauma (including post-surgical cognitive dysfunction and spinal cord or brain stem injury), as well as the neurological aspects of disorders such as degenerative disk disease and sciatica.

A compound of the present invention, or a pharmaceutically acceptable salt thereof, may be used in a method of treating or preventing a CNS disorder in a mammal. CNS disorders include, but are not limited to, multiple sclerosis, Parkinson's disease, Alzheimer's disease, stroke, cerebral ischemia, retinal ischemia, post-surgical cognitive dysfunction, migraine, peripheral neuropathy/neuropathic pain, spinal cord injury, cerebral edema and head injury.

### Cardiovascular Disorders

Cardiovascular phenotypes observed after targeted deletion of lysophospholipid receptors reveal important roles for lysophospholipid signaling in the development and maturation of blood vessels, formation of atherosclerotic plaques and maintenance of heart rate (Ishii, I. et al. Annu. Rev. Biochem. 73, 321-354, 2004). Angiogenesis, the formation of new capillary networks from pre-existing vasculature, is normally invoked in wound healing, tissue growth and myocardial angiogenesis after ischemic injury. Peptide growth factors (*e.g.* vascular endothelial growth factor (VEGF)) and lysophospholipids control coordinated proliferation, migration, adhesion, differentiation and assembly of vascular endothelial cells (VECs) and surrounding vascular smooth-muscle cells (VSMCs). Dysregulation of the processes mediating angiogenesis may lead to atherosclerosis, hypertension, tumor growth, rheumatoid arthritis and diabetic retinopathy (Osborne, N. and Stainier, D.Y. Annu. Rev. Physiol. 65, 23-43, 2003).

Downstream signaling pathways evoked by lysophospholipid receptors include Rac-dependent lamellipodia formation (*e.g.* LPA₁) and Rho-dependent stress-fiber formation (*e.g.* LPA₁), which is important in cell migration and adhesion. Dysfunction of the vascular endothelium can shift the balance from vasodilatation to vasoconstriction and lead to hypertension and vascular remodeling, which are risk factors for atherosclerosis (Maguire, J.J. et al., Trends Pharmacol. Sci. 26, 448-454, 2005).

LPA contributes to both the early phase (barrier dysfunction and monocyte adhesion of the endothelium) and the late phase (platelet activation and intra-arterial thrombus formation) of atherosclerosis, in addition to its overall progression. In the early phase, LPA from numerous sources accumulates in lesions and activates its cognate GPCRs (LPA₁ and LPA₃) expressed on platelets (Siess, W. Biochim. Biophys. Acta 1582, 204-215, 2002; Rother, E. et al. Circulation 108, 741-747, 2003). This triggers platelet shape change and aggregation, leading to intra-arterial thrombus formation and, potentially, myocardial infarction and stroke. In support of its atherogenic activity, LPA can also be a mitogen and motogen to VSMCs and an activator of endothelial cells and macrophages. Mammals with cardiovascular disease may benefit from LPA receptor antagonists that prevent thrombus and neointima plaque formation.

A compound of the present invention, or a pharmaceutically acceptable salt thereof, may be used in a method of treating or preventing cardiovascular disease in mammal.

The term "cardiovascular disease," as used herein refers to diseases affecting the heart or blood vessels or both, including but not limited to: arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue..

Compounds of the present invention may be used in a method for preventing or treating vasoconstriction, atherosclerosis and its sequelae myocardial ischemia, myocardial infarction, aortic aneurysm, vasculitis and stroke comprising administering at least once to the mammal an effective amount of at least one compound of the present invention, or a pharmaceutically acceptable salt thereof, or pharmaceutical composition or medicament which includes a compound of the present invention, or a pharmaceutically acceptable salt thereof.

Compounds of the present invention may be used in a method for reducing cardiac reperfusion injury following myocardial ischemia and/or endotoxic shock comprising administering at least once to the mammal an effective amount of at least one compound of the present invention, or a pharmaceutically acceptable salt thereof.

ICompounds of the present invention may be used in a method for reducing the constriction of blood vessels in a mammal comprising administering at least once to the mammal an effective amount of at least one compound of the present invention, or a pharmaceutically acceptable salt thereof.

Compounds of the present invention may be used in a method for lowering or preventing an increase in blood pressure of a mammal comprising administering at least once to the mammal an effective amount of at least one compound of the present invention, or a pharmaceutically acceptable salt thereof.

### Inflammation

LPA has been shown to regulate immunological responses by modulating activities/functions of immune cells such as T-/B-lymphocytes and macrophages. In activated T cells, LPA activates IL-2 production/cell proliferation through LPA₁ (Gardell et al, TRENDS in Molecular Medicine Vol. 12 No.2 February 2006). Expression of LPA-induced inflammatory response genes is mediated by LPA₁ and LPA₃ (Biochem Biophys Res Commun. 363(4): 1001-8, 2007). In addition, LPA modulates the chemotaxis of inflammatory cells (Biochem Biophys Res Commun., 1993, 15; 193(2), 497). The proliferation and cytokine-secreting activity in response to LPA of immune cells (J. Imuunol. 1999, 162, 2049), platelet aggregation activity in response to LPA, acceleration of migration activity in monocytes, activation of NF-_{K}B in fibroblast, enhancement of fibronectin-binding to the cell surface, and the like are known. Thus, LPA is associated with various inflammatory/immune diseases.

A compound of the present invention, or a pharmaceutically acceptable salt thereof, may be used in a method of treating or preventing inflammation in a mammal. Antagonists of LPA₁ and/or LPA₃ find use in the treatment or prevention of inflammatory/immune disorders in a mammal. The antagonist of LPA₁ may be a compound of the present invention, or a pharmaceutically acceptable salt thereof.

Examples of inflammatory/immune disorders include psoriasis, rheumatoid arthritis, vasculitis, inflammatory bowel disease, dermatitis, osteoarthritis, asthma, inflammatory muscle disease, allergic rhinitis, vaginitis, interstitial cystitis, scleroderma, eczema, allogeneic or xenogeneic transplantation (organ, bone marrow, stem cells and other cells and tissues) graft rejection, graft-versus-host disease, lupus erythematosus, inflammatory disease, type I diabetes, pulmonary fibrosis, dermatomyositis, Sjogren's syndrome, thyroiditis (e.g., Hashimoto's and autoimmune thyroiditis), myasthenia gravis, autoimmune hemolytic anemia, multiple sclerosis, cystic fibrosis, chronic relapsing hepatitis, primary biliary cirrhosis, allergic conjunctivitis and atopic dermatitis.

### Other Diseases, Disorders or Conditions

Compounds of the present invention may be used in methods for treating, preventing, reversing, halting or slowing the progression of LPA-dependent or LPA-mediated diseases or conditions once it becomes clinically evident, or treating the symptoms associated with or related to LPA-dependent or LPA-mediated diseases or conditions, by administering to the mammal a compound of the present invention, or a pharmaceutically acceptable salt thereof. The subject may already havea LPA-dependent or LPA-mediated disease or condition at the time of administration, or may be at risk of developing a LPA-dependent or LPA-mediated disease or condition.

The activity of LPA₁ in a mammal may be directly or indirectly modulated by the administration of (at least once) a therapeutically effective amount of at least one compound of the present invention, or a pharmaceutically acceptable salt thereof. Such modulation includes, but is not limited to, reducing and/or inhibiting the activity of LPA₁. The activity of LPA in a mammal may be directly or indirectly modulated, including reducing and/or inhibiting, by the administration of (at least once) a therapeutically effective amount of at least one compound of the present invention, or a pharmaceutically acceptable salt thereof. Such modulation includes, but is not limited to, reducing and/or inhibiting the amount and/or activity of a LPA receptor. The LPA receptor may be LPA₁.

LPA may have a contracting action on bladder smooth muscle cell isolated from bladder, and promotes growth of prostate-derived epithelial cell *(*J. Urology, 1999, 162, 1779-1784; J. Urology, 2000, 163, 1027-1032). LPA may contract the urinary tract and prostate *in vitro* and increases intraurethral pressure *in vivo* (WO 02/062389).

Compounds of the present invention may be used in methods for preventing or treating eosinophil and/or basophil and/or dendritic cell and/or neutrophil and/or monocyte and/or T-cell recruitment comprising administering at least once to the mammal an effective amount of at least one compound of the present invention, or a pharmaceutically acceptable salt thereof.

Compounds of the present invention may be used in methods for the treatment of cystitis, including, e.g.,interstitial cystitis, comprising administering at least once to the mammal a therapeutically effective amount of at least one compound of the present invention, or a pharmaceutically acceptable salt thereof.

Compounds of the present invention may be used in methods described herein which may include the diagnosis or determination of whether or not a patient is suffering from a LPA-dependent or LPA-mediated disease or condition by administering to the subject a therapeutically effective amount of a compound of the present invention, or a pharmaceutically acceptable salt thereof, and determining whether or not the patient responds to the treatment.

Compounds of the present invention, pharmaceutically acceptable salts, and pharmaceutically acceptable solvates thereof, which are antagonists of LPA₁, may be used in a method of treating patients suffering from one or more LPA-dependent or LPA-mediated conditions or diseases, including, but not limited to, lung fibrosis, kidney fibrosis, liver fibrosis, scarring, asthma, rhinitis, chronic obstructive pulmonary disease, pulmonary hypertension, interstitial lung fibrosis, arthritis, allergy, psoriasis, inflammatory bowel disease, adult respiratory distress syndrome, myocardial infarction, aneurysm, stroke, cancer, pain, proliferative disorders and inflammatory conditions. LPA-dependent conditions or diseases may include those wherein an absolute or relative excess of LPA is present and/or observed.

The LPA-dependent or LPA-mediated diseases or conditions may include, but are not limited to, organ fibrosis, asthma, allergic disorders, chronic obstructive pulmonary disease, pulmonary hypertension, lung or pleural fibrosis, peritoneal fibrosis, arthritis, allergy, cancer, cardiovascular disease, ult respiratory distress syndrome, myocardial infarction, aneurysm, stroke, and cancer.

A compound of the present invention, or a pharmaceutically acceptable salt thereof, may be used in a method to improve the corneal sensitivity decrease caused by corneal operations such as laser-assisted in situ keratomileusis (LASIK) or cataract operation, corneal sensitivity decrease caused by corneal degeneration, and dry eye symptom caused thereby.

A compound of the present invention, or a pharmaceutically acceptable salt thereof, may be used in a method of treating or preventing ocular inflammation and allergic conjunctivitis, vernal keratoconjunctivitis, and papillary conjunctivitis in a mammal comprising administering at least once to the mammal an effective amount of at least one compound of the present invention, or a pharmaceutically acceptable salt thereof.

A compound of the present invention, or a pharmaceutically acceptable salt thereof, may be used in a method of treating or preventing Sjogren disease or inflammatory disease with dry eyes in a mammal comprising administering at least once to the mammal an effective amount of at least one compound of the present invention, or a pharmaceutically acceptable salt thereof.

LPA and LPA receptors (*e*.*g*. LPA₁) may be involved in the pathogenesis of osteoarthritis (Kotani et al, Hum. Mol. Genet., 2008, 17, 1790-1797). A compound of the present invention, or a pharmaceutically acceptable salt thereof, may be used in a method of treating or preventing osteoarthritis in a mammal comprising administering at least once to the mammal an effective amount of at least one compound of the present invention, or a pharmaceutically acceptable salt thereof.

LPA receptors (*e*.*g*. LPA₁, LPA₃) may contribute to the pathogenesis of rheumatoid arthritis (Zhao et al, Mol. Pharmacol., 2008, 73(2), 587-600). A compound of the present invention, or a pharmaceutically acceptable salt thereof, may be used in a method of treating or preventing rheumatoid arthritis in a mammal comprising administering at least once to the mammal an effective amount of at least one compound of the present invention, or a pharmaceutically acceptable salt thereof.

LPA receptors (*e.g.* LPA₁) may contribute to adipogenesis. (Simon et al, J.Biol. Chem., 2005, vol. 280, no. 15, p. 14656). A compound of the present invention, or a pharmaceutically acceptable salt thereof, may be used in a method of promoting adipose tissue formation in a mammal comprising administering at least once to the mammal an effective amount of at least one compound of the present invention, or a pharmaceutically acceptable salt thereof.

### a. In Vitro Assays

The effectiveness of compounds of the present invention as LPA1 inhibitors can be determined in an LPA1 functional antagonist assay as follows:
Chinese hamster ovary cells overexpressing human LPA1 were plated overnight (15,000 cells/well) in poly-D-lysine coated 384-well microplates (Greiner bio-one, Cat#781946) in DMEM/F12 medium (Gibco, Cat#11039). Following overnight culture, cells were loaded with calcium indicator dye (AAT Bioquest Inc, Cat# 34601) for 30 minutes at 37 °C. The cells were then equilibrated to room temperature for 30 minutes before the assay. Test compounds solubilized in DMSO were transferred to 384 well non-binding surface plates (Corning, Cat# 3575) using the Labcyte Echo acoustic dispense and diluted with assay buffer [1X HBSS with calcium/magnesium (Gibco Cat# 14025-092), 20 mM HEPES (Gibco Cat# 15630-080) and 0.1% fatty acid free BSA (Sigma Cat# A9205)] to a final concentration of 0.5% DMSO. Diluted compounds were added to the cells by FDSS6000 (Hamamatsu) at final concentrations ranging from 0.08 nM to 5 µM. and were then incubated for 20 min at room temperature at which time LPA (Avanti Polar Lipids Cat#857130C) was added at final concentrations of 10 nM to stimulate the cells. The compound IC₅₀ value was defined as the concentration of test compound which inhibited 50% of the calcium flux induced by LPA alone. IC₅₀ values were determined by fitting data to a 4-parameter logistic equation (GraphPad Prism, San Diego CA).

### b. In Vivo Assays

LPA Challenge with plasma histamine evaluation.

Compound is dosed orally p.o. 2 hours to CD-1 female mice prior to the LPA challenge. The mice are then dosed via tail vein (IV) with 0.15 mL of LPA in 0.1%BSA/ PBS (2 µg/µL). Exactly 2 minutes following the LPA challenge, the mice are euthanized by decapitation and the trunk blood is collected. These samples are collectively centrifuged and individual 75 µL samples are frozen at -20°C until the time of the histamine assay.

The plasma histamine analysis was run by standard EIA (Enzyme Immunoassay) methods. Plasma samples were thawed and diluted 1:30 in 0.1% BSA in PBS. The EIA protocol for histamine analysis as outlined by the manufacturer was followed (Histamine EIA, Oxford Biomedical Research, EA#31).

The LPA used in the assay is formulated as follows: LPA (1-oleoyl-2-hydroxy-sn-glycero-3-phosphate (sodium salt), 857130P, Avanti Polar Lipids) is prepared in 0.1%BSA/PBS for total concentration of 2 µg/µL. 13 mg of LPA is weighed and 6.5 mL 0.1%BSA added, vortexed and sonicated for ~1 hour until a clear solution is achieved.

### V. PHARMACEUTICAL COMPOSITIONS, FORMULATIONS AND COMBINATIONS

In some embodiments, provided is a pharmaceutical composition comprising a therapeutically effective amount of a compound of the present invention, or a pharmaceutically acceptable salt thereof. In some embodiments, the pharmaceutical composition also contains at least one pharmaceutically acceptable inactive ingredient.

A pharmaceutical composition may comprise a therapeutically effective amount of a compound of the present invention, or a pharmaceutically acceptable salt thereof, and at least one pharmaceutically acceptable inactive ingredient. The pharmaceutical composition may be formulated for intravenous injection, subcutaneous injection, oral administration, inhalation, nasal administration, topical administration, ophthalmic administration or otic administration. In some embodiments, the pharmaceutical composition is a tablet, a pill, a capsule, a liquid, an inhalant, a nasal spray solution, a suppository, a suspension, a gel, a colloid, a dispersion, a suspension, a solution, an emulsion, an ointment, a lotion, an eye drop or an ear drop.

The pharmaceutical composition may further comprise one or more additional therapeutically active agents selected from: corticosteroids (*e*.*g*., dexamethasone or fluticasone), immunosuppresants (*e*.*g*., tacrolimus & pimecrolimus), analgesics, anti-cancer agent, anti-inflammatories, chemokine receptor antagonists, bronchodilators, leukotriene receptor antagonists (*e*.*g*., montelukast or zafirlukast), leukotriene formation inhibitors, monoacylglycerol kinase inhibitors, phospholipase A₁ inhibitors, phospholipase A₂ inhibitors, and lysophospholipase D (lysoPLD) inhibitors, autotaxin inhibitors, decongestants, antihistamines (*e*.*g*., loratidine), mucolytics, anticholinergics, antitussives, expectorants, anti-infectives (*e.g*., fusidic acid, particularly for treatment of atopic dermatitis), anti-fungals (*e.g.*, clotriazole, particularly for atopic dermatitis), anti-IgE antibody therapies (*e*.*g*., omalizumab), β-2 adrenergic agonists (*e*.*g*., albuterol or salmeterol), other PGD2 antagonists acting at other receptors such as DP antagonists, PDE4 inhibitors (*e*.*g*., cilomilast), drugs that modulate cytokine production, *e*.*g*., TACE inhibitors, drugs that modulate activity of Th2 cytokines IL-4 & IL-5 (*e.g.,* blocking monoclonal antibodies & soluble receptors), PPARγ agonists (*e*.*g*., rosiglitazone and pioglitazone), 5-lipoxygenase inhibitors (*e*.*g*., zileuton).

The pharmaceutical composition may further comprise one or more additional anti-fibrotic agents selected from pirfenidone, nintedanib, thalidomide, carlumab, FG-3019, fresolimumab, interferon alpha, lecithinized superoxide dismutase, simtuzumab, tanzisertib, tralokinumab, hu3G9, AM-152, IFN-gamma-1b, IW-001, PRM-151, PXS-25, pentoxifylline/N-acetyl-cysteine, pentoxifylline/vitamin E, salbutamol sulfate, [Sar9,Met(O2)11]-Substance P, pentoxifylline, mercaptamine bitartrate, obeticholic acid, aramchol, GFT-505, eicosapentaenoic acid ethyl ester, metformin, metreleptin, muromonab-CD3, oltipraz, IMM-124-E, MK-4074, PX-102, RO-5093151. A method may comprise administering a compound of the present invention, or a pharmaceutically acceptable salt thereof, to a human with a LPA-dependent or LPA-mediated disease or condition. The human may have already been administered one or more additional therapeutically active agents other than a compound of the present invention, or a pharmaceutically acceptable salt thereof. The method may further comprise administering one or more additional therapeutically active agents other than a compound of the present invention, or a pharmaceutically acceptable salt thereof.

The one or more additional therapeutically active agents other than a compound of the present invention, or a pharmaceutically acceptable salt thereof, may be selected from: corticosteroids (e.g,. dexamethasone or fluticasone), immunosuppresants (*e.g*., tacrolimus & pimecrolimus), analgesics, anti-cancer agent, anti-inflammatories, chemokine receptor antagonists, bronchodilators, leukotriene receptor antagonists (*e*.*g*., montelukast or zafirlukast), leukotriene formation inhibitors, monoacylglycerol kinase inhibitors, phospholipase A₁ inhibitors, phospholipase A₂ inhibitors, and lysophospholipase D (lysoPLD) inhibitors, autotaxin inhibitors, decongestants, antihistamines (*e*.*g*., loratidine), mucolytics, anticholinergics, antitussives, expectorants, anti-infectives (*e*.*g*., fusidic acid, particularly for treatment of atopic dermatitis), anti-fungals (*e*.*g*., clotriazole, particularly for atopic dermatitis), anti-IgE antibody therapies (*e*.*g*., omalizumab), β-2 adrenergic agonists (*e*.*g*., albuterol or salmeterol), other PGD2 antagonists acting at other receptors such as DP antagonists, PDE4 inhibitors (*e*.*g*., cilomilast), drugs that modulate cytokine production, *e*.*g*. TACE inhibitors, drugs that modulate activity of Th2 cytokines IL-4 & IL-5 (*e*.*g*., blocking monoclonal antibodies & soluble receptors), PPARγ agonists (*e*.*g*., rosiglitazone and pioglitazone), 5-lipoxygenase inhibitors (*e*.*g*., zileuton).

The one or more additional therapeutically active agents other than a compound of the present invention, or a pharmaceutically acceptable salt thereof, may be other anti-fibrotic agents selected from pirfenidone, nintedanib, thalidomide, carlumab, FG-3019, fresolimumab, interferon alpha, lecithinized superoxide dismutase, simtuzumab, tanzisertib, tralokinumab, hu3G9, AM-152, IFN-gamma-1b, IW-001, PRM-151, PXS-25, pentoxifylline/N-acetyl-cysteine, pentoxifylline/vitamin E, salbutamol sulfate, [Sar9,Met(O2)11]-Substance P, pentoxifylline, mercaptamine bitartrate, obeticholic acid, aramchol, GFT-505, eicosapentyl ethyl ester, metformin, metreleptin, muromonab-CD3, oltipraz, IMM-124-E, MK-4074, PX-102, RO-5093151.

The one or more additional therapeutically active agents other than a compound of the present invention, or a pharmaceutically acceptable salt thereof, may be selected from ACE inhibitors, ramipril, AII antagonists, irbesartan, anti-arrythmics, dronedarone, PPARα activators, PPARγ activators, pioglitazone, rosiglitazone, prostanoids, endothelin receptor antagonists, elastase inhibitors, calcium antagonists, beta blockers, diuretics, aldosterone receptor antagonists, eplerenone, renin inhibitors, rho kinase inhibitors, soluble guanylate cyclase (sGC) activators, sGC sensitizers, PDE inhibitors, PDE5 inhibitors, NO donors, digitalis drugs, ACE/NEP inhibitors, statins, bile acid reuptake inhibitors, PDGF antagonists, vasopressin antagonists, aquaretics, NHE1 inhibitors, Factor Xa antagonists, Factor XIIIa antagonists, anticoagulants, anti-thrombotics, platelet inhibitors, profibroltics, thrombin-activatable fibrinolysis inhibitors (TAFI), PAI-1 inhibitors, coumarins, heparins, thromboxane antagonists, serotonin antagonists, COX inhibitors, aspirin, therapeutic antibodies, GPIIb/IIIa antagonists, ER antagonists, SERMs, tyrosine kinase inhibitors, RAF kinase inhibitors, p38 MAPK inhibitors, pirfenidone, multi-kinase inhibitors, nintedanib, sorafenib.

The one or more additional therapeutically active agents other than a compound of the present invention, or a pharmaceutically acceptable salt thereof, may be selected from Gremlin-1 mAb, PA1-1 mAb, Promedior (PRM-151; recombinant human Pentraxin-2); FGF21, TGFβ antagonists, αvβ6 & αvβ pan-antagonists; FAK inhibitors, TG2 inhibitors, LOXL2 inhibitors, NOX4 inhibitors, MGAT2 inhibitors, GPR120 agonists.

Pharmaceutical formulations described herein are administrable to a subject in a variety of ways by multiple administration routes, including but not limited to, oral, parenteral (e.g., intravenous, subcutaneous, intramuscular), intranasal, buccal, topical or transdermal administration routes. The pharmaceutical formulations described herein include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations, and mixed immediate and controlled release formulations.

The compound of the present invention, or a pharmaceutically acceptable salt thereof, may be administered orally.

The compound of the present invention, or a pharmaceutically acceptable salt thereof, may be administered topically. The compound of the present invention, or a pharmaceutically acceptable salt thereof, may be formulated into a variety of topically administrable compositions, such as solutions, suspensions, lotions, gels, pastes, shampoos, scrubs, rubs, smears, medicated sticks, medicated bandages, balms, creams or ointments. Such pharmaceutical compounds can contain solubilizers, stabilizers, tonicity enhancing agents, buffers and preservatives. The compound of the present invention, or a pharmaceutically acceptable salt thereof, may be administered topically to the skin.

The compound of the present invention, or a pharmaceutically acceptable salt thereof, may be administered by inhalation. The compound of the present invention, or a pharmaceutically acceptable salt thereof, may be administered by inhalation that directly targets the pulmonary system.

The compound of the present invention, or a pharmaceutically acceptable salt thereof, may be formulated for intranasal administration. Such formulations may include nasal sprays, nasal mists, and the like.

The compound of the present invention, or a pharmaceutically acceptable salt thereof, may be formulated as eye drops.

A compound of the present invention, or a pharmaceutically acceptable salt thereof, may be used in a method of treating a disease, disorder or conditions in which the activity of at least one LPA receptor contributes to the pathology and/or symptoms of the disease or condition. The LPA may be selected from LPA₁, LPA₂, LPA₃, LPA₄, LPA₅ and LPA₆. The LPA receptor may be LPA₁. The disease or condition may be any of the diseases or conditions specified herein.

In any of the aforementioned aspects are further embodiments in which: (a) the effective amount of the compound of the present invention, or a pharmaceutically acceptable salt thereof, may be systemically administered to the mammal; and/or (b) the effective amount of the compound may be administered orally to the mammal; and/or (c) the effective amount of the compound may be intravenously administered to the mammal; and/or (d) the effective amount of the compound may be administered by inhalation; and/or (e) the effective amount of the compound may be administered by nasal administration; or and/or (f) the effective amount of the compound may be administered by injection to the mammal; and/or (g) the effective amount of the compound may be administered topically to the mammal; and/or (h) the effective amount of the compound may be administered by ophthalmic administration; and/or (i) the effective amount of the compound may be administered rectally to the mammal; and/or (j) the effective amount may be administered non-systemically or locally to the mammal.

In any of the aforementioned aspects are further embodiments comprising single administrations of the effective amount of the compound, including further embodiments in which (i) the compound may be administered once; (ii) the compound may be administered to the mammal multiple times over the span of one day; (iii) continually; or (iv) continuously.

In any of the aforementioned aspects are further embodiments comprising multiple administrations of the effective amount of the compound, including further embodiments in which (i) the compound may be administered continuously or intermittently: as in a a single dose; (ii) the time between multiple administrations may be every 6 hours; (iii) the compound may be administered to the mammal every 8 hours; (iv) the compound may beadministered to the mammal every 12 hours; (v) the compound may be administered to the mammal every 24 hours. The method may comprise a drug holiday, wherein the administration of the compound is temporarily suspended or the dose of the compound being administered is temporarily reduced; at the end of the drug holiday, dosing of the compound is resumed. The length of the drug holiday may vary from 2 days to 1 year.

A compound of the present invention may be used in a method of inhibiting the physiological activity of LPA in a mammal comprising administering a therapeutically effective amount of a compound of the present invention or a pharmaceutically acceptable salt thereof to the mammal in need thereof.

A compound of the present invention may be used in a medicament for treating a LPA-dependent or LPA-mediated disease or condition in a mammal comprising a therapeutically effective amount of a compound of the present invention, or a pharmaceutically acceptable salt thereof.

A a compound of the present invention, or a pharmaceutically acceptable salt thereof, may be used in the manufacture of a medicament for the treatment of a LPA-dependent or LPA-mediated disease or condition.

A compound of the present invention, or a pharmaceutically acceptable salt thereof, may be used in a method of treating or preventing an LPA-dependent or LPA-mediated disease or condition.

A compound of the present invention may be used in a method for treating or preventing a LPA-dependent or LPA-mediated disease or condition in a mammal comprising administering a therapeutically effective amount of a compound of the present invention, or a pharmaceutically acceptable salt thereof.

LPA-dependent or LPA-mediated diseases or conditions include, but are not limited to, fibrosis of organs or tissues, scarring, liver diseases, dermatological conditions, cancer, cardiovascular disease, respiratory diseases or conditions, inflammatory disease, gastrointestinal tract disease, renal disease, urinary tract-associated disease, inflammatory disease of lower urinary tract, dysuria, frequent urination, pancreas disease, arterial obstruction, cerebral infarction, cerebral hemorrhage, pain, peripheral neuropathy, and fibromyalgia.
the LPA-dependent or LPA-mediated disease or condition may be a respiratory disease or condition. The respiratory disease or condition may be asthma, chronic obstructive pulmonary disease (COPD), pulmonary fibrosis, pulmonary arterial hypertension or acute respiratory distress syndrome.

The LPA-dependent or LPA-mediated disease or condition may be selected from idiopathic pulmonary fibrosis; other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease); radiation induced fibrosis; chronic obstructive pulmonary disease (COPD); scleroderma; bleomycin induced pulmonary fibrosis; chronic asthma; silicosis; asbestos induced pulmonary fibrosis; acute respiratory distress syndrome (ARDS); kidney fibrosis; tubulointerstitium fibrosis; glomerular nephritis; focal segmental glomerular sclerosis; IgA nephropathy; hypertension; Alport; gut fibrosis; liver fibrosis; cirrhosis; alcohol induced liver fibrosis; toxic/drug induced liver fibrosis; hemochromatosis; nonalcoholic steatohepatitis (NASH); biliary duct injury; primary biliary cirrhosis; infection induced liver fibrosis; viral induced liver fibrosis; and autoimmune hepatitis; corneal scarring; hypertrophic scarring; Duputren disease, keloids, cutaneous fibrosis; cutaneous scleroderma; spinal cord injury/fibrosis; myelofibrosis; vascular restenosis; atherosclerosis; arteriosclerosis; Wegener's granulomatosis; Peyronie's disease, chronic lymphocytic leukemia, tumor metastasis, transplant organ rejection, endometriosis, neonatal respiratory distress syndrome and neuropathic pain.

The LPA-dependent or LPA-mediated disease or condition may be as described herein.

A compound of the present invention may be used in a method for the treatment or prevention of organ fibrosis in a mammal comprising administering a therapeutically effective amount of a compound of the present invention or a pharmaceutically acceptable salt thereof to a mammal in need thereof.

The organ fibrosis may comprise lung fibrosis, renal fibrosis, or hepatic fibrosis.

A compound of the present invention may be used in a method of improving lung function in a mammal comprising administering a therapeutically effective amount of a compound of the present invention, or a pharmaceutically acceptable salt thereof to the mammal in need thereof. In one aspect, the mammal has been diagnosed as having lung fibrosis.

Compounds disclosed herein may be used in a method of treating idiopathic pulmonary fibrosis (usual interstitial pneumonia) in a mammal.

Compounds disclosed herein may be used in a method of treating diffuse parenchymal interstitial lung diseases in mammal: iatrogenic drug induced, occupational/environmental (Farmer lung), granulomatous diseases (sarcoidosis, hypersensitivity pneumonia), collagen vascular disease (scleroderma and others), alveolar proteinosis, langerhans cell granulonmatosis, lymphangioleiomyomatosis, Hermansky-Pudlak Syndrome, Tuberous sclerosis, neurofibromatosis, metabolic storage disorders, familial interstitial lung disease.

Compounds disclosed herein may be used in a method of treating post-transplant fibrosis associated with chronic rejection in a mammal: Bronchiolitis obliterans for lung transplant.

Compounds disclosed herein may be used in a method of treating cutaneous fibrosis in a mammal: cutaneous scleroderma, Dupuytren disease, keloids.

Compounds disclosed herein may be used in a method of treating hepatic fibrosis with or without cirrhosis in a mammal: toxic/drug induced (hemochromatosis), alcoholic liver disease, viral hepatitis (hepatitis B virus, hepatitis C virus, HCV), nonalcoholic liver disease (NAFLD, NASH), metabolic and auto-immune disease.

Compounds disclosed herein may be used in a method of treating renal fibrosis in a mammal: tubulointerstitium fibrosis, glomerular sclerosis.

In any of the aforementioned aspects involving the treatment of LPA dependent diseases or conditions are further embodiments which may comprise administering at least one additional agent in addition to the administration of a compound having the structure of the present invention, or a pharmaceutically acceptable salt thereof. Each agent may be administered in any order, including simultaneously.

The mammal may be a human.

Compounds provided herein may be administered to a human.

Compounds provided herein may be orally administered.

Compounds provided herein may be used as antagonists of at least one LPA receptor. Compounds provided herein may be used for inhibiting the activity of at least one LPA receptor or for the treatment of a disease or condition that would benefit from inhibition of the activity of at least one LPA receptor. The LPA receptor may be LPA₁.

Compounds provided herein may be used in a method of inhibiting LPA₁ activity.

Articles of manufacture, which include packaging material, a compound of the present invention, or a pharmaceutically acceptable salt thereof, within the packaging material, and a label that indicates that the compound or composition, or pharmaceutically acceptable salt, tautomers, pharmaceutically acceptable N-oxide, or pharmaceutically acceptable solvate thereof, may be used for inhibiting the activity of at least one LPA receptor, or for the treatment, prevention or amelioration of one or more symptoms of a disease or condition that would benefit from inhibition of the activity of at least one LPA receptor, are provided.

### VI. GENERAL SYNTHESIS INCLUDING SCHEMES

The compounds of the present invention can be prepared in a number of ways known to one skilled in the art of organic synthesis. The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al., (Protective Groups in Organic Synthesis, Fourth Edition, Wiley-Interscience (2006)).

The compounds of the present invention may be prepared by the exemplary processes described in the following schemes and working examples, as well as relevant published literature procedures that are used by one skilled in the art. Exemplary reagents and procedures for these reactions appear herein after and in the working examples. Protection and deprotection in the processes below may be carried out by procedures generally known in the art (see, for example, Wuts, P.G.M., Greene's Protective Groups in Organic Synthesis, 5th Edition, Wiley (2014)). General methods of organic synthesis and functional group transformations are found in: Trost, B.M. et al., Eds., Comprehensive Organic Synthesis: Selectivity, Strategy & Efficiency in Modern Organic Chemistry, Pergamon Press, New York, NY (1991); Smith, M.B. et al., March's Advanced Organic Chemistry: Reactions, Mechanisms, and Structure. 7th Edition, Wiley, New York, NY (2013); Katritzky, A.R. et al., Eds., Comprehensive Organic Functional Group Transformations II, 2nd Edition, Elsevier Science Inc., Tarrytown, NY (2004); Larock, R.C., Comprehensive Organic Transformations, 2nd Edition, Wiley-VCH, New York, NY (1999), and references therein.

Scheme 1 describes the synthesis of O-carbamoyl triazole aryl(heteroaryl)oxy-cycloheptyl acids 15 (not claimed). Conjugate addition of vinyl magnesium bromide (catalyzed by e.g. CuBr) to cycloheptenone 1 provides the 3-vinyl cycloheptanone 2. Reduction of cycloheptenone 2 (e.g. with NaBH₄) provides 3-vinyl cycloheptanol 3 as a mixture of diastereomers. A dihalo (preferably dibromo) phenyl or azine (e.g. pyridine) derivative 4 is coupled with an appropriately protected (*e.g.* as a tetrahydropyranyl ether) propargyl alcohol 5 under Sonogashira conditions (e.g. Alper, P. et al, WO 2008097428) to give the corresponding bromo-aryl or bromo-heteroaryl protected propargyl alcohol 6. Thermal reaction of alkyne 6 with an alkyl azide R⁵N₃ (with or without an appropriate catalyst; Qian, Y. et al, J. Med. Chem., 2012, 55 , 7920-7939 or Boren, B. C., et al., J. Am. Chem. Soc., 2008, 130, 8923-8930) provides the corresponding regioisomeric protected hydroxylmethyl-triazoles, from which the desired triazole regioisomer 7 can be isolated. Reaction of the bromoaryl- or bromoheteroaryl-triazoles 7 with bis(pinacolato)diboron in the presence of an appropriate palladium catalyst (e.g. Ishiyama, T. et al, J. Org. Chem. 1995, 60, 7508-7510) provides the corresponding pinacol boronate 8, which is then oxidized with hydrogen peroxide to give the corresponding phenol or hydroxyheteroarene 9 (Fukumoto, S. et al, WO 2012137982). Reaction of phenol/hydroxyheteroarene 9 with cycloheptanol 3 under Mitsunobu reaction conditions (Kumara Swamy, K. C., Chem. Rev., 2009, 109, 2551-2651) furnishes the corresponding triazole cycloheptyl ether 10. Oxidative cleavage of the vinyl group of triazole cycloheptyl ether 10 (e.g. with RuCl₃/NaIO₄, Carlsen, P. H. J., et al, J. Org. Chem., 1981, 46, 3936-3938) provides the corresponding carboxylic acid, which is then appropriately protected to give the triazole cycloheptyl ester 11. Deprotection of the cycloheptyl triazole 11 provides the triazole alcohol 12, which is then reacted with 4-nitrophenyl chloroformate in the presence of an appropriate base to give the triazole 4-nitrophenyl carbonate 13. The triazole 4-nitrophenyl carbonate 13 is then reacted with an amine 14 in the presence of an appropriate base to give the corresponding triazole carbamate, which is then deprotected to give the desired triazole-carbamate cycloheptyl acids 15.

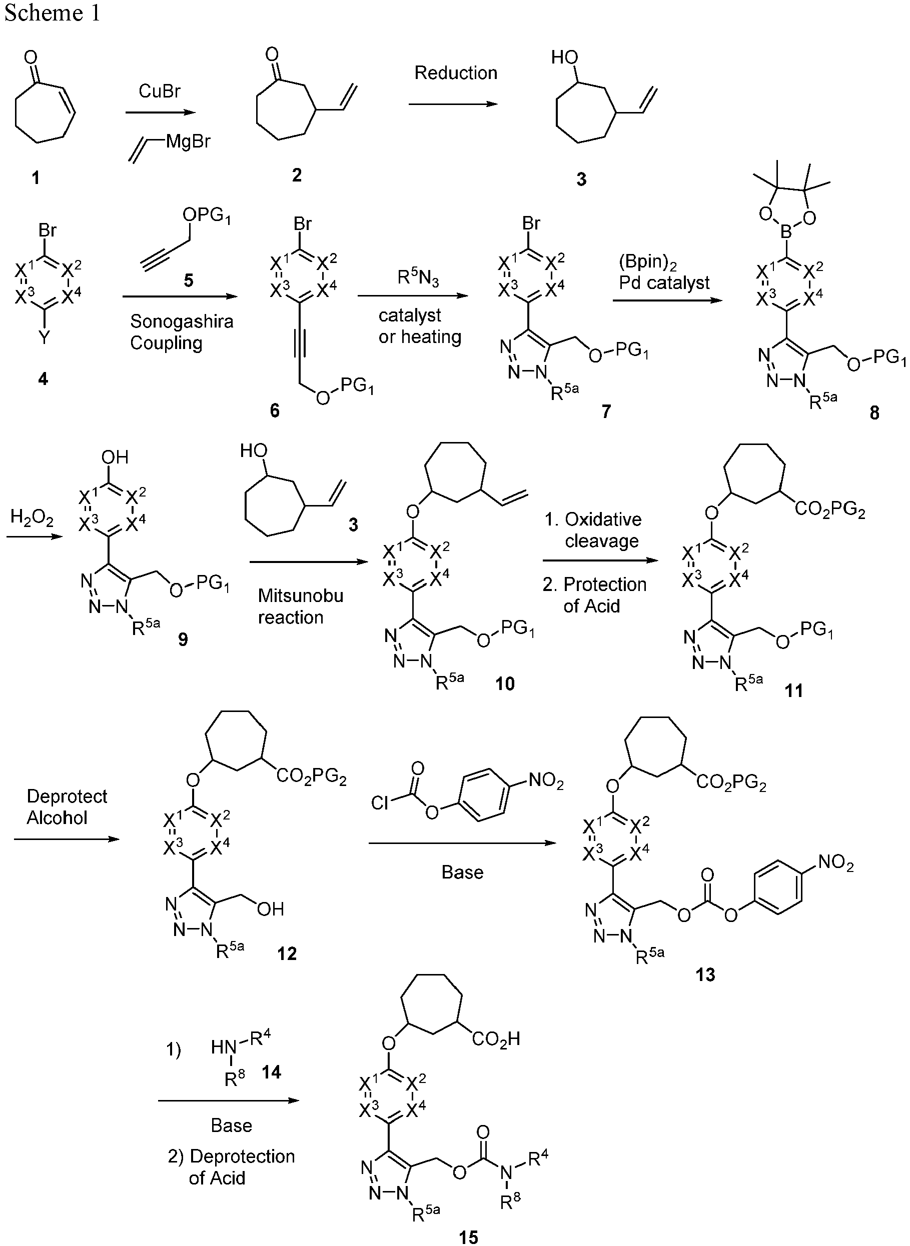

For the specific example of analogs 15, where R^{5a} = CH₃ (Scheme 1A, not claimed), trimethylsilyl azide (Qian, Y. et al, J. Med. Chem., 2012, 55 , 7920-7939) is used in the cycloaddition reaction with the protected aryl propargyl alcohol 6. This cycloaddition can occur under either thermal or, preferably, transition-metal catalyzed conditions (e.g. Boren, B.C. et. al., J. Am. Chem. Soc., 2008, 130, 8923-8930; Ramasamy, S., et al., Org. Process Res. Dev., 2018, 22, 880-887) to provide the desired triazole regioisomer 16 as the major product. The trimethylsilyl group is subsequently removed under standard desilylation conditions (*e.g.* Bu₄NF, as in Qian, Y. et al, J. Med. Chem., 2012, 55, 7920-7939) to provide the triazole 17. This triazole intermediate is subsequently carried forward (using the general synthetic sequence as described in Scheme 1) to triazole carbamate cycloheptyl acids 15 (where R^{5a} = CH₃).

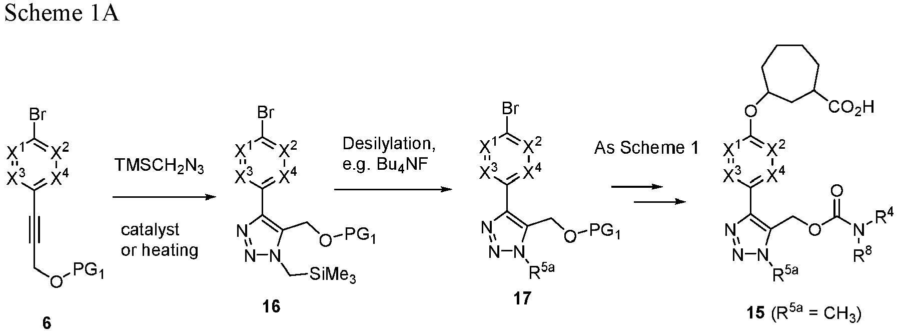

Scheme 2 describes the synthesis of N-carbamoyl triazole-aryloxy cycloheptyl acids 20. Triazole alcohol 12 is oxidized to the triazole acid 18 (e.g. directly to the acid with pyridinium dichromate or via a 2-step procedure via the aldehyde [Swern oxidation or Dess-Martin periodinane followed by NaClO₂ oxidation to the acid, e.g. Lindgren, B. O., Acta Chem. Scand. 1973, 27, 888]). Curtius rearrangement (e.g. with (PhO)₂PON₃) of acid 18 in the presence of an alcohol R⁴-OH provides the triazole NH-carbamate 19. Deprotection of the triazole NH-carbamate ester 19 provides the cycloheptyl triazole NH-carbamoyl acids 20.

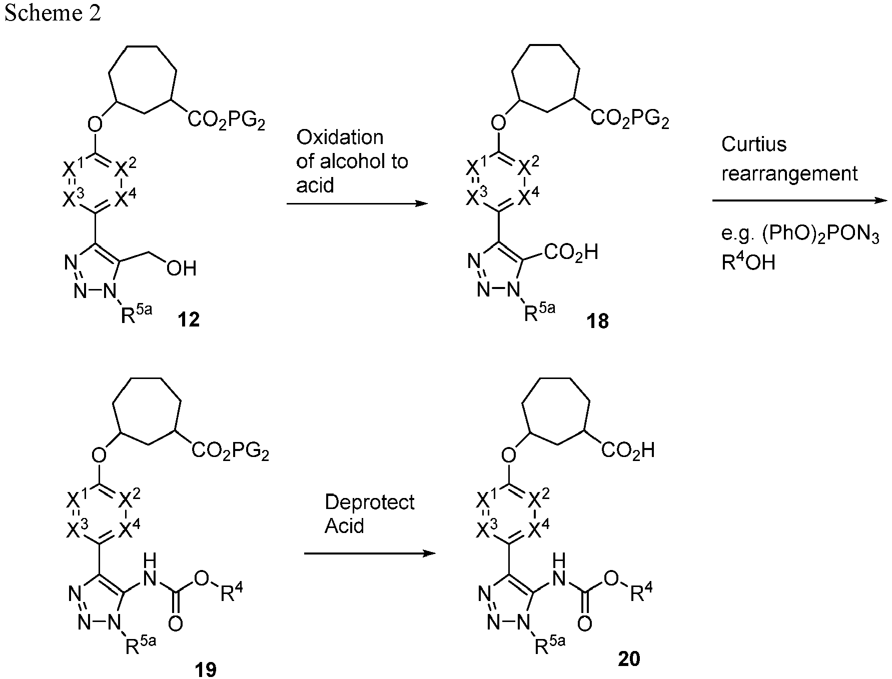

Scheme 3 describes the synthesis of triazole N-carbamoyl-aryloxy cycloheptyl acids 24. Cycloheptyl ester triazole alcohol 12 is reacted with a brominating agent (e.g. PBr₃ or CBr₄/ Ph₃P) to give the corresponding bromide 21. Displacement of bromide 21 with NaN₃ (or another appropriate azide reagent) provides the corresponding azide, which undergoes reduction (e.g. Staudinger reduction with Ph₃P/H₂O) to afford the triazole amine 22. Amine 22 is reacted with an appropriate chloroformate 23 (or the corresponding 4-nitrophenyl carbonate) in the presence of an appropriate base to give the corresponding NH-carbamate. Ester deprotection of this triazole N-H carbamate ester provides the desired triazole-carbamate cycloheptyl acids 24.

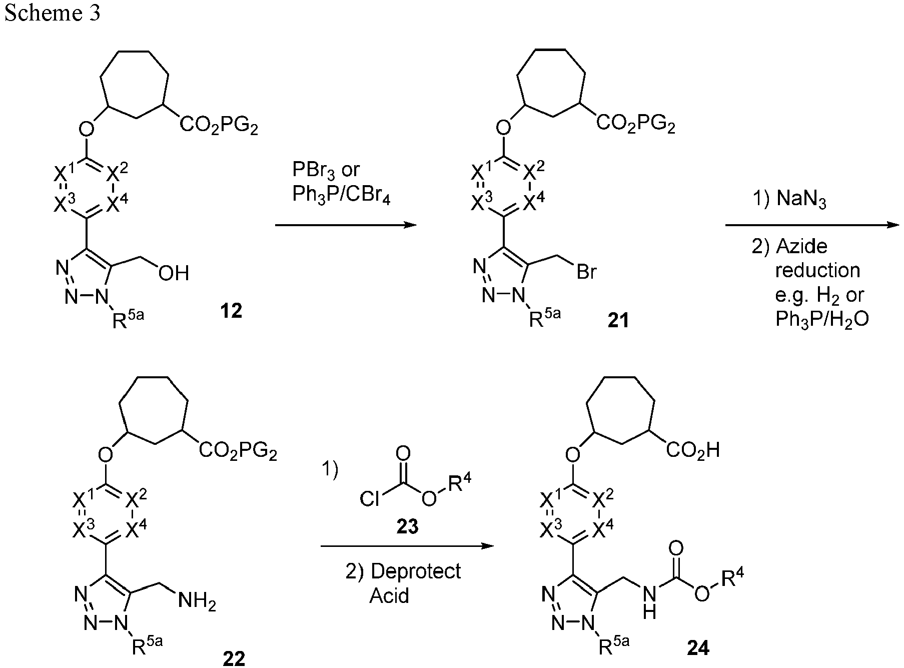

Scheme 4 describes the synthesis of: 1) amino-azine triazole aryloxy cycloheptyl acids 27 and 2) oxy-azine triazole aryloxy cycloheptyl acids 29, wherein one of A¹, A² and A³ is N and the other two are CR. Cycloheptyl triazole amine 22 is reacted with halo-azine 25 in the presence of an appropriate base or via transition-metal catalyzed amination (e.g. Lim, C.-H., et al., J. Am. Chem. Soc., 2018, 140, 7667-7673) to give the triazole amino-azine 26, which is then subjected to ester deprotection to provide the desired amino-azine triazole aryloxy cycloheptyl acids 27. Cycloheptyl triazole alcohol 12 is reacted with halo-azine 25 in the presence of an appropriate base or via transition-metal catalyzed C-O cross-coupling (e.g. Maligres, P. E., et al., Angew. Chem. Int. Ed., 2012, 51, 9071-9074) to give the triazole oxy-azine 28, which is then subjected to ester deprotection to provide the oxy-azine triazole aryloxy cycloheptyl acids 29.

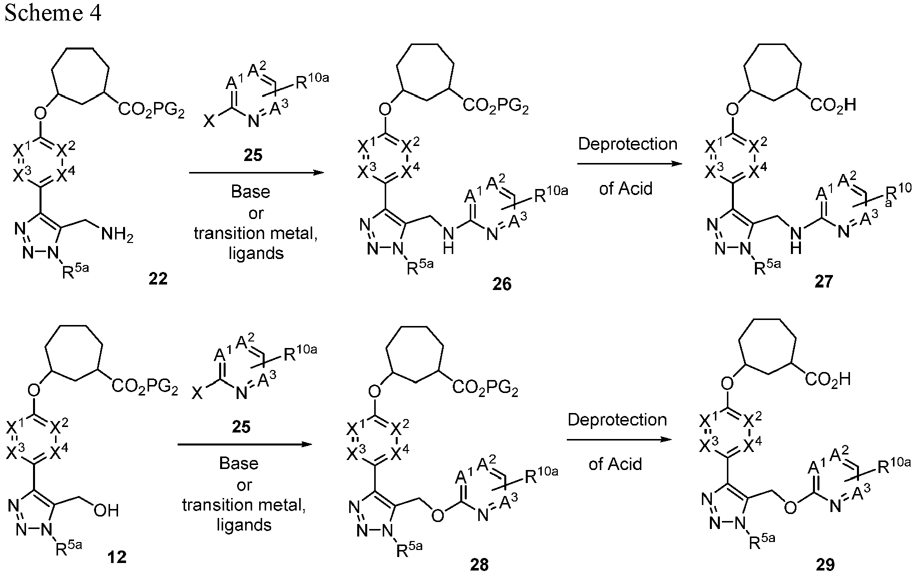

Scheme 5 describes the synthesis of: 1) amino-azole triazole aryloxy cycloheptyl acids 32 and 2) oxy-azole triazole aryloxy cycloheptyl acids 34, wherein A⁴, A⁵, A⁶ and A⁷ are N or CR. Cycloheptyl triazole amine 22 is reacted with an appropriate halo-azole 30 in the presence of an appropriate base or via transition metal-catalyzed amination to give the triazole amino-azole 31, which is then subjected to ester deprotection to provide the desired amino-azole triazole aryloxy cycloheptyl acids 32. Cycloheptyl triazole alcohol 12 is reacted with an appropriate halo-azole 30 in the presence of an appropriate base or via transition metal-catalyzed C-O cross-coupling to give the triazole oxy-azole 33, which is then subjected to ester deprotection to provide the oxy-azole triazole aryloxy cycloheptyl acids 34.

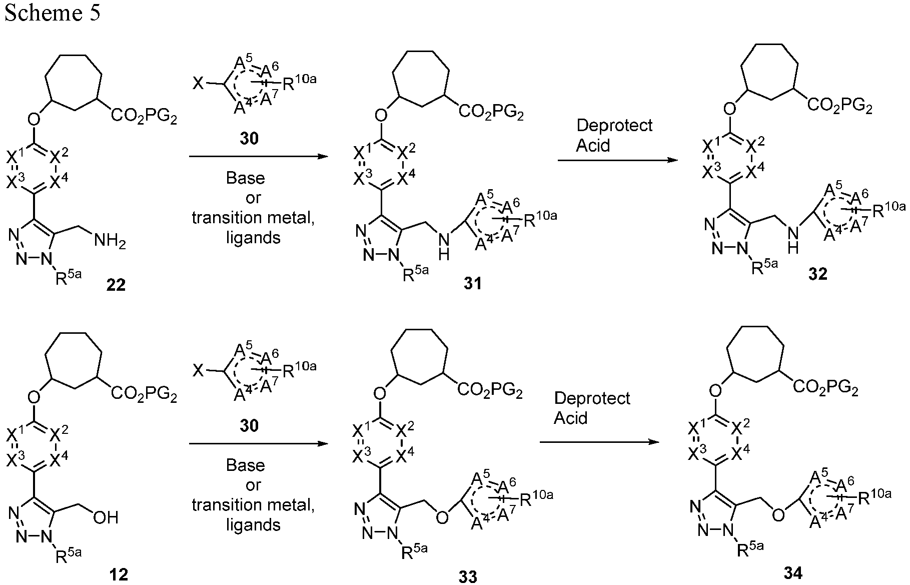

### VII. EXAMPLES

The following Examples are offered as illustrative, as a partial scope and particular embodiments of the invention and are not meant to be limiting of the scope of the invention. Abbreviations and chemical symbols have their usual and customary meanings unless otherwise indicated. Unless otherwise indicated, the compounds described herein have been prepared, isolated and characterized using the schemes and other methods disclosed herein or may be prepared using the same.

As appropriate, reactions were conducted under an atmosphere of dry nitrogen (or argon). For anhydrous reactions, DRISOLV^{®} solvents from EM were employed. For other reactions, reagent grade or HPLC grade solvents were utilized. Unless otherwise stated, all commercially obtained reagents were used as received.

Microwave reactions were carried out using a 400W Biotage Initiator instrument in microwave reaction vessels under microwave (2.5 GHz) irradiation.

HPLC/MS and preparatory/analytical HPLC methods employed in characterization or purification of examples.

NMR (nuclear magnetic resonance) spectra were typically obtained on Bruker or JEOL 400 MHz and 500 MHz instruments in the indicated solvents. All chemical shifts are reported in ppm from tetramethylsilane with the solvent resonance as the internal standard. In the examples where ¹H NMR spectra were collected in d₆-DMSO, a water-suppression sequence is often utilized. This sequence effectively suppresses the water signal and any proton peaks in the same region usually between 3.30-3.65 ppm which will affect the overall proton integration.

¹HNMR spectral data are typically reported as follows: chemical shift, multiplicity (s = singlet, br s = broad singlet, d = doublet, dd = doublet of doublets, t = triplet, q = quartet, sep = septet, m = multiplet, app = apparent), coupling constants (Hz), and integration.

The term HPLC refers to a Shimadzu high performance liquid chromatography instrument with one of following methods:
HPLC-1: Sunfire C18 column (4.6 × 150 mm) 3.5 µm, gradient from 10 to 100% B:A for 12 min, then 3 min hold at 100% B.
   Mobile phase A: 0.05% TFA in water:CH₃CN (95:5)
   Mobile phase B: 0.05% TFA in CH₃CN:water (95:5)
   TFA Buffer pH = 2.5; Flow rate: 1 mL/ min; Wavelength: 254 nm, 220 nm.
HPLC-2: XBridge Phenyl (4.6 × 150 mm) 3.5 µm, gradient from 10 to 100% B:A for 12 min, then 3 min hold at 100% B.
   Mobile phase A: 0.05% TFA in water:CH₃CN (95:5)
   Mobile phase B: 0.05% TFA in CH₃CN:water (95:5)
   TFA Buffer pH = 2.5; Flow rate: 1 mL/ min; Wavelength: 254 nm, 220 nm.
HPLC-3: Chiralpak AD-H, 4.6 × 250 mm, 5 µm.
   Mobile Phase: 30% EtOH-heptane (1:1) / 70% CO₂
   Flow rate = 40 mL/min, 100 Bar, 35 °C; Wavelength: 220 nm
HPLC-4: Waters Acquity UPLC BEH C18, 2.1 × 50 mm, 1.7-µm particles;
   Mobile Phase A: 5:95 CH₃CN:water with 10 mM NH₄OAc;
   Mobile Phase B: 95:5 CH₃CN:water with 10 mM NH₄OAc;
   Temperature: 50 °C; Gradient: 0-100% B over 3 min, then a 0.75-min hold at 100% B; Flow: 1.11 mL/min; Detection: UV at 220 nm.
HPLC-5: Waters Acquity UPLC BEH C18, 2.1 × 50 mm, 1.7-µm particles;
   Mobile Phase A: 5:95 CH₃CN:water with 0.1% TFA;
   Mobile Phase B: 95:5 CH₃CN:water with 0.1% TFA;
   Temperature: 50 °C; Gradient: 0-100% B over 3 min, then a 0.75-min hold at 100% B; Flow: 1.11 mL/min; Detection: UV at 220 nm.

### Intermediate 1. Methyl 3-((6-(5-(hydroxymethyl)-1-methyl-1H-1,2,3-triazol-4-yl)-2-methylpyridin-3-yl)oxy)cycloheptane-1-carboxylate

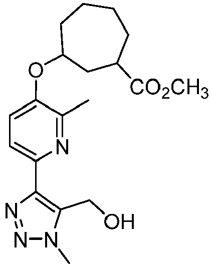

### Intermediate 1A. 3-vinylcycloheptan-1-one

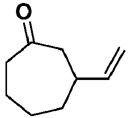

To a solution of cyclohept-2-enone (1.45 g, 13.16 mmol) in THF (65.8 ml) was added CuBr•SMe₂ complex (0.27 g, 1.3 mmol) under Ar at RT and the reaction was stirred at RT for 15 min, then was cooled to -40°C. Vinylmagnesium bromide (19.7 mL of a 1M solution in THF; 19.7 mmol) was added over 1 h via syringe pump at -40°C. The reaction mixture was stirred at -40°C for 1 h, then was quenched with satd aq. NH₄Cl at -40°C. The mixture was allowed to warm to RT and extracted with EtOAc (50 mL × 2). The combined organic extracts were dried (Na₂SO₄) and concentrated in vacuo. The crude product was chromatographed (80 g SiO₂ ISCO column; continuous gradient from 0% to 50% EtOAc in hexanes over 20 min) to give the title compound (800 mg, 5.79 mmol, 44.0 % yield). ¹H NMR (400 MHz, CDCl₃) δ 5.89 - 5.74 (m, 1H), 5.10 - 4.94 (m, 2H), 2.61 - 2.49 (m, 4H), 2.46 - 2.35 (m, 1H), 2.03 - 1.86 (m, 3H), 1.72 - 1.60 (m, 1H), 1.52 - 1.40 (m, 2H).

### Intermediate 1B. 3-vinylcycloheptan-1-ol

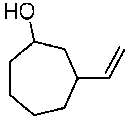

To a solution of Intermediate 1A (500 mg, 3.62 mmol) in THF (10 mL) and H₂O (10 mL) was slowly added NaBH₄ (547 mg, 14.5 mmol) at 23 °C and the reaction was stirred at RT overnight. Aq. 10% KHSO₄ (10 mL) was added slowly to the reaction mixture, which was then extracted with EtOAc (2×30 mL). The combined organic extracts were dried (Na₂SO₄) and concentrated in vacuo. The residue was chromatographed (12 g SiO₂; continuous gradient from 0-60% EtOAc in hexanes over 10 min, then continuous gradient from 60-100% EtOAc in hexanes over 10 min) to give the title compound (500 mg, 3.57 mmol, 99 % yield). ¹H NMR (400 MHz, CDCl₃) δ 5.94 - 5.74 (m, 1H), 5.07 - 4.83 (m, 2H), 4.09 - 3.81 (m, 1H), 2.53 - 2.10 (m, 1H), 2.04 - 1.90 (m, 1H), 1.84 - 1.68 (m, 3H), 1.64 - 1.46 (m, 4H), 1.42 - 1.18 (m, 3H).

### 1C. 2-methyl-6-(1-methyl-5-(((tetrahydro-2H-pyran-2-yl)oxy)methyl)-1H-1,2,3-triazol-4-yl)-3-((3-vinylcycloheptyl)oxy)pyridine

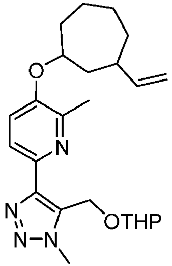

To a solution of (E)-diazene-1,2-diylbis(piperidin-1-ylmethanone) (1.40 g, 5.65 mmol) in 1,4-dioxane (10 mL) was added nBu₃P (1.4 mL, 5.65 mmol). The resulting mixture was stirred at RT for 5 min, then was added to a solution of 2-methyl-6-(1-methyl-5-(((tetrahydro-2H-pyran-2-yl)oxy)methyl)-1H-1,2,3-triazol-4-yl)pyridin-3-ol (synthesized according to the procedure described for Example 1C in WO2017/223016; 860 mg, 2.83 mmol) and Intermediate 1B (396 mg, 2.83 mmol) in 1,4-dioxane (28 mL) at RT over 30 min. The reaction mixture was heated at 75 °C for 16 h, then was cooled to RT. The mixture was filtered and the filter cake was rinsed with DCM (10 mL); the combined filtrates were concentrated in vacuo. The residue was chromatographed (40 g SiO₂; continuous gradient from 0-60% EtOAc in hexanes over 10 min, then continuous gradient from 60-100% EtOAc in hexanes over 10 min) to give the title compound (1.11 g, 2.60 mmol, 92 % yield). ¹H NMR (400 MHz; CDCl₃) δ: 7.94 (dd, J=8.6, 2.4 Hz, 1H), 7.13 (dd, J=12.8, 8.6 Hz, 1H), 5.77-5.95 (m, 1H), 5.23-5.42 (m, 2H), 4.95-5.06 (m, 1H), 4.92 (dq, J=10.3, 1.3 Hz, 1H), 4.78 (t, J=3.5 Hz, 1H), 4.38-4.65 (m, 1H), 4.16 (s, 3H), 3.91 (ddd, J=11.3, 7.8, 3.1 Hz, 1H), 3.46-3.63 (m, 1H), 2.46-2.52 (m, 3H), 2.15 (br s, 2H), 1.63-1.98 (m, 10H), 1.40-1.58 (m, 5H).

### Intermediate 1

To a solution of Intermediate 1C (1.0 g, 2.34 mmol) in MeCN (5 mL) were successively added a solution of NaIO₄ (2.0 g, 9.38 mmol) in H₂O (5 mL) and RuCl₃ (0.025 g, 0.117 mmol). The reaction mixture was stirred at RT for 1 h, then was concentrated in vacuo. The residue was purified by preparative HPLC (C18 30 × 100 mm column; detection at 220 nm; flow rate = 40 mL/min; continuous gradient from 0% B to 100% B over 10 min + 2 min hold time at 100% B, where A = 90:10:0.1 H₂O:MeCN:TFA and B = 90:10:0.1 MeCN:H₂O:TFA) to give the crude acid product as a clear oil. To a solution of the crude acid (0.30 g, 0.832 mmol) in 1:1 DCM/ MeOH (5 mL each) at 0°C was added TMSCHN₂ (0.50 mL of a 2 M solution in Et₂O, 1.00 mmol). The reaction mixture was stirred at RT overnight, then was concentrated in vacuo. The crude product was chromatographed (SiO₂; continuous gradient from 0% to 100% EtOAc in hexanes over 20 min) to give the title compound (190 mg, 20% over 2 steps). ¹H NMR (400 MHz, CDCl₃) δ 8.25 - 8.03 (m, 1H), 7.26 - 7.12 (m, 1H), 4.93 - 4.81 (m, 2H), 4.76 - 4.35 (m, 1H), 4.11 - 4.06 (m, 3H), 3.73 - 3.66 (m, 3H), 2.92 - 2.81 (m, 1H), 2.62 - 2.44 (m, 4H), 2.47 - 2.37 (m, 1H), 2.30 - 2.13 (m, 1H), 2.04 - 1.95 (m, 1H), 1.95 - 1.71 (m, 5H), 1.68 - 1.52 (m, 2H).

### Intermediate 2. 4-(5-((3-(methoxycarbonyl)cycloheptyl)oxy)-6-methylpyridin-2-yl)-1-methyl-1H-1,2,3-triazole-5-carboxylic acid

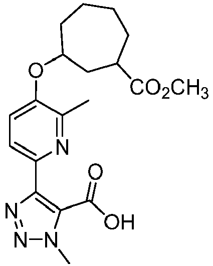

To a solution of Intermediate 1 (47 mg, 0.13 mmol) in MeCN (0.5 mL) was added a solution of NaIO₄ (107 mg, 0.502 mmol) in H₂O (0.5 mL) and RuCl₃ (1.3 mg, 6.3 µmol). The reaction mixture was stirred at RT for 1 h, then was concentrated in vacuo. The residue was purified by preparative HPLC (C18 30 × 100 mm column; detection at 220 nm; flow rate = 40 mL/min; continuous gradient from 0% B to 100% B over 10 min + 2 min hold time at 100% B, where A = 90:10:0.1 H₂O:MeCN:TFA and B = 90:10:0.1 MeCN:H₂O:TFA) to give the title compound (37 mg, 0.095 mmol, 76 % yield) as a clear oil. LCMS [M + H]⁺ = 389.2.

### Example 1. (±)-3-((6-(5-(((benzyl(methyl)carbamoyl)oxy)methyl)-1-methyl-1H-1,2,3-triazol-4-yl)-2-methylpyridin-3-yl)oxy)cycloheptane-1-carboxylic acid (mixture of cis & trans diastereomers)

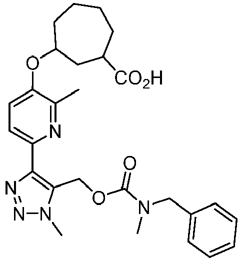

### 1A. Methyl 3-((2-methyl-6-(1-methyl-5-((((4-nitrophenoxy)carb onyl)oxy)methyl)-1H-1,2,3 - triazol-4-yl)pyridin-3-yl)oxy)cycloheptane-1-carboxylate

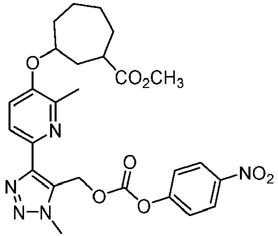

To a solution of Intermediate 1 (95 mg, 0.25 mmol) and pyridine (0.062 mL, 0.76 mmol) in DCM (2 mL) was added 4-nitrophenyl chloroformate (102 mg, 0.507 mmol). The reaction mixture was stirred at RT for 18 h, then was filtered and concentrated in vacuo to give the title compound (90 mg, 0.17 mmol, 66% yield) as a light yellow oil. LCMS [M + H]⁺ = 540.2

### 1B. Methyl 3-((6-(5-(((benzyl(methyl)carbamoyl)oxy)methyl)-1-methyl-1H-1,2,3-triazol-4-yl)-2-methylpyridin-3-yl)oxy)cycloheptane-1-carboxylate

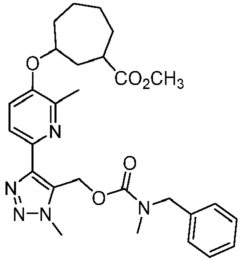

To a solution of compound 1A (27 mg, 0.050 mmol) in THF (1 mL) was added N-methyl-1-phenylmethanamine (7.3 mg, 0.060 mmol) followed by iPr₂NEt (0.022 mL, 0.12 mmol). The reaction mixture was stirred at RT for 18 h, then was concentrated in vacuo. The crude product was chromatographed (4 g SiO₂; continuous gradient from 0% to 100% EtOAc in hexane over 14 min) to give the title compound (25 mg, 0.17 mmol, 96% yield) as a light yellow oil. LCMS [M + H]⁺ = 522.3

### Example 15. (±)-3-((6-(5-(((6-ethylpyrimidin-4-yl)oxy)methyl)-1-methyl-1H-1,2,3-triazol-4-yl)-2-methylpyridin-3-yl)oxy)cycloheptane-1-carboxylic acid (mixture of cis & trans diastereomers)

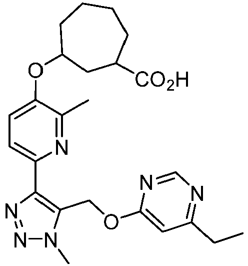

To a solution of Intermediate 1 (10 mg, 0.027 mmol) in 1,4-dioxane (1.5 mL) at 0°C was added NaH (10.6 mg, 0.27 mmol). The mixture was stirred for 10 min at RT, after which 4-chloro-6-ethylpyrimidine (7.6 mg, 0.053 mmol) was added. The mixture was stirred in a microwave reactor at 120°C for 1h, then was cooled to RT. Water (1 mL) was added, and the mixture was stirred at RT for 1h, then was concentrated in vacuo. The crude product was purified by preparative HPLC (C18 30 × 100 mm column; detection at 220 nm; flow rate = 40 mL/min; continuous gradient from 0% B to 100% B over 10 min + 2 min hold time at 100% B, where A = 90:10:0.1 HzO:MeCN:TFA and B = 90:10:0.1 MeCN:HzO:TFA) to give the title compound (8.7 mg, 88 % yield) as a clear oil. ¹H NMR (500 MHz, DMSO-d₆) δ 8.81 - 8.64 (m, 1H), 8.00 - 7.77 (m, 1H), 7.56 - 7.36 (m, 1H), 6.92 - 6.72 (m, 1H), 6.13 - 5.92 (m, 2H), 4.83 - 4.52 (m, 1H), 4.20 - 4.01 (m, 3H), 2.76 - 2.59 (m, 3H), 2.31 - 2.16 (m, 3H), 2.18 - 2.07 (m, 1H), 2.09 - 1.84 (m, 2H), 1.82 - 1.38 (m, 7H), 1.27 - 1.06 (m, 3H). ); LCMS, [M + H]⁺ =467.3; hLPA₁ Antagonist IC₅₀ = 32 nM.

The compounds listed in Table 2 were prepared by using same synthetic sequence and the same intermediates as described for the synthesis of Example 15.

**Table 2**

| Ex # | Structure & Name | Analytical Data | Method |
|---|---|---|---|
| 16 | 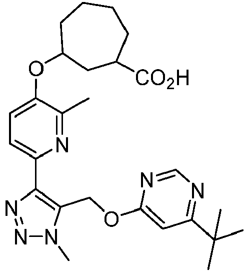 (±)-3-((6-(5-(((6-(tert-butyl)pyrimidin-4-yl)oxy)methyl)-1-methyl-1H-1,2,3-triazol-4-yl)-2-methylpyridin-3 - yl)oxy)cycloheptane-1-carboxylic acid (mixture of cis & trans diastereomers) | LCMS, [M + H]⁺ = 495.3; ¹H NMR (500 MHz, DMSO-d₆) δ 8.86 - 8.68 (m, 1H), 7.89 - 7.76 (m, 1H), 7.52 - 7.39 (m, 1H), 6.91 - 6.75 (m, 1H), 6.12 - 5.94 (m, 2H), 4.83 - 4.67 (m, 1H), 4.20 - 4.07 (m, 3H), 3.59 - 3.45 (m, 1H), 2.72 - 2.59 (m, 1H), 2.27 - 2.15 (m, 3H), 2.12 - 2.01 (m, 1H), 2.00 - 1.83 (m, 3H), 1.80 - 1.35 (m, 6H), 1.28 - 1.10 (m, 9H); | Example 15 |
| | | hLPA₁ IC₅₀ = 154 nM. | |
| 17 | 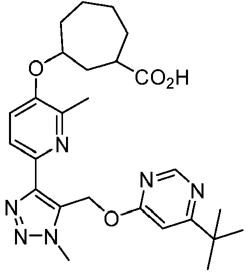 (±)-3-((6-(5-(((6-(tert-butyl) pyrimidin-4-yl)oxy)methyl)-1 -methyl- 1H-1,2,3 -triazol-4-yl)-2-methylpyridin-3-yl)oxy)cycloheptane-1-carboxylic acid (mixture of cis & trans diastereomers) | LCMS, [M + H]⁺ = 495.3.; ¹H NMR (500 MHz, DMSO-d₆) δ 8.83 - 8.70 (m, 1H), 7.92 - 7.78 (m, 1H), 7.50 - 7.36 (m, 1H), 6.97 - 6.77 (m, 1H), 6.11 - 5.96 (m, 2H), 4.61 - 4.47 (m, 1H), 4.21 - 4.03 (m, 3H), 3.66 - 3.31 (m, 3H), 2.46 - 2.33 (m, 1H), 2.30 - 2.11 (m, 3H), 2.00 - 1.88 (m, 2H), 1.80 - 1.39 (m, 6H), 1.31 - 1.06 (m, 9H); | Example 15 |
| | | hLPA₁ IC₅₀ = 325 nM. | |
| 18 | 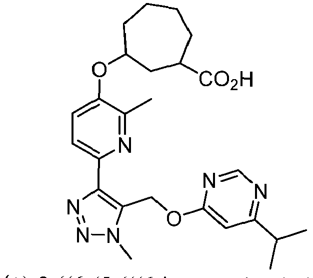 (±)-3-((6-(5-(((6-isopropylpyrimidin-4-yl)oxy)methyl)-1-methyl-1H-1,2,3-triazol-4-yl)-2-methylpyridin-3 - yl)oxy)cycloheptane-1-carboxylic acid (diastereomer 1) | LCMS, [M + H]⁺ = 481.3; ¹H NMR (500 MHz, DMSO-d₆) δ 8.81 - 8.61 (m, 1H), 7.92 - 7.69 (m, 1H), 7.50 - 7.33 (m, 1H), 6.88 - 6.73 (m, 1H), 6.05 - 5.90 (m, 2H), 4.62 - 4.47 (m, 1H), 4.19 - 4.05 (m, 3H), 3.70 - 3.39 (m, 3H), 2.96 - 2.77 (m, 1H), 2.30 - 2.09 (m, 2H), 2.01 - 1.83 (m, 3H), 1.81 - 1.40 (m, 6H), 1.21 - 1.04 (m, 6H); | Example 15 |
| | | hLPA₁ IC₅₀ = 145 nM. | |
| 19 | 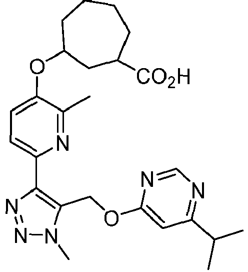 (±)-3-((6-(5-(((6-isopropylpyrimidin-4-yl)oxy)methyl)-1-methyl- 1H-1,2,3 triazol-4-yl)-2-methylpyridin-3 - yl)oxy)cycloheptane-1-carboxylic acid (diastereomer 2) | LCMS, [M + H]⁺ = 481.3; ¹H NMR (500 MHz, DMSO-d₆) δ 8.79 - 8.63 (m, 1H), 7.90 - 7.75 (m, 1H), 7.55 - 7.37 (m, 1H), 6.87 - 6.71 (m, 1H), 6.10 - 5.90 (m, 2H), 4.87 - 4.68 (m, 1H), 4.16 - 4.05 (m, 3H), 3.61 - 3.47 (m, 1H), 2.91 - 2.80 (m, 1H), 2.67 - 2.57 (m, 1H), 2.30 - 2.18 (m, 2H), 2.14 - 2.02 (m, 1H), 1.99 - 1.79 (m, 4H), 1.77 - 1.33 (m, 6H), 1.24 - 1.04 (m, 6H); | Example 15 |
| | | hLPA₁ IC₅₀ = 48 nM. | |
| 20 | 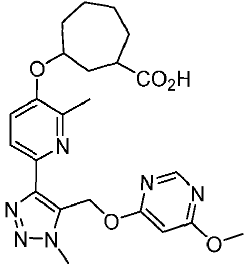 (±)3-((6-(5-(((6-methoxypyrimidin-4-yl)oxy)methyl)-1-methyl- 1H-1,2,3 triazol-4-yl)-2-methylpyridin-3 - yl)oxy)cycloheptane-1-carboxylic acid (diastereomer 1) | LCMS, [M + H]⁺ = 469.3; ¹H NMR (500 MHz, DMSO-d₆) δ 8.55 - 8.38 (m, 1H), 7.91 - 7.76 (m, 1H), 7.51 - 7.32 (m, 1H), 6.41 - 6.23 (m, 1H), 6.05 - 5.87 (m, 2H), 4.61 - 4.46 (m, 1H), 4.18 - 4.00 (m, 3H), 3.91 - 3.78 (m, 3H), 3.76 - 3.48 (m, 2H), 2.29 - 2.17 (m, 4H), 1.99 - 1.85 (m, 2H), 1.85 - 1.44 (m, 7H); | Example 15 |
| | | hLPA₁ IC₅₀ = 67 nM. | |
| 21 | 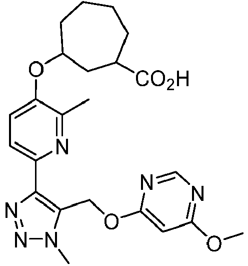 (±)3-((6-(5-(((6-methoxypyrimidin-4-yl)oxy)methyl)-1-methyl-1H-1,2,3-triazol-4-yl)-2-methylpyridin-3-yl)oxy)cycloheptane-1-carboxylic acid (diastereomer 2) | LCMS, [M + H]⁺ = 469.3; ¹H NMR (500 MHz, DMSO-d₆) δ 8.58 - 8.45 (m, 1H), 7.86 - 7.78 (m, 1H), 7.50 - 7.41 (m, 1H), 6.35 - 6.24 (m, 1H), 6.03 - 5.89 (m, 2H), 4.81 - 4.69 (m, 1H), 4.17 - 4.00 (m, 3H), 3.91 - 3.77 (m, 3H), 3.71 - 3.48 (m, 2H), 2.73 - 2.59 (m, 1H), 2.29 - 2.20 (m, 2H), 2.13 - 2.04 (m, 1H), 1.99 - 1.90 (m, 3H), 1.82 - 1.35 (m, 6H); | Example 15 |
| | | hLPA₁ IC₅₀ = 26 nM. | |

### Example 22. (±)-3-((2-methyl-6-(1-methyl-5-((((R)-1-phenylethoxy)carbonyl)amino)-1H-1,2,3-triazol-4-yl)pyridin-3-yl)oxy)cycloheptane-1-carboxylic acid (mixture of cis & trans diastereomers)

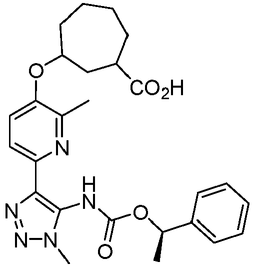

A mixture of Intermediate 2 (8 mg, 0.021 mmol), (PhO)₂PON₃ (6.8 mg, 0.025 mmol), (R)-1-phenylethan-1-ol (2.8 mg, 0.023 mmol) and Et₃N (3.7 µL, 0.027 mmol) in toluene (1 mL) was stirred at 80 °C for 4 h, then was cooled to RT and concentrated in vacuo. The crude product was chromatographed (4 g SiO₂; continuous gradient from 0% to 100% EtOAc in hexane over 14 min) to give N-carbamate triazole ester crude product (9 mg, 0.018 mmo, 86% yield) as a light yellow oil. LCMS [M + H]⁺ = 508.3. This material was used in the next step without further purification.

The crude N-carbamate triazole ester was dissolved in THF (1 mL) and aq. 2 M LiOH (0.044 mL, 0.089 mmol) and the reaction mixture was stirred at RT for 18 h, then was concentrated *in vacuo.* The residue was dissolved in H₂O (1 mL), and the pH was adjusted with 1N aq. HCl to ~3 and extracted with EtOAc (2 × 1 mL). The combined organic extracts were washed with brine (1 mL), dried (MgSO₄) and concentrated *in vacuo.* The crude product was purified by preparative LC/MS: Column: Waters XBridge C18, 19 × 200 mm, 5-µm particles; Guard Column: Waters XBridge C18, 19 × 10 mm, 5-µm particles; Mobile Phase A: 5:95 MeCN:H₂O with 0.1% TFA; Mobile Phase B: 95:5 MeCN:H₂O with 0.1% TFA; Gradient: 50-90% B over 20 min, then a 5-min hold at 100% B; Flow: 20 mL/min. Fractions containing the desired product were combined and concentrated *in vacuo* by centrifugal evaporation to give the title compound (7.7 mg, 70% yield). LCMS [M + H]⁺ = 494.3; ¹H NMR (500 MHz, CDCl₃) δ: 7.99-8.10 (m, 1H), 7.90 (d, J=9.2 Hz, 1H), 7.63 (dd, J=8.9, 4.1 Hz, 1H), 7.37 (br d, J=4.6 Hz, 4H), 7.32 (dt, J=4.4, 2.2 Hz, 1H), 5.81 (q, J=6.5 Hz, 1H), 4.55-4.93 (m, 1H), 3.95-4.07 (m, 3H), 2.84-2.94 (m, 1H), 2.59-2.73 (m, 4H), 2.20-2.42 (m, 1H), 2.11-2.20 (m, 2H), 1.94-2.06 (m, 1H), 1.75-1.95 (m, 4H), 1.63-1.72 (m, 1H), 1.60 (br d, J=6.6 Hz, 3H); hLPA₁ IC₅₀ = 53 nM.

The compounds listed in Table 3 were prepared by using the same synthetic sequence and the same intermediates as described for the synthesis of Example 22.

**Table 3**

| Ex # | Structure & Name | Analytical Data | Method |
|---|---|---|---|
| 23 | 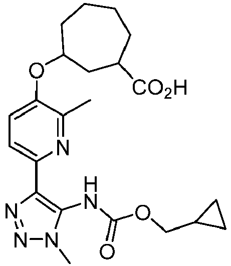 (±)-(((cyclopropylmethoxy) carbonyl)amino)-1 -methyl- 1H-1,2,3-triazol-4-yl)-2-methylpyridin-3-yl)oxy) cycloheptane-1-carboxylic acid (mixture of cis & trans diastereomers) | LCMS [M + H]⁺ = 444.3; ¹H NMR (CDCl₃) δ: 8.02-8.16 (m, 1H), 7.63-8.02 (m, 1H), 4.55-4.92 (m, 1H), 4.06 (d, J=1.1 Hz, 3H), 4.00 (d, J=7.3 Hz, 2H), 2.87-2.94 (m, 0.5H), 2.74 (d, J=7.7 Hz, 3H), 2.59-2.68 (m, 0.5H), 2.23-2.46 (m, 1H), 2.08-2.22 (m, 3H), 1.95-2.08 (m, 1H), 1.75-1.95 (m, 4H), 1.48-1.73 (m, 2H), 1.11-1.23 (m, 1H), 0.55-0.68 (m, 2H), 0.32 (q, J=5.1 Hz, 2H); | Example 22 |
| | | hLPA₁ IC₅₀ = 127 nM . | |
| 24 | 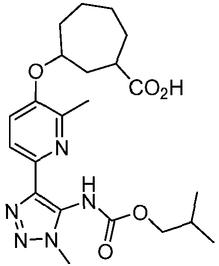 (±)-3-((6-(5-((isobutoxycarbonyl) amino)-1-methyl-1H-1,2,3-triazol-4-yl)-2-methylpyridin-3-yl)oxy) cycloheptane-1-carboxylic acid (mixture of cis & trans diastereomers) | LCMS [M + H]⁺ = 446.3; ¹H NMR (CDCl₃) δ: 8.07-8.18 (m, 1H), 8.01 (s, 1H), 7.72 (d, J=9.0 Hz, 1H), 4.64-4.92 (m, 1H), 4.06 (d, J=0.9 Hz, 3H), 3.94 (br d, J=6.6 Hz, 2H), 2.84-2.99 (m, 0.5H), 2.74 (d, J=6.6 Hz, 3H), 2.64-2.69 (m, 0.5H), 2.63 (d, J=9.2 Hz, 1H), 2.23-2.44 (m, 1H), 2.12-2.20 (m, 2H), 1.96-2.07 (m, 2H), 1.77-1.92 (m, 4H), 1.55-1.72 (m, 2H), 0.96 (d, J=6.6 Hz, 6H); | Example 22 |
| | | hLPA₁ IC₅₀ = 91 nM. | |

## Claims

1. A compound of Formula (I): 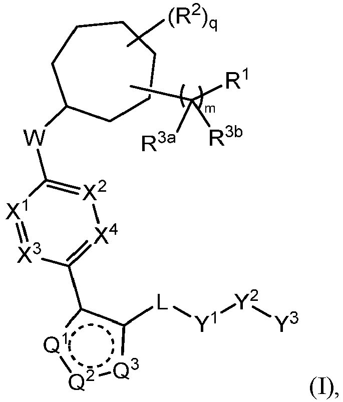 or a stereoisomer, tautomer, or pharmaceutically acceptable salt or solvate thereof, wherein:
X¹, X², X³, and X⁴ are each independently CR⁶ or N; provided that no more than two of X¹, X², X³, or X⁴ are N;
the 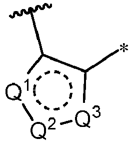 moiety is independently 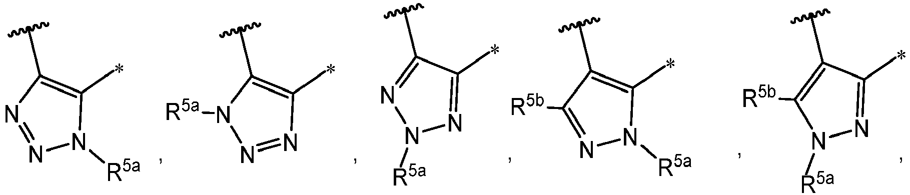 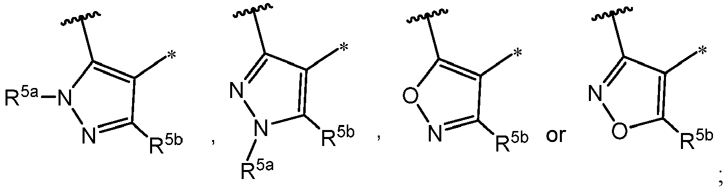
* denotes the attachment point to L;
L is independently a covalent bond or CH₂;
WisO;
the 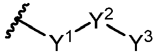 moiety is independently -OR^{4b}, -NR⁷R^{4b}, 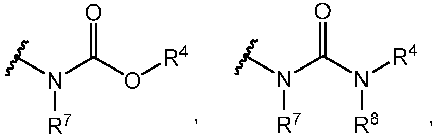 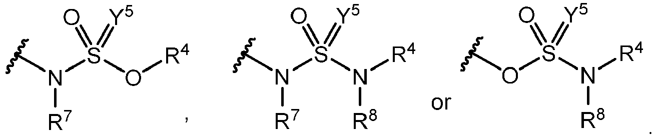
Y⁵ independently is O or NH;
R¹ is CO₂H;
R² is each independently halo, cyano, hydroxyl, amino, C₁₋₄ alkoxy, C₁₋₄ haloalkyl, C₁₋₄ haloalkoxy, C₁₋₄ alkylamino, -(CH₂)₀₋₁-(C₃₋₆ cycloalkyl), -(CH₂)₀₋₁-phenyl, or C₁₋₆ alkyl;
R^{3a} is independently hydrogen, halo, hydroxyl, or C₁₋₄ alkyl;
R^{3b} is independently hydrogen, halo, cyano, hydroxyl, amino, C₁₋₄ alkyl, C₁₋₄ haloalkyl, C₁₋₄ alkoxy, C₁₋₄ haloalkoxy or C₁₋₆ alkyl substituted with 0 to 2 R^{a};
or alternatively, R^{3a} and R^{3b} together, with the carbon atom they are attached to, form a C₃₋₄ carbocyclyl;
R⁴ is -L₁-R^{4a};
L₁ is independently a covalent bond or C₁₋₄ alkylene substituted with 0 to 4 R⁹;
R^{4a} is independently C₁₋₁₀ alkyl, C₁₋₁₀ haloalkyl, C₁₋₁₀ alkenyl, C₃₋₈ cycloalkyl, C₆₋₁₀ aryl, 3 to 8-membered heterocyclyl, 5 to 6-membered heteroaryl; wherein each of the alkyl, alkenyl, alkylene, cycloalkyl, aryl, heterocyclyl, and heteroaryl, by itself or as part of other moiety, is independently substituted with 0 to 3 R¹⁰;
R^{4b} is independently C₁₋₁₀ alkyl, C₁₋₁₀ haloalkyl, C₁₋₁₀ alkenyl, C₃₋₈ cycloalkyl, C₆₋₁₀ aryl, 3 to 8-membered heterocyclyl, 5 to 6-membered heteroaryl; wherein each of the alkyl, alkenyl, alkylene, cycloalkyl, aryl, heterocyclyl, and heteroaryl, by itself or as part of other moiety, is independently substituted with 0 to 3 R^{10a};
R^{5a} is independently hydrogen, C₁₋₄ haloalkyl, -(CH₂)₀₋₁-(C₃₋₆ cycloalkyl), -(CH₂)₀₋₁-phenyl, or C₁₋₆ alkyl substituted with 0 to 3 R^{a};
R^{5b} is independently hydrogen, halo, cyano, hydroxyl, amino, C₁₋₄ alkoxy, C₁₋₄ haloalkyl, C₁₋₄ haloalkoxy, C₁₋₄ alkylamino, -(CH₂)₀₋₁-(C₃₋₆ cycloalkyl), -(CH₂)₀₋₁-phenyl, or C₁₋₆ alkyl substituted with 0 to 3 R^{b};
R⁶ is each independently hydrogen, halo, cyano, hydroxyl, amino, C₁₋₄ alkylamino, C₁₋₄ haloalkyl, C₁₋₄ alkoxy, C₁₋₄ haloalkoxy or C₁₋₆ alkyl substituted with 0 to 1 R^{b};
R⁷ and R⁸ are each independently hydrogen, C₁₋₆ alkyl, C₁₋₄ haloalkyl, C₃₋₆ cycloalkyl or C₁₋₆ alkyl;
R⁹ is each independently halo, oxo, cyano, hydroxyl, amino, C₁₋₄ haloalkyl, C₁₋₄ alkoxy, C₁₋₄ haloalkoxy, C₃₋₆ cycloalkyl, or C₁₋₆ alkyl substituted with 0 to 3 R^{a};
R¹⁰ and R^{10a} are each independently halo, hydroxyl, amino, cyano, C₂₋₆ alkenyl, C₂₋₆ alkynyl, C₁₋₄ alkylamino, C₁₋₄ haloalkyl, C₁₋₄ alkoxy, C₁₋₄ haloalkoxy, phenyl, or 5 to 6 membered heteroaryl, C₁₋₆ alkyl substituted with 0 to 3 R^{b};
R^{a} is independently halo, cyano, hydroxyl, C₁₋₄ alkoxy, C₁₋₄ haloalkyl, or C₁₋₄ haloalkoxy;
R^{b} is independently halo, cyano, hydroxyl, amino, C₁₋₄ alkoxy, C₁₋₄ haloalkyl, or C₁₋₄ haloalkoxy;
m is an integer of 0, 1, or 2; and
q is an integer of 0, 1, or 2.

2. The compound according to claim 1, wherein:
R^{3a} and R^{3b} are independently hydrogen or C₁₋₄ alkyl;
or alternatively, R^{3a} and R^{3b} together, with the carbon atom they are attached to, form C₃₋₄ cycloalkyl.

3. The compound according to claim 1 or claim 2, wherein:
X¹, X², X³, and X⁴ are independently CR⁶; or X¹, X² and X³ are CR⁶ and X⁴ is N; or X² and X³ are CR⁶ and X¹ and X⁴ are N; or X¹ and X² are CR⁶ and X³ and X⁴ are N; and R⁶ is independently hydrogen, halo, hydroxyl, C₁₋₆ alkyl, C₁₋₄ haloalkyl, or C₁₋₄ alkoxy.

4. The compound according to any one of claims 1 to 3, wherein:
X¹, X², X³, and X⁴ are independently CR⁶; or X¹, X² and X³ are CR⁶ and X⁴ is N; or X² and X³ are CR⁶ and X¹ and X⁴ are N;
the 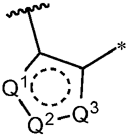 moiety is independently 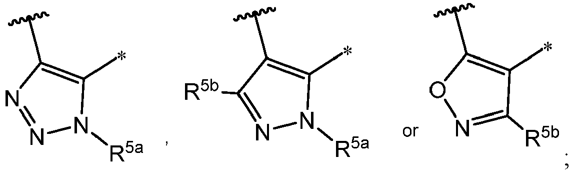
^{∗} denotes the attachment point to L;
the 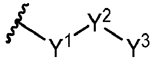 moiety is independently -OR^{4b}, or 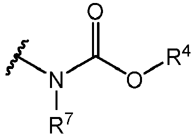
R² is each independently halo, cyano, hydroxyl, C₁₋₄ alkoxy, C₁₋₄ haloalkyl or C₁₋₄ haloalkoxy;
R⁴ is independently C₃₋₆ alkyl, -(CH₂)₀₋₁-C₃₋₆ cycloalkyl, -(CH(C₁₋₂ alkyl))-C₃₋₆ cycloalkyl, -(CH₂)₀₋₁-phenyl or -(CH(C₁₋₂ alkyl))-phenyl, wherein each of said cycloalkyl and phenyl is independently substituted with 0 to 3 R¹⁰;
R^{4b} is independently 5 to 6-membered heteroaryl substituted with 0 to 2 R^{10a};
R^{5a} is independently C₁₋₆ alkyl, or -(CH₂)₀₋₁-(C₃₋₆ cycloalkyl);
R^{5b} is independently hydrogen, halo, cyano, hydroxyl, C₁₋₄ alkyl, C₁₋₄ alkoxy, C₁₋₄ haloalkyl, C₁₋₄ haloalkoxy, or -(CH₂)₀₋₁-(C₃₋₆ cycloalkyl);
R⁶ is each independently hydrogen, halo, hydroxyl, C₁₋₆ alkyl, C₁₋₄ haloalkyl, or C₁₋₄ alkoxy;
R⁷ and R⁸ are each independently hydrogen or C₁₋₂ alkyl;
R¹⁰ is each independently halo, cyano, hydroxyl, C₁₋₆ alkyl, C₁₋₆ haloalkyl, C₁₋₆ alkoxy, or C₁₋₆ haloalkoxy;
R^{10a} is each independently halo, cyano, hydroxyl, C₁₋₆ alkyl, C₁₋₆ haloalkyl, C₁₋₆ alkoxy, C₁₋₆ haloalkoxy, -(CH₂)₀₋₃-C₃₋₆ cycloalkyl, phenyl or 5 to 6 membered heteroaryl;
m is 0 or 1; and
q is 0 or 1.

5. The compound according to any one of claims 1 to 4, wherein:
the 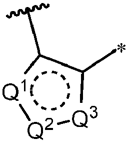 moiety is independently 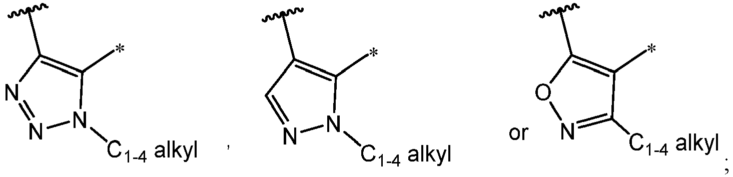
^{∗} denotes the attachment point to L;
the 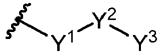 moiety is independently -OR^{4b}, or 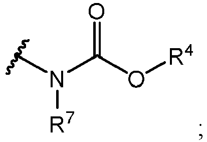
R^{3a} and R^{3b} are each independently hydrogen or C₁₋₄ alkyl;
or alternatively, R^{3a} and R^{3b} together, with the carbon atom they are attached to, form C₃₋₄ cycloalkyl;
R⁴ is independently C₃₋₆ alkyl, -(CH₂)₀₋₁-C₃₋₆ cycloalkyl, -(CH₂)-phenyl, or -(CH(CH₃))-phenyl; wherein wherein each of said alkyl, cycloalkyl and phenyl is independently substituted with 0 to 2 R¹⁰;
R^{4b} is independently 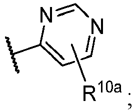
R⁶ is independently hydrogen, halo or C₁₋₄ alkyl;
R⁷ and R⁸ are each independently hydrogen or C₁₋₂ alkyl;
R¹⁰ is each independently halo or C₁₋₄ alkyl; and
R^{10a} is independently C₁₋₄ alkyl, C₁₋₄ alkoxy, -(CH₂)₁₋₃-C₃₋₆ cycloalkyl, phenyl or pyridyl.

6. A compound according to claim 1, which is a compound of Formula (II): 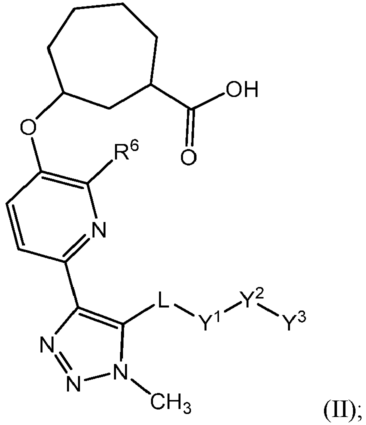 or a stereoisomer, tautomer, or pharmaceutically acceptable salt or solvate thereof, wherein:
the 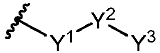 moiety is independently -OR^{4b}, or 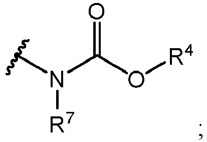
R⁴ is independently C₃₋₆ alkyl, -(CH₂)₀₋₁-C₃₋₆ cycloalkyl, -(CH₂)-phenyl, or -(CH(CH₃))-phenyl;
R^{4b} is independently 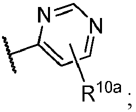
R⁶ is independently hydrogen, halo or C₁₋₄ alkyl;
R⁷ and R⁸ are each independently hydrogen or methyl;
R^{10a} is independently C₁₋₄ alkyl or C₁₋₄ alkoxy.

7. The compound according to claim 1, which is selected from:
(±)-3-((6-(5-(((6-ethylpyrimidin-4-yl)oxy)methyl)-1-methyl-1H-1,2,3-triazol-4-yl)-2-methylpyridin-3-yl)oxy)cycloheptane-1-carboxylic acid;
(±)-3-((6-(5-(((6-(tert-butyl)pyrimidin-4-yl)oxy)methyl)-1-methyl-1H-1,2,3-triazol-4-yl)-2-methylpyridin-3-yl)oxy)cycloheptane-1-carboxylic acid;
(±)-3-((6-(5-(((6-(tert-butyl) pyrimidin-4-yl)oxy)methyl)-1-methyl-1H-1,2,3-triazol-4-yl)-2-methylpyridin-3-yl)oxy)cyclo- heptane-1-carboxylic acid;) (±)-3-((6-(5-(((6-isopropylpyrimidin-4-yl)oxy)methyl)-1-methyl-1H-1,2,3-triazol-4-yl)-2-methylpyridin-3-yl)oxy)cycloheptane-1-carboxylic acid;
(±)3-((6-(5-(((6-methoxypyrimidin-4-yl)oxy)methyl)-1-methyl-1H-1,2,3-triazol-4-yl)-2-methylpyridin-3-yl)oxy)cycloheptane-1-carboxylic acid;
(±)-3-((2-methyl-6-(1-methyl-5-((((R)-1-phenylethoxy)carbonyl)amino)-1H-1,2,3-triazol-4-yl)pyridin-3-yl)oxy)cycloheptane-1-carboxylic acid;
(±)-(((cyclopropylmethoxy) carbonyl)amino)-1-methyl-1H-1,2,3-triazol-4-yl)-2-methylpyridin-3-yl)oxy) cycloheptane-1-carboxylic acid;
(±)-3-((6-(5-((isobutoxycarbonyl)amino)-1-methyl-1H-1,2,3-triazol-4-yl)-2-methylpyridin-3-yl)oxy) cycloheptane-1-carboxylic acid;
or a stereoisomer, a tautomer, or a pharmaceutically acceptable salt or solvate thereof.

8. A pharmaceutical composition comprising one or more compounds according to any one of claims 1 to 7, or a stereoisomer, tautomer, or pharmaceutically acceptable salt or solvate thereof; and a pharmaceutically acceptable carrier or diluent.

9. A compound or a stereoisomer, tautomer, or pharmaceutically acceptable salt or solvate thereof according to any one of claims 1 to 7, or a pharmaceutical composition according to claim 8, for use in therapy.

10. A compound or a stereoisomer, tautomer, or pharmaceutically acceptable salt or solvate thereof according to any one of claims 1 to 7, or a pharmaceutical composition according to claim 8, for use in treating a disease, disorder, or condition associated with dysregulation of lysophosphatidic acid receptor 1 (LPA1), wherein the disease, disorder, or condition is related to pathological fibrosis, transplant rejection, cancer, osteoporosis, or inflammatory disorders.

11. The compound or composition for use according to claim 10, wherein the pathological fibrosis is pulmonary, liver, renal, cardiac, dernal, ocular, or pancreatic fibrosis.

12. The compound or composition for use according to claim 10, wherein the disease, disorder, or condition is idiopathic pulmonary fibrosis (IPF), non-alcoholic steatohepatitis (NASH), non-alcoholic fatty liver disease (NAFLD), chronic kidney disease, diabetic kidney disease, or systemic sclerosis.

13. The compound or composition for use according to claim 10, wherein the cancer is of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid.

14. A compound or a stereoisomer, tautomer, or pharmaceutically acceptable salt or solvate thereof according to any one of claims 1 to 7, or a pharmaceutical composition according to claim 8, for use in treating fibrosis in a mammal, the use comprising administering a therapeutically effective amount of the compound or the pharmaceutical composition, to the mammal in need thereof, preferably wherein the fibrosis is idiopathic pulmonary fibrosis (IPF), nonalcoholic steatohepatitis (NASH), chronic kidney disease, diabetic kidney disease, or systemic sclerosis.

15. A compound or a stereoisomer, tautomer, or pharmaceutically acceptable salt or solvate thereof according to any one of claims 1 to 7, or a pharmaceutical composition according to claim 8, for use in treating lung fibrosis (idiopathic pulmonary fibrosis), asthma, chronic obstructive pulmonary disease (COPD), renal fibrosis, acute kidney injury, chronic kidney disease, liver fibrosis (non-alcoholic steatohepatitis), skin fibrosis, fibrosis of the gut, breast cancer, pancreatic cancer, ovarian cancer, prostate cancer, glioblastoma, bone cancer, colon cancer, bowel cancer, head and neck cancer, melanoma, multiple myeloma, chronic lymphocytic leukemia, cancer pain, tumor metastasis, transplant organ rejection, scleroderma, ocular fibrosis, age related macular degeneration (AMD), diabetic retinopathy, collagen vascular disease, atherosclerosis, Raynaud's phenomenon, or neuropathic pain in a mammal, the use ccomprising administering a therapeutically effective amount of the compound or the pharmaceutical composition, to the mammal in need thereof.

## Patentansprüche

1. Verbindung der Formel (I): 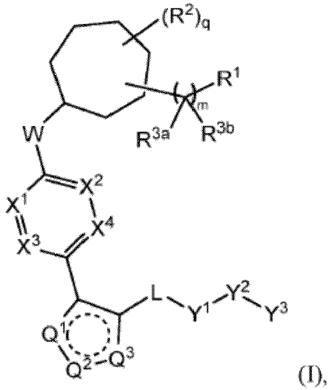 oder ein Stereoisomer, Tautomer, oder pharmazeutisch verträgliches Salz oder Solvat davon, wobei:
X¹, X², X³ und X⁴ jeweils unabhängig CR⁶ oder N sind; mit der Maßgabe, dass nicht mehr als zwei von X¹, X², X³ oder X⁴ N sind;
der 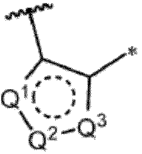 Teil unabhängig ist 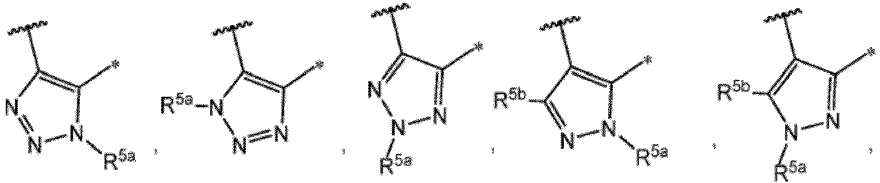 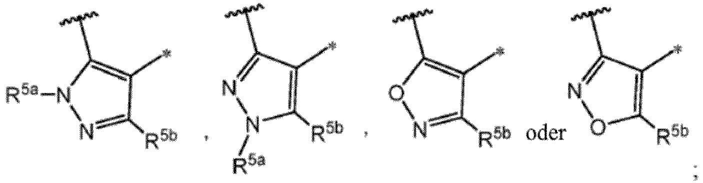
^{∗} den Punkt der Anknüpfung an L bezeichnet;
L unabhängig eine kovalente Bindung oder CH₂ ist;
W O ist;
der 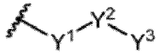 Teil unabhängig -OR^{4b}, -NR⁷R^{4b}, 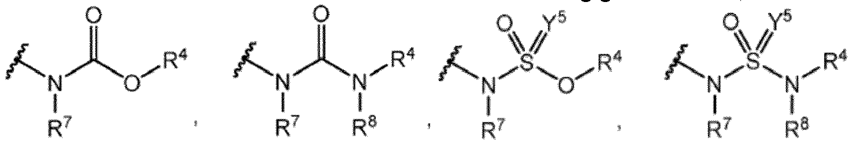 oder 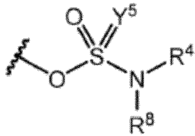 ist;
Y⁵ unabhängig O oder NH ist;
R¹ CO₂H ist;
R² jeweils unabhängig Halogen, Cyano, Hydroxyl, Amino, C₁₋₄-Alkoxy, C₁₋₄-Halogenalkyl, C₁₋₄-Halogenalkoxy, C₁₋₄-Alkylamino, -(CH₂)₀₋₁-(C₃₋₆-Cycloalkyl), -(CH₂)₀₋₁-Phenyl oder C₁₋₆-Alkyl ist;
R^{3a} unabhängig Wasserstoff, Halogen, Hydroxyl oder C₁₋₄-Alkyl ist;
R^{3b} unabhängig Wasserstoff, Halogen, Cyano, Hydroxyl, Amino, C₁₋₄-Alkyl, C₁₋₄-Halogenalkyl, C₁₋₄-Alkoxy, C₁₋₄-Halogenalkoxy oder C₁₋₆-Alkyl, substituiert mit 0 bis 2 R^{a}, ist;
oder alternativ R^{3a} und R^{3b} zusammen, mit dem Kohlenstoffatom, an das sie gebunden sind, ein C₃₋₄-Carbocyclyl bilden;
R⁴ -L₁-R^{4a} ist;
L₁ unabhängig eine kovalente Bindung oder C₁₋₄-Alkylen, substituiert mit 0 bis 4 R⁹, ist;
R^{4a} unabhängig C₁₋₁₀-Alkyl, C₁₋₁₀-Halogenalkyl, C₁₋₁₀-Alkenyl, C₃₋₈-Cycloalkyl, C₆₋₁₀-Aryl, 3- bis 8-gliedriges Heterocyclyl, 5- bis 6-gliedriges Heteroaryl ist; wobei jedes von dem Alkyl, Alkenyl, Alkylen, Cycloalkyl, Aryl, Heterocyclyl und Heteroaryl, für sich allein oder als Bestandteil eines anderen Teils, unabhängig substituiert ist mit 0 bis 3 R¹⁰;
R^{4b} unabhängig C₁₋₁₀-Alkyl, C₁₋₁₀-Halogenalkyl, C₁₋₁₀-Alkenyl, C₃₋₈-Cycloalkyl, C₆₋₁₀-Aryl, 3- bis 8-gliedriges Heterocyclyl, 5- bis 6-gliedriges Heteroaryl ist; wobei jedes von dem Alkyl, Alkenyl, Alkylen, Cycloalkyl, Aryl, Heterocyclyl und Heteroaryl, für sich allein oder als Bestandteil eines anderen Teils, unabhängig substituiert ist mit 0 bis 3 R^{10a};
R^{5a} unabhängig Wasserstoff, C₁₋₄-Halogenalkyl, -(CH₂)₀₋₁-(C₃₋₆-Cycloalkyl), -(CH₂)₀₋₁-Phenyl oder C₁₋₆-Alkyl, substituiert mit 0 bis 3 R^{a}, ist;
R^{5b} unabhängig Wasserstoff, Halogen, Cyano, Hydroxyl, Amino, C₁₋₄-Alkoxy, C₁₋₄-Halogenalkyl, C₁₋₄-Halogenalkoxy, C₁₋₄-Alkylamino, -(CH₂)₀₋₁-(C₃₋₆-Cycloalkyl), -(CH₂)₀₋₁-Phenyl oder C₁₋₆-Alkyl, substituiert mit 0 bis 3 R^{b}, ist;
R⁶ jeweils unabhängig Wasserstoff, Halogen, Cyano, Hydroxyl, Amino, C₁₋₄-Alkylamino, C₁₋₄-Halogenalkyl, C₁₋₄-Alkoxy, C₁₋₄-Halogenalkoxy oder C₁₋₆-Alkyl, substituiert mit 0 bis 1 R^{b}, ist;
R⁷ und R⁸ jeweils unabhängig Wasserstoff, C₁₋₆-Alkyl, C₁₋₄-Halogenalkyl, C₃₋₆-Cycloalkyl oder C₁₋₆-Alkyl sind;
R⁹ jeweils unabhängig Halogen, Oxo, Cyano, Hydroxyl, Amino, C₁₋₄-Halogenalkyl, C₁₋₄-Alkoxy, C₁₋₄-Halogenalkoxy, C₃₋₆-Cycloalkyl oder C₁₋₆-Alkyl, substituiert mit 0 bis 3 R^{a}, ist;
R¹⁰ und R^{10a} jeweils unabhängig Halogen, Hydroxyl, Amino, Cyano, C₂₋₆-Alkenyl, C₂₋₆-Alkinyl, C₁₋₄-Alkylamino, C₁₋₄-Halogenalkyl, C₁₋₄-Alkoxy, C₁₋₄-Halogenalkoxy, Phenyl oder 5- bis 6-gliedriges Heteroaryl, C₁₋₆-Alkyl, substituiert mit 0 bis 3 R^{b}, sind;
R^{a} unabhängig Halogen, Cyano, Hydroxyl, C₁₋₄-Alkoxy, C₁₋₄-Halogenalkyl oder C₁₋₄-Halogenalkoxy ist;
R^{b} unabhängig Halogen, Cyano, Hydroxyl, Amino, C₁₋₄-Alkoxy, C₁₋₄-Halogenalkyl oder C₁₋₄-Halogenalkoxy ist;
m eine ganze Zahl von 0, 1 oder 2 ist; und
q eine ganze Zahl von 0, 1 oder 2 ist.

2. Verbindung nach Anspruch 1, wobei:
R^{3a} und R^{3b} unabhängig Wasserstoff oder C₁₋₄-Alkyl sind;
oder alternativ R^{3a} und R^{3b} zusammen, mit dem Kohlenstoffatom, an das sie gebunden sind, C₃₋₄-Cycloalkyl bilden.

3. Verbindung nach Anspruch 1 oder Anspruch 2, wobei:
X¹, X², X³ und X⁴ unabhängig CR⁶ sind; oder X¹, X² und X³ CR⁶ sind und X⁴ N ist; oder X² und X³ CR⁶ sind und X¹ und X⁴ N sind; oder X¹ und X² CR⁶ sind und X³ und X⁴ N sind; und R⁶ unabhängig Wasserstoff, Halogen, Hydroxyl, C₁₋₆-Alkyl, C₁₋₄-Halogenalkyl oder C₁₋₄-Alkoxy ist.

4. Verbindung nach einem der Ansprüche 1 bis 3, wobei:
X¹, X², X³ und X⁴ unabhängig CR⁶ sind; oder X¹, X² und X³ CR⁶ sind und X⁴ N ist; oder X² und X³ CR⁶ sind und X¹ und X⁴ N sind;
der 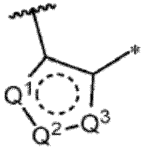 Teil unabhängig 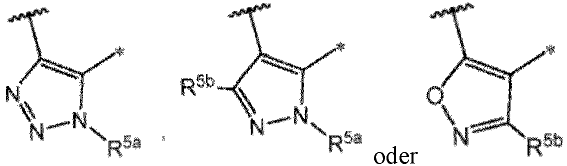 ist;
^{∗} den Punkt der Anknüpfung an L bezeichnet;
der 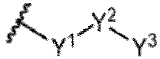 Teil unabhängig -OR^{4b} oder 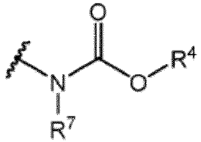 ist;
R² jeweils unabhängig Halogen, Cyano, Hydroxyl, C₁₋₄-Alkoxy, C₁₋₄-Halogenalkyl oder C₁₋₄-Halogenalkoxy ist;
R⁴ unabhängig C₃₋₆-Alkyl, -(CH₂)₀₋₁-C₃₋₆-Cycloalkyl, -(CH(C₁₋₂-Alkyl))-C₃₋₆-cycloalkyl, -(CH₂)₀₋₁-Phenyl oder -(CH(C₁₋₂-Alkyl))-phenyl ist, wobei jedes von dem Cycloalkyl und Phenyl unabhängig substituiert ist mit 0 bis 3 R¹⁰;
R^{4b} unabhängig 5- bis 6-gliedriges Heteroaryl, substituiert mit 0 bis 2 R^{10a}, ist;
R^{5a} unabhängig C₁₋₆-Alkyl oder -(CH₂)₀₋₁-(C₃₋₆-Cycloalkyl) ist;
R^{5b} unabhängig Wasserstoff, Halogen, Cyano, Hydroxyl, C₁₋₄-Alkyl, C₁₋₄-Alkoxy, C₁₋₄-Halogenalkyl, C₁₋₄-Halogenalkoxy oder -(CH₂)₀₋₁-(C₃₋₆-Cycloalkyl) ist;
R⁶ jeweils unabhängig Wasserstoff, Halogen, Hydroxyl, C₁₋₆-Alkyl, C₁₋₄-Halogenalkyl oder C₁₋₄-Alkoxy ist;
R⁷ und R⁸ jeweils unabhängig Wasserstoff oder C₁₋₂-Alkyl sind;
R¹⁰ jeweils unabhängig Halogen, Cyano, Hydroxyl, C₁₋₆-Alkyl, C₁₋₆-Halogenalkyl, C₁₋₆-Alkoxy oder C₁₋₆-Halogenalkoxy ist;
R^{10a} jeweils unabhängig Halogen, Cyano, Hydroxyl, C₁₋₆-Alkyl, C₁₋₆-Halogenalkyl, C₁₋₆-Alkoxy, C₁₋₆-Halogenalkoxy, -(CH₂)₀₋₃-C₃₋₆-Cycloalkyl, Phenyl oder 5- bis 6-gliedriges Heteroaryl ist;
m 0 oder 1 ist; und
q 0 oder 1 ist.

5. Verbindung nach einem der Ansprüche 1 bis 4, wobei:
der 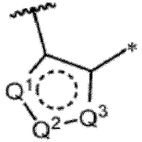 Teil unabhängig 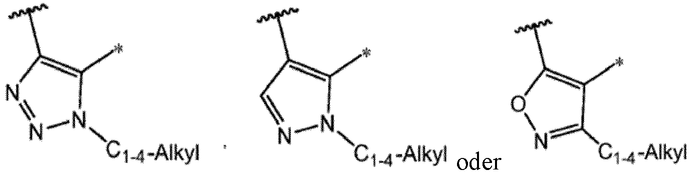 ist;
^{∗} den Punkt der Anknüpfung an L bezeichnet;
der 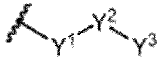 Teil unabhängig -OR^{4b} oder 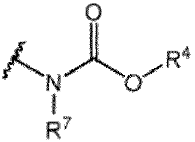 ist;
R^{3a} und R^{3b} jeweils unabhängig Wasserstoff oder C₁₋₄-Alkyl sind;
oder alternativ R^{3a} und R^{3b} zusammen, mit dem Kohlenstoffatom, an das sie gebunden sind, C₃₋₄-Cycloalkyl bilden;
R⁴ unabhängig C₃₋₆-Alkyl, -(CH₂)₀₋₁-C₃₋₆-Cycloalkyl, -(CH₂)-Phenyl oder -(CH(CH₃))-Phenyl ist; wobei jedes von dem Alkyl, Cycloalkyl und Phenyl unabhängig substituiert ist mit 0 bis 2 R¹⁰;
R^{4b} unabhängig 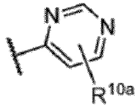 ist;
R⁶ unabhängig Wasserstoff, Halogen oder C₁₋₄-Alkyl ist;
R⁷ und R⁸ jeweils unabhängig Wasserstoff oder C₁₋₂-Alkyl sind;
R¹⁰ jeweils unabhängig Halogen oder C₁₋₄-Alkyl ist; und
R^{10a} unabhängig C₁₋₄-Alkyl, C₁₋₄-Alkoxy, -(CH₂)₁₋₃-C₃₋₆-Cycloalkyl, Phenyl oder Pyridyl ist.

6. Verbindung nach Anspruch 1, die eine Verbindung der Formel (II) ist: 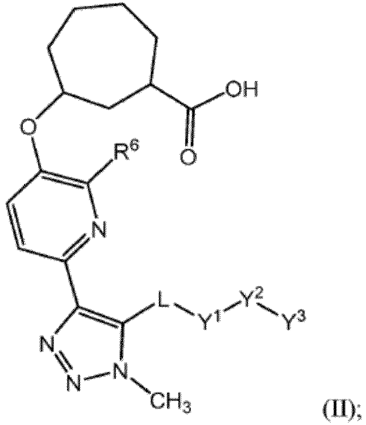
oder ein Stereoisomer, Tautomer, oder pharmazeutisch verträgliches Salz oder Solvat davon, wobei:
der 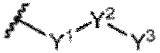 Teil unabhängig -OR^{4b} oder 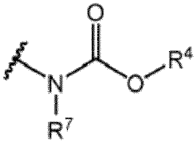 ist;
R⁴ unabhängig C₃₋₆-Alkyl, -(CH₂)₀₋₁-C₃₋₆-Cycloalkyl, -(CH₂)-Phenyl oder -(CH(CH₃))-Phenyl ist;
R^{4b} unabhängig 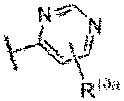 ist;
R⁶ unabhängig Wasserstoff, Halogen oder C₁₋₄-Alkyl ist;
R⁷ und R⁸ jeweils unabhängig Wasserstoff oder Methyl sind;
R^{10a} unabhängig C₁₋₄-Alkyl oder C₁₋₄-Alkoxy ist.

7. Verbindung nach Anspruch 1, die ausgewählt ist aus:
(±)-3-((6-(5-(((6-Ethylpyrimidin-4-yl)oxy)methyl)-1-methyl-1H-1,2,3-triazol-4-yl)-2-methylpyridin-3-yl)oxy)cycloheptan-1-carbonsäure;
(±)-3-((6-(5-(((6-(tert-Butyl)pyrimidin-4-yl)oxy)methyl)-1-methyl-1H-1,2,3-triazol-4-yl)-2-methylpyridin-3-yl)oxy)cycloheptan-1-carbonsäure;
(±)-3-((6-(5-(((6-(tert-Butyl)pyrimidin-4-yl)oxy)methyl)-1-methyl-1H-1,2,3-triazol-4-yl)-2-methylpyridin-3-yl)oxy)cyclo-heptan-1-carbonsäure;
(±)-3-((6-(5-(((6-(Isopropylpyrimidin-4-yl)oxy)methyl)-1-methyl-1H-1,2,3-triazol-4-yl)-2-methylpyridin-3-yl)oxy)cycloheptan-1-carbonsäure, (±)-3 -((6-(5-(((6-Methoxypyrimidin-4-yl)oxy)methyl)-1-methyl-1H-1,2,3-triazol-4-yl)-2-methylpyridin-3-yl)oxy)cycloheptan-1-carbonsäure;
(±)-3-((2-Methyl-6-(1-methyl-5-((((R)-1-phenylethoxy)carbonyl)amino)-1H-1,2,3-triazol-4-yl)pyridin-3-yl)oxy)cycloheptan-1-carbonsäure;
(±)-(((Cyclopropylmethoxy)carbonyl)amino)-1-methyl-1H-1,2,3-triazol-4-yl)-2-methylpyridin-3-yl)oxy)cycloheptan-1-carbonsäure;
(±)-3-((6-(5-((Isobutoxycarbonyl)amino)-1-methyl-1H-1,2,3-triazol-4-yl)-2-methylpyridin-3-yl)oxy)cycloheptan-1-carbonsäure;
oder ein Stereoisomer, ein Tautomer, oder ein pharmazeutisch verträgliches Salz oder Solvat davon.

8. Pharmazeutische Zusammensetzung, umfassend eine oder mehrere Verbindungen nach einem der Ansprüche 1 bis 7, oder ein Stereoisomer, Tautomer, oder pharmazeutisch verträgliches Salz oder Solvat davon; und einen pharmazeutisch verträglichen Träger oder ein pharmazeutisch verträgliches Verdünnungsmittel.

9. Verbindung oder ein Stereoisomer, Tautomer, oder pharmazeutisch verträgliches Salz oder Solvat davon nach einem der Ansprüche 1 bis 7, oder pharmazeutische Zusammensetzung nach Anspruch 8, zur Verwendung in der Therapie.

10. Verbindung oder ein Stereoisomer, Tautomer, oder pharmazeutisch verträgliches Salz oder Solvat davon nach einem der Ansprüche 1 bis 7, oder pharmazeutische Zusammensetzung nach Anspruch 8, zur Verwendung bei der Behandlung einer Erkrankung, einer Störung oder eines Zustands, assoziiert mit Dysregulation des Lysophosphatidsäure-Rezeptors 1 (LPA1), wobei die Erkrankung, die Störung oder der Zustand mit pathologischer Fibrose, Transplantatabstoßung, Krebs, Osteoporose oder entzündlichen Störungen in Zusammenhang steht.

11. Verbindung oder Zusammensetzung zur Verwendung nach Anspruch 10, wobei die pathologische Fibrose Lungen-, Leber-, Nieren-, Herz-, Haut-, Augen- oder Pankreasfibrose ist.

12. Verbindung oder Zusammensetzung zur Verwendung nach Anspruch 10, wobei die Erkrankung, die Störung oder der Zustand idiopathische pulmonale Fibrose (IPF), nichtalkoholische Steatohepatitis (NASH), nichtalkoholische Fettlebererkrankung (NAFLD), chronische Nierenerkrankung, diabetische Nierenerkrankung oder systemische Sklerose ist.

13. Verbindung oder Zusammensetzung zur Verwendung nach Anspruch 10, wobei der Krebs Blasen-, Blut-, Knochen-, Hirn-, Brust-, Zentralnervensystem-, Zervix-, Kolon-, Endometrium-, Speiseröhren-, Gallenblasen-, Genital-, Urogenitaltrakt-, Kopf-, Nieren-, Kehlkopf-, Leber-, Lungen-, Muskelgewebs-, Hals-, Mund- oder Nasenschleimhaut-, Eierstock-, Pankreas-, Prostata-, Haut-, Milz-, Dünndarm-, Dickdarm-, Magen-, Hoden- oder Schilddrüsenkrebs ist.

14. Verbindung oder ein Stereoisomer, Tautomer, oder pharmazeutisch verträgliches Salz oder Solvat davon nach einem der Ansprüche 1 bis 7, oder pharmazeutische Zusammensetzung nach Anspruch 8, zur Verwendung bei der Behandlung von Fibrose bei einem Säuger, wobei die Verwendung das Verabreichen einer therapeutisch wirksamen Menge der Verbindung oder der pharmazeutischen Zusammensetzung, an den Säuger mit Bedarf daran, umfasst, wobei, vorzugsweise, die Fibrose idiopathische pulmonale Fibrose (IPF), nichtalkoholische Steatohepatitis (NASH), chronische Nierenerkrankung, diabetische Nierenerkrankung oder systemische Sklerose ist.

15. Verbindung oder ein Stereoisomer, Tautomer, oder pharmazeutisch verträgliches Salz oder Solvat davon nach einem der Ansprüche 1 bis 7, oder pharmazeutische Zusammensetzung nach Anspruch 8, zur Verwendung bei der Behandlung von Lungenfibrose (idiopathischer pulmonaler Fibrose), Asthma, chronisch obstruktiver Lungenerkrankung (COPD), Nierenfibrose, akuter Nierenschädigung, chronischer Nierenerkrankung, Leberfibrose (nichtalkoholischer Steatohepatitis), Hautfibrose, Fibrose des Darms, Brustkrebs, Pankreaskrebs, Eierstockkrebs, Prostatakrebs, Glioblastom, Knochenkrebs, Kolonkrebs, Darmkrebs, Kopf-Hals-Krebs, Melanom, multiplem Myelom, chronischer lymphatischer Leukämie, Krebsschmerz, Tumormetastasierung, Abstoßung eines transplantierten Organs, Sklerodermie, Augenfibrose, altersbedingter Makuladegeneration (AMD), diabetischer Retinopathie, vaskulärer Kollagenkrankheit, Atherosklerose, Raynaud-Syndrom oder neuropathischem Schmerz bei einem Säuger, wobei die Verwendung das Verabreichen einer therapeutisch wirksamen Menge der Verbindung oder der pharmazeutischen Zusammensetzung, an den Säuger mit Bedarf daran, umfasst.

## Revendications

1. Composé de Formule (I) : 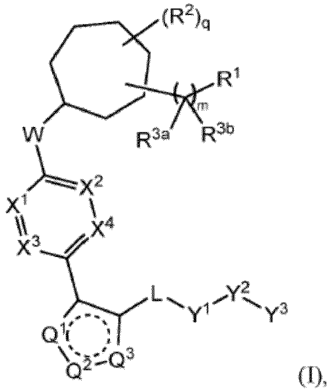 ou un stéréoisomère, tautomère, ou sel ou solvate pharmaceutiquement acceptable de celui-ci, dans lequel :
X¹, X², X³ et X⁴ sont chacun indépendamment CR⁶ ou N ; à condition que pas plus de deux de X¹, X², X³ ou X⁴ ne soient N ;
la fraction 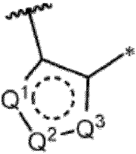 est indépendamment 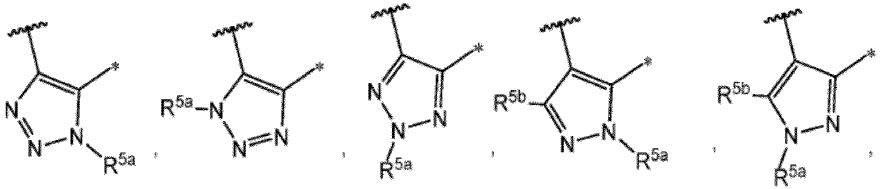 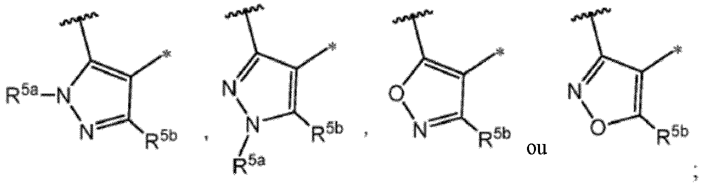
* dénote le point d'attache à L ;
L est indépendamment une liaison covalente ou CH₂ ;
W est O ;
la fraction 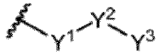 est indépendamment -OR^{4b}, -NR⁷R^{4b}, 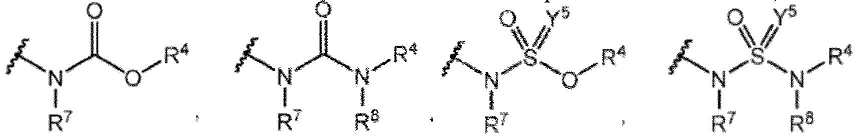
ou 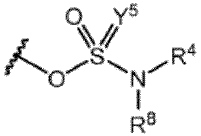
Y⁵ est indépendamment O ou NH ;
R¹ est CO₂H;
chaque R² est indépendamment un halo, cyano, hydroxyle, amino, C₁₋₄ alcoxy, C₁₋₄ haloalkyle, C₁₋₄ haloalcoxy, C₁₋₄ alkylamino, -(CH₂)₀₋₁-(C₃₋₆ cycloalkyle), -(CH₂)₀₋₁-phényle ou C₁₋₆ alkyle ;
R^{3a} est indépendamment un hydrogène, halo, hydroxyle ou C₁₋₄-alkyle ;
R^{3b} est indépendamment un hydrogène, halo, cyano, hydroxyle, amino, C₁₋₄ alkyle, C₁₋₄ haloalkyle, C₁₋₄ alcoxy, C₁₋₄ haloalcoxy ou C₁₋₆ alkyle substitué par 0 à 2 R^{a} ;
ou alternativement, R^{3a} et R^{3b} ensemble, avec l'atome de carbone auquel ils sont attachés, forment un C₃₋₄ carbocyclyle ;
R⁴ est -L₁-R₄ₐ ;
L₁ est indépendamment une liaison covalente ou un C₁₋₄ alkylène substitué par 0 à 4 R⁹ ;
R^{4a} est indépendamment un C₁₋₁₀ alkyle, C₁₋₁₀ haloalkyle, C₁₋₁₀ alcényle, C₃₋₈ cycloalkyle, C₆₋₁₀ aryle, hétérocyclyle à 3 à 8 chaînons, hétéroaryle à 5 à 6 chaînons ; dans lequel chacun de l'alkyle, alcényle, alkylène, cycloalkyle, aryle, hétérocyclyle et hétéroaryle, seul ou comme faisant partie d'autre fraction, est indépendamment substitué par 0 à 3 R¹⁰ ;
R^{4b} est indépendamment un C₁₋₁₀ alkyle, C₁₋₁₀ haloalkyle, C₁₋₁₀ alcényle, C₃₋₈ cycloalkyle, C₆₋₁₀ aryle, hétérocyclyle à 3 à 8 chaînons, hétéroaryle à 5 à 6 chaînons ; dans lequel chacun de l'alkyle, alcényle, alkylène, cycloalkyle, aryle, hétérocyclyle et hétéroaryle, seul ou comme faisant partie d'autre fraction, est indépendamment substitué par 0 à 3 R^{10a} ;
R^{5a} est indépendamment un hydrogène, C₁₋₄ haloalkyle, -(CH₂)₀₋₁-(C₃₋₆ cycloalkyle), -(CH₂)₀₋₁-phényle ou C₁₋₆ alkyle substitué par 0 à 3 R^{a} ;
R^{5b} est indépendamment un hydrogène, halo, cyano, hydroxyle, amino, C₁₋₄ alcoxy, C₁₋₄ haloalkyle, C₁₋₄ haloalcoxy, C₁₋₄ alkylamino, -(CH₂)₀₋₁-(C₃₋₆ cycloalkyle), -(CH₂)₀₋₁-phényle ou C₁₋₆ alkyle substitué par 0 à 3 R^{b} ;
chaque R⁶ est indépendamment un hydrogène, halo, cyano, hydroxyle, amino, C₁₋₄ alkylamino, C₁₋₄ haloalkyle, C₁₋₄ alcoxy, C₁₋₄ haloalcoxy ou C₁₋₆ alkyle substitué par 0 à 1 R^{b} ;
R⁷ et R⁸ sont chacun indépendamment un hydrogène, C₁₋₆ alkyle, C₁₋₄ haloalkyle, C₃₋₆ cycloalkyle ou C₁₋₆ alkyle ;
chaque R⁹ est indépendamment un halo, oxo, cyano, hydroxyle, amino, C₁₋₄ haloalkyle, C₁₋₄ alcoxy, C₁₋₄ haloalcoxy, C₃₋₆ cycloalkyle ou C₁₋₆ alkyle substitué par 0 à 3 R^{a} ;
R¹⁰ et R^{10a} sont chacun indépendamment un halo, hydroxyle, amino, cyano, C₂₋₆ alcényle, C₂₋₆ alcynyle, C₁₋₄ alkylamino, C₁₋₄ haloalkyle, C₁₋₄ alcoxy, C₁₋₄ haloalcoxy, phényle ou hétéroaryle à 5 à 6 chaînons, C₁₋₆ alkyle substitué par 0 à 3 R^{b} ;
R^{a} est indépendamment un halo, cyano, hydroxyle, C₁₋₄ alcoxy, C₁₋₄ haloalkyle ou C₁₋₄ haloalcoxy ;
R^{b} est indépendamment un halo, cyano, hydroxyle, amino, C₁₋₄ alcoxy, C₁₋₄ haloalkyle ou C₁₋₄ haloalcoxy ;
m est un entier de 0, 1 ou 2 ; et
q est un entier de 0, 1 ou 2.

2. Composé selon la revendication 1, dans lequel :
R^{3a} et R^{3b} sont indépendamment un hydrogène ou un C₁₋₄ alkyle ;
ou alternativement, R^{3a} et R^{3b} ensemble, avec l'atome de carbone auquel ils sont attachés, forment un C₃₋₄ cycloalkyle.

3. Composé selon la revendication 1 ou la revendication 2, dans lequel :
X¹, X², X³ et X⁴ sont indépendamment CR⁶ ; ou X¹, X² et X³ sont CR⁶ et X⁴ est N ; ou X² et X³ sont CR⁶ et X¹ et X⁴ sont N ; ou X¹ et X² sont CR⁶ et X³ et X⁴ sont N ; et R⁶ est indépendamment un hydrogène, halo, hydroxyle, C₁₋₆ alkyle, C₁₋₄ haloalkyle ou C₁₋₄ alcoxy.

4. Composé selon l'une quelconque des revendications 1 à 3, dans lequel :
X¹, X², X³ et X⁴ sont indépendamment CR⁶ ; ou X¹, X² et X³ sont CR⁶ et X⁴ est N ; ou X² et X³ sont CR⁶ et X¹ et X⁴ sont N ;
la fraction 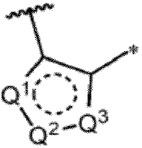 est indépendamment 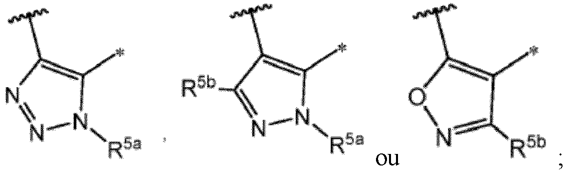
^{∗} dénote le point d'attache à L ;
la fraction 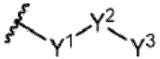 est indépendamment -OR^{4b}, ou 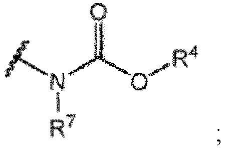
R² est indépendamment un halo, cyano, hydroxyle, C₁₋₄ alcoxy, C₁₋₄ haloalkyle ou C₁₋₄ haloalcoxy ;
R⁴ est indépendamment un C₃₋₆ alkyle, -(CH₂)₀₋₁-C₃₋₆ cycloalkyle, -(CH(C₁₋₂ alkyle))-C₃₋₆ cycloalkyle, -(CH₂)₀₋₁-phényle ou -(CH(C₁₋₂ alkyle))-phényle, dans lequel chacun desdits cycloalkyle et phényle est indépendamment substitué par 0 à 3 R¹⁰ ;
R^{4b} est indépendamment un hétéroaryle à 5 à 6 chaînons substitué par 0 à 2 R^{10a} ;
R^{5a} est indépendamment un C₁₋₆ alkyle ou un -(CH₂)₀₋₁-(C₃₋₆ cycloalkyle) ;
R^{5b} est indépendamment un hydrogène, halo, cyano, hydroxyle, C₁₋₄ alkyle, C₁₋₄ alcoxy, C₁₋₄ haloalkyle, C₁₋₄ haloalcoxy ou -(CH₂)₀₋₁-(C₃₋₆ cycloalkyle) ;
chaque R⁶ est indépendamment un hydrogène, halo, hydroxyle, C₁₋₆ alkyle, C₁₋₄ haloalkyle ou C₁₋₄ alcoxy ;
R⁷ et R⁸ sont chacun indépendamment un hydrogène ou un C₁₋₂ alkyle ;
chaque R¹⁰ est indépendamment un halo, cyano, hydroxyle, C₁₋₆ alkyle, C₁₋₆ haloalkyle, C₁₋₆ alcoxy ou C₁₋₆ haloalcoxy ;
chaque R^{10a} est indépendamment un halo, cyano, hydroxyle, C₁₋₆ alkyle, C₁₋₆ haloalkyle, C₁₋₆ alcoxy, C₁₋₆ haloalcoxy, -(CH₂)₀₋₃-C₃₋₆ cycloalkyle, phényle ou hétéroaryle à 5 à 6 chaînons ;
m est 0 ou 1 ; et
q est 0 ou 1.

5. Composé selon l'une quelconque des revendications 1 à 4, dans lequel :
la fraction 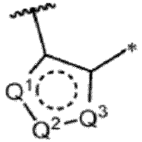 est indépendamment 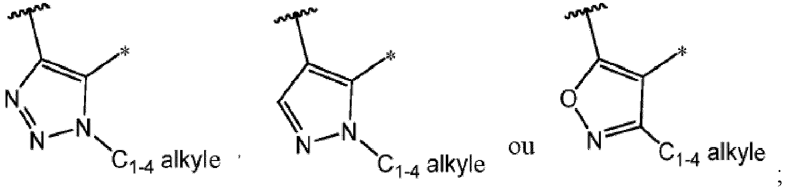
^{∗} dénote le point d'attache à L ;
la fraction 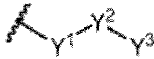 est indépendamment -OR^{4b} ou 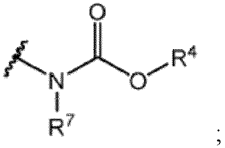
R^{3a} et R^{3b} sont chacun indépendamment un hydrogène ou un C₁₋₄ alkyle ;
ou alternativement, R^{3a} et R^{3b} ensemble, avec l'atome de carbone auquel ils sont attachés, forment un C₃₋₄ cycloalkyle ;
R⁴ est indépendamment un C₃₋₆ alkyle, -(CH₂)₀₋₁-C₃₋₆ cycloalkyle, -(CH₂)-phényle ou -(CH(CH₃))-phényle ; dans lequel chacun desdits alkyle, cycloalkyle et phényle est indépendamment substitué par 0 à 2 R¹⁰ ;
R^{4b} est indépendamment 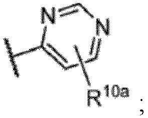
R⁶ est indépendamment un hydrogène, halo ou C₁₋₄ alkyle ;
R⁷ et R⁸ sont chacun indépendamment un hydrogène ou un C₁₋₂ alkyle ;
chaque R¹⁰ est indépendamment un halo ou un C₁₋₄ alkyle ; et
R^{10a} est indépendamment un C₁₋₄ alkyle, C₁₋₄ alcoxy, -(CH₂)₁₋₃-C₃₋₆ cycloalkyle, phényle ou pyridyle.

6. Composé selon la revendication 1, qui est un composé de Formule (II) : 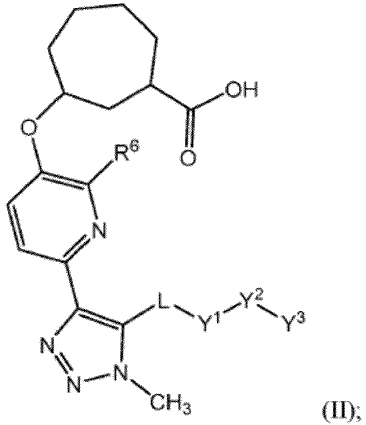
ou un stéréoisomère, tautomère, ou un sel ou solvate pharmaceutiquement acceptable de celui-ci, dans lequel :
la fraction 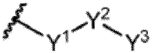 est indépendamment -OR^{4b} ou 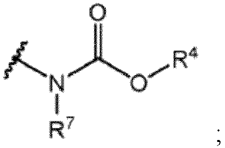
R⁴ est indépendamment un C₃₋₆-alkyle, -(CH₂)₀₋₁-C₃₋₆-cycloalkyle, -(CH₂)-phényle ou -(CH(CH₃))-phényle ;
R^{4b} est indépendamment 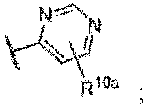
R⁶ est indépendamment un hydrogène, halo ou C₁₋₄ alkyle ;
R⁷ et R⁸ sont chacun indépendamment un hydrogène ou un méthyle ;
R^{10a} est indépendamment un C₁₋₄ alkyle ou C₁₋₄ alcoxy.

7. Composé selon la revendication 1, qui est sélectionné parmi :
Acide (±)-3-((6-(5-(((6-éthylpyrimidin-4-yl)oxy)méthyl)-1-méthyl-1H-1,2,3-triazol-4-yl)-2-méthylpyridin-3-yl)oxy)cycloheptane-1-carboxylique ;
Acide (±)-3-((6-(5-(((6-(tert-butyl)pyrimidin-4-yl)oxy)méthyl)-1-méthyl-1H-1,2,3-triazol-4-yl)-2-méthylpyridin-3-yl)oxy)cycloheptane-1-carboxylique ;
Acide (±)-3-((6-(5-(((6-(tert-butyl)pyrimidin-4-yl)oxy)méthyl)-1-méthyl-1H-1,2,3-triazol-4-yl)-2-méthylpyridin-3-yl)oxy)cyclo-heptane-1-carboxylique ;
Acide (±)-3-((6-(5-(((6-isopropylpyrimidin-4-yl)oxy)méthyl)-1-méthyl-1H-1,2,3-triazol-4-yl)-2-méthylpyridin-3-yl)oxy)cycloheptane-1-carboxylique ;
Acide (±)-3-((6-(5-(((6-méthoxypyrimidin-4-yl)oxy)méthyl)-1-méthyl-1H-1,2,3-triazol-4-yl)-2-méthylpyridin-3-yl)oxy)cycloheptane-1-carboxylique ;
Acide (±)-3-((2-méthyl-6-(1-méthyl-5-((((R)-1-phényléthoxy)carbonyl)amino)-1H-1,2,3-triazol-4-yl)pyridin-3-yl)oxy)cycloheptane-1-carboxylique ;
Acide (±)-(((cyclopropylméthoxy)carbonyl)amino)-1-méthyl-1H-1H-1,2,3-triazol-4-yl)-2-méthylpyridin-3-yl)oxy)cycloheptane-carboxylique ;
Acide (±)-3-((6-(5-((isobutoxycarbonyl)amino)-1-méthyl-1H-1,2,3-triazol-4-yl)-2-méthylpyridin-3-yl)oxy)cycloheptane-1-carboxylique ;
ou un stéréoisomère, un tautomère, ou un sel ou solvate pharmaceutiquement acceptable de ceux-ci.

8. Composition pharmaceutique comprenant un ou plusieurs composés selon l'une quelconque des revendications 1 à 7, ou un stéréoisomère, tautomère, ou sel ou solvate pharmaceutiquement acceptable de ceux-ci ; et un véhicule ou diluant pharmaceutiquement acceptable.

9. Composé ou un stéréoisomère, tautomère, ou sel ou solvate pharmaceutiquement acceptable de celui-ci selon l'une quelconque des revendications 1 à 7, ou composition pharmaceutique selon la revendication 8, pour utilisation en thérapie.

10. Composé ou un stéréoisomère, tautomère, ou sel ou solvate pharmaceutiquement acceptable de celui-ci selon l'une quelconque des revendications 1 à 7, ou composition pharmaceutique selon la revendication 8, pour utilisation dans le traitement d'une maladie, d'un trouble ou d'une condition associé à une dysrégulation du récepteur 1 de l'acide lysophosphatidique (LPA1), où la maladie, le trouble ou la condition est lié à une fibrose pathologique, à un rejet de greffe, à un cancer, à l'ostéoporose ou à des troubles inflammatoires.

11. Composé ou composition pour utilisation selon la revendication 10, où la fibrose pathologique est une fibrose pulmonaire, hépatique, rénale, cardiaque, du derme, oculaire ou du pancréas.

12. Composé ou composition pour utilisation selon la revendication 10, où la maladie, trouble ou condition est une fibrose pulmonaire idiopathique (FPI), stéatohépatite non alcoolique (NASH), maladie du foie gras non alcoolique (NAFLD), maladie rénale chronique, maladie rénale diabétique ou sclérose systémique.

13. Composé ou composition pour utilisation selon la revendication 10, où le cancer est un cancer de la vessie, du sang, des os, du cerveau, du sein, du système nerveux central, du col de l'utérus, du côlon, de l'endomètre, de l'oesophage, de la vésicule biliaire, des organes génitaux, du tractus génito-urinaire, de la tête, du rein, du larynx, du foie, du poumon, des tissus musculaires, du cou, de la muqueuse buccale ou nasale, de l'ovaire, du pancréas, de la prostate, de la peau, de la rate, de l'intestin grêle, du gros intestin, de l'estomac, du testicule ou de la thyroïde.

14. Composé ou un stéréoisomère, tautomère, ou sel ou solvate pharmaceutiquement acceptable de celui-ci selon l'une quelconque des revendications 1 à 7, ou composition pharmaceutique selon la revendication 8, pour utilisation dans le traitement d'une fibrose chez un mammifère, l'utilisation comprenant administrer une quantité thérapeutiquement efficace du composé ou de la composition pharmaceutique, au mammifère en ayant besoin, de préférence où la fibrose est une fibrose pulmonaire idiopathique (FPI), stéatohépatite non alcoolique (NASH), maladie rénale chronique, maladie rénale diabétique ou sclérose systémique.

15. Composé ou un stéréoisomère, tautomère, ou sel ou solvate pharmaceutiquement acceptable de celui-ci selon l'une quelconque des revendications 1 à 7, ou composition pharmaceutique selon la revendication 8, pour utilisation dans le traitement d'une fibrose pulmonaire (fibrose pulmonaire idiopathique), de l'asthme, maladie pulmonaire obstructive chronique (MPOC), fibrose rénale, lésion rénale aiguë, maladie rénale chronique, fibrose du foie (stéatohépatite non alcoolique), fibrose de la peau, fibrose de l'intestin, cancer du sein, cancer du pancréas, cancer de l'ovaire, cancer de la prostate, glioblastome, cancer des os, cancer du côlon, cancer de l'intestin, cancer de la tête et du cou, mélanome, myélome multiple, leucémie lymphocytaire chronique, douleur cancéreuse, métastase tumorale, rejet de greffe d'organe, scléroderme, fibrose oculaire, dégénérescence maculaire liée à l'âge (DMA), rétinopathie diabétique, maladie du collagène vasculaire, athérosclérose, syndrome de Raynaud ou douleur neuropathique chez un mammifère, l'utilisation comprenant l'administration d'une quantité thérapeutiquement efficace du composé ou de la composition pharmaceutique au mammifère en ayant besoin.
